# EUROPEAN PATENT APPLICATION

(11) **EP 1 473 564 A1**
(43) Date of publication of application: **03.11.2004**
(21) Application number: 02743705.2
(22) Date of filing: 24.06.2002
(51) Int. Cl.: G01N 33/53, C07K 7/06

(54) **METHOD OF DETECTING CELLULAR IMMUNITY AND APPLICATION THEREOF TO DRUGS**

(30) Priority: 18.09.2001 JP 2001283413
(71) Applicant: Itoh, Kyogo, Miyaki-gun, Saga 841-0205 (JP)
(72) Inventor: ITOH, Kyogo, Miyaki-gun, Saga 841-0205 (JP); HIDA, Naoya, Kurume-shi, Fukuoka, Fukuoka 830-0011 (JP)
(74) Representative: VOSSIUS & PARTNER
(86) International application number: PCT/JP2002/006298
(87) International publication number: WO 2003/025569

(57) **Abstract**

It is intended to provide a convenient immunity monitoring system whereby T cell frequencies specific to a plural number of antigen peptides can be assayed by using a relatively small amount of blood. Peripheral monocytes are collected and frequently stimulated with an antigen without directly using any antigen presenting cells. Then T cells specific to the antigen in the thus stimulated peripheral monocytes are detected to thereby detect antigen-specific T cells. thus, diseases such as cancer can be prevented or treated with the use of a peptide having such a function, in particular, cancer tumor-rejection antigen peptide.

SEQUENCE LISTING

<110> ITOH, Kyogo
<120> METHOD OF DETECTING CELLULAR IMMUNITY AND APPLICATION
THEREOF TO DRUGS
<130> K 2113 EP
<140> PCT/JP02/06298
<141> 2002-06-24
<150> JP 2001-283413
<151> 2001-09-18
<160> 32
<170> PatentIn version 3.1
<210> 1
<211> 9
<212> PRT
<213> Homo sapiens
<300>
<303> Int. J. Cancer
<304> 88
<306> 633-639
<307> 2000
<400> 1
Leu Leu Gln Ala Glu Ala Pro Arg Leu
1               5
<210> 2
<211> 9
<212> PRT
<213> Homo sapiens
<300>
<303> Int. J. Cancer
<304> 88
<306> 633-639
<307> 2000
<400> 2
Arg Leu Ala Glu Tyr Gln Ala Tyr Ile
1               5
<210> 3
<211> 10
<212> PRT
<213> Homo sapiens
<300>

<303> Jpn. J. Cancer Res.
<304> 92
<306> 762-767
<307> 2001
<400> 3
Lys Leu Lys His Tyr Gly Pro Gly Trp Val
1               5                   10
<210> 4
<211> 8
<212> PRT
<213> Homo sapiens
<300>
<303> Jpn. J. Cancer Res.
<304> 92
<306> 762-767
<307> 2001
<400> 4
Val Leu Glu Gly Met Glu Val Val
1               5
<210> 5
<211> 9
<212> PRT
<213> Homo sapiens
<300>
<303> Int. J. Cancer
<307> 2001
<400> 5
Lys Leu Val Glu Arg Leu Gly Ala Ala
1               5
<210> 6
<211> 9
<212> PRT
<213> Homo sapiens
<300>
<303> Int. J. Cancer
<307> 2001
<400> 6
Asp Val Trp Ser Phe Gly Ile Leu Leu
1               5
<210> 7
<211> 9

<212> PRT
<213> Homo sapiens
<300>
<303> Cancer Res.
<304> 61
<306> 2038-2046
<307> 2001
<400> 7
Arg Ile Ile Tyr Asp Arg Lys Phe Leu
1               5
<210> 8
<211> 9
<212> PRT
<213> Homo sapiens
<300>
<303> Cancer Res.
<304> 61
<306> 2038-2046
<307> 2001
<400> 8
Gly Leu Leu Phe Leu His Thr Arg Thr
1               5
<210> 9
<211> 9
<212> PRT
<213> Homo sapiens
<300>
<303> Cancer Res.
<304> 61
<306> 2038-2046
<307> 2001
<400> 9
Asp Leu Leu Ser His Ala Phe Phe Ala
1               5
<210> 10
<211> 9
<212> PRT
<213> Homo sapiens
<300>
<303> Cancer Res.
<304> 61

<306> 2038-2046
<307> 2001
<400> 10
Ala Ser Leu Asp Ser Asp Pro Trp Val
1               5
<210> 11
<211> 9
<212> PRT
<213> Homo sapiens
<300>
<303> Cancer Res.
<304> 61
<306> 2038-2046
<307> 2001
<400> 11
Ile Leu Gly Glu Leu Arg Glu Lys Val
1               5
<210> 12
<211> 9
<212> PRT
<213> Homo sapiens
<300>
<303> Cancer Res.
<304> 61
<306> 2038-2046
<307> 2001
<400> 12
Arg Leu Gln Glu Trp Cys Ser Val Ile
1               5
<210> 13
<211> 9
<212> PRT
<213> Homo sapiens
<300>
<303> Cancer Res.
<304> 61
<306> 2038-2046
<307> 2001
<400> 13
Leu Ile Ala Asp Phe Leu Ser Gly Leu
1               5

<210> 14
<211> 9
<212> PRT
<213> Homo sapiens
<300>
<303> Cancer Res.
<304> 61
<306> 2038-2046
<307> 2001
<400> 14
Ile Leu Pro Arg Lys His His Arg Ile
1               5
<210> 15
<211> 9
<212> PRT
<213> Homo sapiens
<300>
<303> Cancer Res.
<304> 61
<306> 2038-2046
<307> 2001
<400> 15
Ala Leu Val Glu Phe Glu Asp Val Leu
1               5
<210> 16
<211> 10
<212> PRT
<213> Homo sapiens
<300>
<303> Cancer Res.
<304> 61
<306> 2038-2046
<307> 2001
<400> 16
Asn Val Leu His Phe Phe Asn Ala Pro Leu
1               5                   10
<210> 17
<211> 9
<212> PRT
<213> Homo sapiens
<300>

<303> Int. J. Cancer
<304> 81
<306> 459-466
<307> 1999
<400> 17
Glu Tyr Arg Gly Phe Thr Gln Asp Phe
1               5
<210> 18
<211> 9
<212> PRT
<213> Homo sapiens
<300>
<303> J. Immunol.
<304> 164
<306> 2565-2574
<307> 2000
<400> 18
Asp Tyr Ser Ala Arg Trp Asn Glu Ile
1               5
<210> 19
<211> 9
<212> PRT
<213> Homo sapiens
<300>
<303> J. Immunol.
<304> 164
<306> 2565-2574
<307> 2000
<400> 19
Ala Tyr Asp Phe Leu Tyr Asn Tyr Leu
1               5
<210> 20
<211> 9
<212> PRT
<213> Homo sapiens
<300>
<303> J. Immunol.
<304> 164
<306> 2565-2574
<307> 2000
<400> 20

Ser Tyr Thr Arg Leu Phe Leu Ile Leu
1               5
<210> 21
<211> 10
<212> PRT
<213> Homo sapiens
<300>
<303> Cancer Res.
<304> 59
<306> 4056-4063
<307> 1999
<400> 21
Val Tyr Asp Tyr Asn Cys His Val Asp Leu
1               5                   10
<210> 22
<211> 9
<212> PRT
<213> Homo sapiens
<300>
<303> Cancer Res.
<304> 59
<306> 4056-4063
<307> 1999
<400> 22
Ala Tyr Ile Asp Phe Glu Met Lys Ile
1               5
<210> 23
<211> 9
<212> PRT
<213> Homo sapiens
<300>
<303> J. Immunol.
<304> 163
<306> 4994-5004
<307> 1999
<400> 23
Lys Phe His Arg Val Ile Lys Asp Phe
1               5
<210> 24
<211> 9
<212> PRT

<213>  Homo sapiens
<300>
<303>  J. Immunol.
<304>  163
<306>  4994-5004
<307>  1999
<400>  24
Asp Phe Met Ile Gln Gly Gly Asp Phe
1               5
<210>  25
<211>  9
<212>  PRT
<213>  Homo sapiens
<300>
<303>  Eur. J. Immunol.
<304>  31
<306>  323-332
<307>  2001
<400>  25
His Tyr Thr Asn Ala Ser Asp Gly Leu
1               5
<210>  26
<211>  9
<212>  PRT
<213>  Homo sapiens
<300>
<303>  Eur. J. Immunol.
<304>  31
<306>  323-332
<307>  2001
<400>  26
Thr Phe Asp Tyr Leu Arg Ser Val Leu
1               5
<210>  27
<211>  10
<212>  PRT
<213>  Homo sapiens
<300>
<303>  Eur. J. Immunol.
<304>  31
<306>  323-332

<307> 2001
<400> 27
Asp Tyr Leu Arg Ser Val Leu Glu Asp Phe
1               5                   10
<210> 28
<211> 8
<212> PRT
<213> Homo sapiens
<300>
<303> Cancer Res.
<304> 60
<306> 3550-3558
<307> 2000
<400> 28
Ala Phe Leu Arg His Ala Ala Leu
1               5
<210> 29
<211> 10
<212> PRT
<213> Homo sapiens
<300>
<303> Cancer Res.
<304> 60
<306> 3550-3558
<307> 2000
<400> 29
Asp Tyr Pro Ser Leu Ser Ala Thr Asp Ile
1               5                   10
<210> 30
<211> 9
<212> PRT
<213> Homo sapiens
<300>
<303> Int. J. Cancer
<304> 88
<306> 633-639
<307> 2000
<400> 30
Glu Tyr Cys Leu Lys Phe Thr Lys Leu
1               5
<210> 31

<211> 9
<212> PRT
<213> EB Virus
<300>
<303> J. Immunol.
<304> 158
<306> 3325-3334
<307> 1997
<400> 31
Thr Tyr Gly Pro Val Phe Met Cys Leu
1               5
<210> 32
<211> 11
<212> PRT
<213> Human immunodeficiency virus
<300>
<303> J. Immunol.
<304> 159
<306> 6242-6252
<307> 1997
<400> 32
Arg Tyr Leu Arg Asp Gln Gln Leu Leu Gly Ile
1               5                   10

## Description

### FIELD OF THE INVENTION

This invention relates to the field of immunotherapy and to a method of detecting cellular immunity and a procedure for use thereof. More specifically, the invention relates to a method of detecting antigen-specific T cells and a procedure for use thereof.

### BACKGROUND OF THE INVENTION

Thanks to the latest developments in the field of cellular immunology and molecular biology, many epitope peptides recognized by cytotoxic T lymphocytes (CTLs), which react to cancerous or virus-infected cells, have been identified. As a result of such developments, we have found new peptide-based immunotherapies for cancers, virus infections, and various autoimmune diseases. T cells associated with cellular immunity, especially antigen-specific T cells, have an important role in the elimination of cancerous or virus-infected cells by a body. Antigen-specific T cells are also known to be involved in autoimmune diseases such as multiple sclerosis and rheumatoid arthritis. The antigen-specific T cells specifically recognize and react with an antigen peptide presented at the surface of a target cell and a complex of the major histocompatibility complex (MHC), using a T cell receptor (TCR). It is thought that the cytotoxic T lymphocytes (CTLs) thereby damage cancerous or virus-infected cells, and that autoreactive T cells cause autoimmune diseases. Therefore, the detection of antigen-specific T cells is essential in order to properly perform immunotherapy which aims at the augmentation of the cytotoxic T lymphocytes (CTLs) against cancerous or virus-infected cells and the inhibition of autoreactive T cells against autoimmune diseases. The detection and monitoring of such T cells determines which antigen peptide will be effective in a specific patient before treatment, or confirms the effect after treatment of the antigen peptide used in a procedure on a patient. As traditional detection methods of antigen-specific T cells, limiting dilution analysis, the ELISPOT method, MHC tetramer technology, and the like are known in the art.

In limiting dilution analysis, serially diluted T cells are cultured in a plate and the T cells subsequently react with sensitized antigen-presenting target cells. A statistical calculation is performed based on the positive well count on each plate, and thereby the frequency of antigen-specific T cells is calculated (J. Immunol. 126:1614-1620, 1981). Although this method is superior in quantifiability, it requires a large number of T cell samples because several concentrations of T cells are generally used and one plate (60-96 wells) is needed for each concentration. In this method, the process is complicated because the lymphocytes used as effector cells are sensitized with antigen peptide multiple times, once a week in order to improve the detection sensitivity of the antigen-specific T cells.

In the ELISPOT method, T cells are cultured along with an antigen in a plate whose wells have coated with antibody to cytokine. The cytokine produced by the T cells is trapped by the antibody at the bottom of the wells, and is subsequently visualized using an enzyme-labeled secondary antibody and a coloring substrate. The number of positive spots is counted to calculate the frequency of specific T cells (J. Immunol. Methods, 128: 65, 1990). In this method, since the noise is high, determining whether a particular spot is positive or not may be difficult.

In the MHC tetramer technique, the terminus of an HLA-antigen peptide complex of antigen peptide, MHC and β2 microglobulin assembled in vitro is biotinated. Antigen-specific T cells are subsequently stained using MHC tetramer produced by binding fluorescent-labeled streptavidin and the T cells are analyzed with a flow cytometer (FACS) (Science 275:94, 1996). In this method, it is possible to analyze multiple specimens, but nonfunctional T cells are also detected because the detection is only based on the bond of TCR and MHC tetramer. In addition, an HLA- antigen peptide complex of each peptide needs to be prepared.

These traditional techniques, including the ELISPOT method and MHC tetramer technology, are useful in the detection of antigen-specific CTL. However, these methods employ stimulation by antigen presenting cells (APCs) and induction of antigen-specific CTL from peripheral-blood mononuclear cells (PBMCs) in a cancer patient, and they require a considerable number of peripheral-blood mononuclear cells as feeder cells in order to study the reactivity to a cluster of peptides.

As set forth above, each traditional method of detecting antigen-specific cells has some problems associated with it, such as the amount of blood to be collected, the complexity of the procedure and detection sensitivity. Therefore, a simple immune monitoring system which can detect the frequency of T cells specific to multiple antigen peptides even from a relatively small amount of blood is highly desirable.

The detection of antigen-specific T cells is performed by quantifying the production of cytokines including interferon-γ (IFN-γ). This quantification is performed by ELISA (Enzyme-Linked Immuno Sorbent Assay) or FLISA (Fluorescence-Linked Immuno Sorbent Assay) methods. ELISA is widely used in the quantification of IFN-γ in the art. On the other hand, FLISA can accurately quantify IFN-γ. The method and the result of quantifying IFN-γ using FLISA are reported in the literature (Komatsu, N., et al., J. Immunol. Methods 263 (2002) 169-176). This publication is incorporated by a reference herein, and should be referenced as a part herein.

### SUMMARY OF THE INVENTION

The inventor investigated the issues mentioned above and earnestly carried out research to establish a simple and useful immune monitoring method, which resulted in the completion of the present invention by finding that it is possible to stimulate PBMCs by frequent stimulation with peptides as an antigen without adding any antigen-presenting cells (APCs). Therefore, the present invention aims at providing a new method of detecting antigen-specific cells while avoiding these issues.

To achieve this goal, the present invention is a method of detecting antigen-specific T cells. In this method the collected peripheral blood mononuclear cells are frequently stimulated with an antigen, and the T cells specific to the antigen are detected, but antigen presenting cells are not directly used to stimulate the peripheral blood mononuclear cells. The present invention also provides a method of detecting antigen-specific T cells in the stimulated peripheral blood mononuclear cells using designated antigen presenting cells.

More specifically, the present invention provides a method of detecting antigen-specific T cells comprising using an antigen peptide, or an antigen peptide derived from the antigen protein, as an antigen; for example, one or more peptides represented by SEQ ID NOs: 1 to 30. In one preferred embodiment, the present invention provides a method comprising frequent, multiple stimulations at an interval of more than twice weekly, or every two to five days. In yet another preferred embodiment, the present invention provides a method comprising detection of antigen-specific T cells using the amount of cytokine, preferably the amount of IFN-γ, produced by the antigen-specific T cells as the measure. In still another embodiment, the present invention provides a method comprising frequent stimulation in a well of a microplate, preferably a well with a U-shaped bottom. In a more preferred embodiment, the present invention provides a method comprising adjusting the number of peripheral blood mononuclear cells to between 2.5 x 10⁴/well - 2 x 10⁵/well with the use of a 96-well microplate, or 2.5 x 10⁴/well - 1 x 10⁵/well with the use of a 384-well microplate.

In another embodiment, the present invention aims at providing a method of detecting antigens reactive to antigen-specific T cells.

In accordance with the present invention, the method of detecting an antigen consists of frequent stimulation of collected peripheral blood mononuclear cells by multiple antigen candidates and detection of an antigen candidate which induces antigen-specific T cells. This method is characterized by stimulation using the antigen itself, without directly using antigen presenting cells to stimulate the peripheral blood mononuclear cells.

The traditional method of detecting antigen-specific T cells was performed by first using an antigen to sensitize antigen presenting cells derived from peripheral blood mononuclear cells collected from a patient, and then stimulating T cells with the antigen presenting cells and then using a designated detection method. In contrast, the method of detecting an antigen, according to the present invention, has the significant advantages that the method needs only a small amount of the patient's blood, is a comparatively simple process, and enables assessment of many antigen peptides at once. This is because the method uses an antigen itself when stimulating peripheral blood mononuclear cells and does not directly use antigen presenting cells for stimulation.

The present invention also comprises a method of using designated antigen presenting cells for the detection of antigen candidates which may induce antigen-specific T cells.

In specific embodiment, the method of detecting antigen according to the present invention consists of using an antigen protein or an antigen peptide derived from the antigen protein; for example, one or more peptides represented by SEQ ID NOs: 1 to 30. In one preferred embodiment, the method according to the present invention consists of frequent, multiple stimulations at intervals of twice weekly, or every two to five days; and detection of an antigen candidate which can stimulate antigen-specific T cells by quantifying the amount of cytokine, preferably IFN-γ, produced by the antigen-specific T cells. In another preferred embodiment, the present invention comprises a method comprising adjusting the number of peripheral blood mononuclear cells to between 2.5 x 10⁴/well - 2 x 10⁵/well with the use of a 96-well microplate or 2.5 x 10⁴/well - 1 x 10⁵/well with the use of a 384-well microplate.

The method of detecting antigen according to the above embodiment of the present invention can provide a beneficial method for the treatment of cancer, AIDS, and the like, since we can easily detect an antigen reactive to antigen-specific T cells. In other words, according to the method of the invention, we can easily detect antigens associated with diseases, such as cancer and AIDS, and thereby provide specific pharmaceutical products tailored to each affected patient. It has been demonstrated that infection by the AIDS virus was inhibited by initially immunizing with a DNA vaccine which codes such an antigen, and subsequently applying additional immunization (Science 292; 69-74, 2001). However, the result simply indicates the efficacy as a protecting vaccine but does not clarify the usefulness of the aforementioned DNA vaccine as a therapeutic vaccine.

Therefore, in another embodiment, the present invention provides a tailored pharmaceutical composition dedicated to a target disease to treat that disease in a patient. That pharmaceutical composition would contain an antigen detected using the above method of the present invention.

In one preferred embodiment, the present invention provides a tailored pharmaceutical composition which contains an antigen peptide or an antigen peptide derived from the antigen protein. Preferably, that antigen would correspond to the antigen detected by the antigen detection method described in another embodiment of the invention. In yet another preferred embodiment, the present invention provides a tailored pharmaceutical composition containing a therapeutically effective amount of such an antigen to treat a target disease in a patient.

In this further embodiment, the present invention provides a method of administering a tailored pharmaceutical composition which allows a tailored medical treatment dedicated to a target disease in a patient by administering a therapeutically effective amount of an antigen related to that target disease in that patient. In the preferred embodiment, the present invention provides a method of administering a therapeutically effective amount of the tailored pharmaceutical composition and a method of administering the tailored pharmaceutical composition by administering the antigen.

In another preferred embodiment, the tailored pharmaceutical composition according to the present invention consists of an antigen that is an antigen peptide or an antigen peptide derived from the antigen protein, preferably one or more antigen peptides represented by SEQ ID NOs: 1 to 30.

In yet another embodiment, the present invention provides a method of treating a disease comprising administering a therapeutically effective amount of antigen associated with a target disease in a patient, so that the tailored pharmaceutical composition of the invention provides tailored medical treatment specific to that target disease in that patient. In this preferred embodiment, the present invention provides a method of administering a therapeutically effective amount of the tailored pharmaceutical composition, more specifically, the method of administering the tailored pharmaceutical composition by which the antigen is administered. In more preferred embodiment of the invention, the present invention provides a method of treating a disease comprising administering an antigen peptide , possibly derived from the antigen protein, as the antigen; more preferably, one or more antigen proteins represented by SEQ ID NOs: 1 to 30.

In another embodiment of the invention, a method of detecting allergic peptide-specific IgE response and type I allergy is provided, using a non-allergic peptide derived from an allergic peptide-retaining antigen, by vaccinating with the non-allergic peptide derived from the allergic peptide-retaining antigen.

In yet another embodiment, the present invention provides a desensitization method using a peptide, comprising inhibiting the allergic peptide-specific IgE response and type I allergy by vaccinating with a non-allergic peptide derived from an allergic peptide-retaining antigen.

### BRIEF DESCRIPTION OF THE DRAWINGS

Figure 1 is a graph showing the production of IFN-γ to the number of peripheral blood mononuclear cells per well used for antigen stimulation in healthy donors.
Figure 2 is a graph showing the influence of detection sensitivity of antigen-specific T cells to the number of peripheral blood mononuclear cells used for antigen stimulation.
Figure 3 is a graph showing the result of detection of T cells specific to EB virus-derived HLA-A24 restricted-peptide in peripheral blood mononuclear cells of 10 healthy HLA-A24 positive donors.
Figure 4 is a graph showing the result of detection of T cells specific to EB virus-derived HLA-A2 restricted-peptide in peripheral blood mononuclear cells of 5 healthy HLA-A24 positive donors.
Figure 5 is a graph showing the cytotoxicity of peptide-stimulated CTL of one healthy donor.
Figure 6 is a graph showing the result of flow cytometry analysis of PBMCs using the culture protocol of the present invention.
Figure 7 is a graph showing the result of HLA tetramer assay of in vitro sensitized PBMCs.
Figure 8 is a graph showing the analytical results on the frequency of CTL precursor in in-vitro stimulated PBMCs using the culture protocol of the present invention.
Figure 9 is a graph showing the results of detecting peptide-specific CTL precursor derived from a cancer antigen in PBMCs of a cancer patient.
Figure 10 is an illustration which depicts the principle of the FLISA method.
Figure 11 is a graph showing the standard curve of IFN-γ based on the FLISA method.

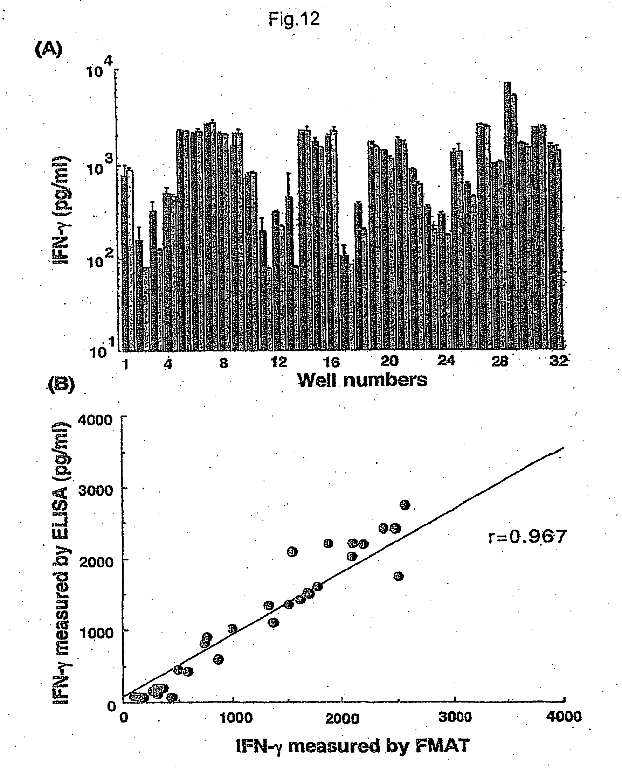

Figure 12 is a graph showing the production of IFN-γ in each well of biological sample. (A) The graph shows the results wherein the PBMCs of a cancer patient were cultured in a 24-well plate along with 10 µM of peptide in the presence of IL-2, and re-stimulated on day 7 and 14, with the production of IFN-γ being measured subsequently. Each column signifies the average of two runs of assay. The columns in dark gray show the results of FLISA measurements, the columns in light gray show the result of ELISA measurements. (B) The graph shows the relationship between the levels of the production of IFN-γ measured by FLISA and ELISA.
Figure 13 is a graph showing the comparison of operation time required for FLISA and ELISA based on the number of wells on a plate.
Figure 14 is a graph showing the result of kinetic analysis of peptide-specific CTL response.
Figure 15 is a graph showing the level of carcinoembryonic antigen (CEA) in a patient with cervical cancer before and after the treatment of antigen peptide selected according to the method of the invention.
Figure 16 is a graph showing the level of squamous cell carcinoma related antigen (SCC) in a patient with cervical cancer before and after the treatment of antigen peptide selected according to the method of the invention.
Figure 17 is a graph showing the reduction ratio of tumor in a patient with cervical cancer before and after the treatment of antigen peptide selected according to the method of the invention.
Figure 18 is a graph showing the level of carcinoembryonic antigen (CEA) in a patient with cervical cancer before and after the treatment of antigen peptide selected according to the method of the invention.
Figure 19 is a graph showing the level of prostate-specific antigen (PSA) in a patient with prostate cancer before and after the treatment of antigen peptide selected according to the method of the invention.
Figure 20 is a graph showing the cytotoxicity to cancerous cells in each patient with pancreatic cancer.
Figure 21 is a graph showing the level of serum IgG and IgE against serum SART3 before and after the vaccination of SART3 peptide in a patient with colorectal cancer.
Figure 22 is a graph showing the production of IFN-γ in positive and negative wells in the analysis of CTL precursor frequency.
Figure 23 is a graph showing the results of the analysis of HLA-A24 restricted CTL precursor frequency.
Figure 24 is a graph showing the results of the analysis of SART3₁₀₉ specific CTL precursor frequency.
Figure 25 is a graph showing the results of kinetic analysis on cellular responsiveness in a patient with colorectal cancer, wherein it shows HLA-A24 restricted CTL activity, SART3₁₀₉ specific CTL activity, and SART3₃₁₅ specific CTL activity, respectively.
Figure 26 is a graph showing the clinical history, tumor markers, and kinetics of immune response in a patient with colorectal cancer.
Figure 27 is a graph showing the CTL activity before and after vaccination of each peptide.
Figure 28 is a graph showing the level of serum IgG against a vaccinated peptide.
Figure 29 is a graph showing the clinical and immunological response in a patient with colorectal cancer.

### DETAILED DESCRIPTION OF THE INVENTION

The detection method according to the invention, as described above, consists of frequent stimulation of peripheral blood mononuclear cells collected from a patient with a disease, using an antigen without adding new antigen-presenting cells, followed by detection of antigen-specific T cells in the peripheral blood mononuclear cells. Alternatively, it may consist of frequent stimulation of the peripheral blood mononuclear cells using multiple antigen candidates without adding new antigen-presenting cells, followed by detection of an antigen which aims at antigen candidates which induce antigen-presenting T cells.

The antigen-specific T cells for the purpose of this invention are the T cells which recognize the complex of antigen peptide presented at the surface of the major histocompatibility complex (MHC) of the target cells using TCR and react; the examples includes CTL and helper T cells.

The detection and processing methods of antigen-specific T cells according to the present invention are described below.

### (1) Collection of Peripheral Blood Mononuclear Cells

The collection of peripheral blood mononuclear cells may be performed by any method known in the art. For example, the collection may be performed by the specific gravity centrifugal method using Ficoll Conray solution. The collected peripheral mononuclear cells are cultured in the standard lymphocyte medium. The cultured peripheral blood mononuclear cells are suspended in the same lymphocyte medium at an appropriate concentration, preferably 5x10⁵ cells/ml, and inoculated on a microplate. The microplate used is preferably one having wells with a U-shaped bottom, without limitation. It is preferable to use a medium having peripheral blood mononuclear cells at a concentration ranging from 2.5x10⁴ to 2/10⁵ cells/well.

### (2) Frequent Stimulation of Peripheral Blood Mononuclear Cells Using an Antigen

The antigen used for stimulation of peripheral blood mononuclear cells may be the antigen itself, or part thereof. For example, a tumor antigen would include a tumor specific protein, i.e. a tumor antigen protein, or an antigen peptide derived from the antigen protein.

MAGE (Science, 254:1643, 1991), gp100 (J. Exp. Med., 179:1005, 1994), MART-1 (Proc. Natl. Acad. Sci. USA, 91:3515, 1994), tyrosinase (J. Exp. Med., 178:489, 1993), MAGE related protein group (J. Exp. Med., 179:921, 1994), β-catechin (J. Exp. Med., 183:1185, 1996), CDK4 (Science, 269:1281, 1995), HER2/neu (J. Exp. Med., 181:2109, 1996), p53 (variant) (Proc. Natl. Acad. Sci. USA, 93:14704, 1996), CEA (J. Natl, Cancer. Inst., 87:982, 1995), PSA (J. Natl. Cancer. Inst., 89:293, 1997), HPV (J. Immunol., 154:5934, 1995), EBV (Int. Immunol., 7:653, 1995) are listed as the tumor antigen proteins.

The tumor antigen protein is produced by intracellular synthesis of tumor cells, followed by intracellular decomposition by proteasome to generate tumor antigen peptides. The resulting tumor antigen peptides form a complex with MHC class I antigen (HLA antigen) in endoplasmic reticulum, and the antigen is carried to and presented at the surface of cells. Examples of tumor antigen peptides include antigen peptides comprising protein fragments of SART1 (J. Exp. Med., 187:277, 1998, International Publication Number 97/46676), SART2 (Kokai number (1999) 318455), SART3 (International Publication Number 00/12701), and ART4. Examples of such tumor peptides are listed in the Table 1 below.

### <Table 1>

### <Table 2>

In addition to such tumor-derived peptides, an exotic antigen-derived peptide may be also used.

The antigen peptide which can be used in the present invention may be synthesized by the usual techniques, such as the Fmoc method, if the sequence of amino acids is known. The amino acids constituting the amino-acid sequence of a peptide are expressed by the following symbols: A represents alanine (Ala); C represents cysteine (Cys); D represents aspartate (Asp); E represents glutamate (Glu); F represents phenylalanine (Phe); G represents glycine (Gly); H represents histidine (His); I represents isoleucine (Ile); K represents lysine (Lys); L represents leucine (Leu); M represents methionine (Met); N represents asparagine (Asn); P represents praline (Pro); Q represents glutamine (Gln); R represents arginine (Arg); S represents serine (Ser); T represents threonine (Thr); V represents valine (Val); W represents tryptophan (Trp); and Y represents tyrosine (Tyr), respectively.

The phrase "to stimulate peripheral blood mononuclear cells using antigen" as used in this description means to add the antigen to the peripheral blood mononuclear cells and culture to react with lymphocytes. Preferably, the stimulation of peripheral blood mononuclear cells is performed in the wells of a microplate, preferably one having wells with a U-shaped bottom.

The phrase "not used directly" as used herein means avoiding direct addition of the antigen presenting cells to the peripheral blood mononuclear cells for stimulation.

The frequency of peptide stimulation has traditionally been once a week, over a few weeks (usually about three), and it has been thought that there is no need to do more. However, it is shown herein that frequent stimulation, for example, several times at intervals of two to five days, preferably induces antigen-specific T cells. Therefore, the "frequent" of frequent stimulation herein signifies multiple stimulations at a frequency of more than twice a week or at intervals of two to five days. It has been shown that induction of antigen-specific T cells by frequent stimulation enables detection in a relatively short period of time, about 13 days, compared to traditional techniques. Therefore, "multiple times" herein signifies stimulation in a certain time period of less than 15 days, preferably less than 13 days. Stimulation for more than 15 days does not significantly improve the effect of the invention, which diminishes the need for it from the standpoint of medical treatment. If used multiple times, for example stimulated at an interval of 3 days, the total number of stimulations is four, including the initial stimulation. Multiple stimulations can be performed by exchanging a portion of the culture supernatant with the same amount of fresh lymphocyte medium containing the antigen.

### (3) Detection of Antigen-specific T Cells in Stimulated Peripheral Blood Mononuclear Cells

The detection of the antigen-specific cells can be performed by various methods. One such method is, to react the post-stimulated antigen-specific T cells to the appropriate targeted cells, thereby quantifying the cytokine produced by the T cells, preferably IFN-γ. However, the invention is not limited to this method and can be applied to any method that can detect antigen-specific T cells unless the method adversely affects the detection process.

To prepare the targeted cells, first, the HLA gene which matched the HLA of the antigen-specific T cells was introduced to various cell lines not expressing those antigens, and was expressed stably. In other words, cells, which do not express antigens, in which the cDNA expressing plasmid of the HLA gene which is matched with HLA of antigen-specific T cells, can be employed as target cells. Various cell lines expressing HLA matched with antigen-specific T cells but not expressing antigens can be also used for the target cells.

Using the antigen or antigen peptide used for the stimulation of peripheral blood mononuclear cells, the prepared cells are sensitized as antigen-presenting cells. Peptides other than the antigen or antigen peptide used for the stimulation of peripheral blood mononuclear cells, sensitize the prepared cells which are used as target cells as a control.

Cytokine levels, or better still INF-γ levels, can be quantified after the reaction with cultured peripheral blood mononuclear cells using a method such as ELISA or FLISA, and the production of desired antigen-specific T cells can be assessed by comparison with the control.

The advantage of quantification of cytokines, especially INF-γ is that INF-γ is produced by various subtypes of lymphocytes, such as CD4⁺, CD8⁺, NK, LAK cells, and the quantification has been widely used to monitor immune reaction in many clinical studies of cancer vaccines because the INF-γ possesses a variety of biological characteristics, for example, immunomodulation activity, antivirus activity, increased expression of cell surface antigens like HAL molecules, and is secreted after stimulation with antigens.

The ELISA method is widely used for the quantification of cytokines including INF-γ and can be used here. FLISA is a method which quantifies cytokines including INF-γ, based on the fluorometric microvolume assay technology (FMAT) developed by the inventor of the present invention. The major characteristic of the FLISA method is that it does not need a washing process.

Fluorometric microvolume assay technology (FMAT) employs a unique macro-confocal imaging system using a helium/neon laser, which can automatically focus on, as well as scan the fluorescent beads present on the inside bottom surface of a multi-well tray. The focal depth of this system is smaller (approximately 100 microns) than the residue level, which makes the background fluorescence minimum against the fluorescence binding beads. Therefore, this system does not require the elimination of non-binding fluorescent groups, and all assays using FMAT are homogeneous. The linear dynamic range and sensitivity of the FLISA method is comparable to the traditional ELISA method.

### (4) Determination of Induction of Antigen-specific T Cells by Antigen Candidates

In another embodiment, the invention is a method of detecting antigen-specific T cells, in which the peripheral blood mononuclear cells collected from a patient are stimulated frequently with multiple antigen candidates without adding antigen-presenting cells and it is determined which of the candidates induced antigen-specific T cells.

This method is identical to the above method except that sample numbers of stimulation of collected peripheral blood mononuclear cells with antigen candidates increase according to the number of the candidates.

Determination of which of the antigen candidates induced antigen-specific T cells can be performed by reacting appropriate target cells with the post-stimulated antigen-specific T cells, quantifying cytokine, preferably IFN-γ, produced by the T cells, and comparing the levels.

As described above, the method of antigen-specific T cells according to the present invention may contribute significantly to the prevention and treatment of diseases such as various cancers, virus infections and autoimmune disease, as well as the diagnosis of such diseases.

Furthermore, according to the present invention, allergic peptide-specific IgE response and type I allergy can be inhibited by vaccinating with a non-allergic peptide from an antigen which retains an allergic peptide.

Furthermore, the present invention provides a desensitization method using inhibition of allergic peptide-specific IgE response and type I allergy by vaccinating with a non-allergic peptide from an antigen which contains an allergic peptide.

### (5) Tailored Pharmaceutical Composition

The tailored pharmaceutical composition of the present invention signifies a pharmaceutical composition which can be tailored to an individual target disease in an individual patient. For example, the antigen included in the pharmaceutical composition of the present invention usually varies between patients, depending on their HLA type and the effectiveness of vaccine in the patients. According to the method of antigen detection of the present invention, using such an antigen enables preparation of these tailored pharmaceutical compositions because antigens specific to the target disease in a patient can be detected. The phrase "the target disease in a patient" used herein signifies a disease in which a certain kind of antigen, which differs between patients depending on their HLA or disease type, is working as a vaccine.

The tailored pharmaceutical composition of the invention may include multiple different equivalent antigens, as well as one antigen. This is because all cancer cells do not always express a same common tumor antigen; considering that two or more different tumor antigen peptides are presented on one cancer cell, one of the reason is that treatment using multiple different tumor antigen peptides may be more effective. In effect, there has been an effort to develop cocktail preparation of multiple peptides because the effect of only one peptide from a single tumor antigen is not adequate in melanoma (Int. J. Cancer, 66:162, 1996; Int. J. Cancer, 67:54, 1996).

Furthermore, in reason, the tailored pharmaceutical composition of the present invention can be formulated similarly in a cancer, wherein two or more different tumor antigens are presented, and in various cancers such as pancreatic, uterine cervix, prostate, and colorectal cancers.

The tailored pharmaceutical composition of the present invention may be a pharmaceutical composition to prevent or treat cancers, virus diseases, infections, and autoimmune diseases.

The pharmaceutical composition with an included antigen peptide as an active ingredient may be administered along with an adjuvant such as mineral oil to establish effective cell-mediated immunity, or may be administered in a granular form. An adjuvant reported in a literature (Clin. Microbiol. Rev., 7:277-289, 1994) or the like can be applied. There may be a liposome composition; a granular composition bound to beads several micrometers in diameter, and a composition bound to lipid. The administration method may include intracutaneous, subcutaneous, and intravenous injections. The dose of tumor antigen peptide of the invention or the derivatives thereof in a composition may be adjusted according to the disease to be treated, patient age, and body weight. However, the dose is usually 0.0001 mg - 1,000 mg, preferably 0.001 mg - 1,000 mg, more preferably 0.1 mg - 10 mg, which is recommended to be administered once every few days or weeks or months.

### (6) Treatment Method

In a further embodiment, the present invention relates to a method of treating a disease, enabling tailored medical treatment dedicated to a target disease in a patient, wherein the characteristic is the administration of a therapeutically effective amount of antigen related to the target disease of the patient.

In the present invention, the tailored medical treatment is matched to individual patients, who may be different in many characteristics. According to the antigen detecting method in the present invention, it is possible to detect a specific antigen effective for the treatment of a target disease in a patient, which makes it possible to perform tailored medical treatment using that antigen.

The method of the invention is a method to enable tailored medical treatment dedicated to a target disease in a patient, wherein one or more antigens, related to the target disease in the patient and selected according to the detection method of the invention, are administered. This method enables clinical selection of the most effective antigen from among several selected antigens. Therefore, the treatment method of the invention has the characteristic that it administers a therapeutically effective amount of the antigen selected. In the present invention, the phrase "therapeutically effective amount" is the dose that may treat a target disease, or prevent development of symptoms or improve or diminish the developed symptoms, or decrease the characteristic signs of such symptoms.

The collection of the peripheral blood mononuclear cells, the frequent stimulation with the antigen candidates, and the induction of antigen-specific T cells by the antigen candidates are similar to those described above.

The dose and therapeutically effective amount of the antigen may be adjusted according to the disease to be treated, patient age, and body weight. However, the dose is usually 0.0001 mg - 1,000 mg, preferably 0.001 mg - 1,000 mg, more preferably 0.1 mg - 10 mg, which is preferably administered once several days or weeks or months. The administration method was described above.

The following examples are provided to illustrate the invention in detail, and are not intended to limit the scope of the invention.

### Example 1

### (Detection of Antigen Peptide-specific T Cells Using an EB Virus-derived Antigen Peptide)

Most healthy people retain EB virus (EBV)-specific T cell precursors relatively frequently from a past EB virus infection. Therefore, we tried to detect the EB virus-specific T cell from peripheral blood mononuclear cells (PBMCs) of healthy donors with a history of EB virus infection with positive blood anti-EB virus antibody.

The PBMCs were isolated from the peripheral blood of HLA-A24 positive healthy people by the specific gravity centrifugal method using Ficoll Conray solution. The PBMCs were cultured in a medium wherein to 45% RPMI-1640, 45% AIM-V (GIBCO BRL Inc.), and 10% FCS, 100 IU/ml of interleukin-2 (IL-2), 0.1mM MEM nonessential amino acid solution (GIBCO BRL Inc., hereinafter called "NEAA") were added (hereinafter called lymphocyte medium). PBMCs were suspended in the lymphocyte medium at 5x10⁵ cells/ml and inoculated in a 96-well plate having U-shaped bottoms at 200 µl/well. To each of the wells, an HLA-A24 restricted antigen peptide from EB virus (sequence of amino acids: TYGPVFMCL) [J. Immunol., 158:3325-3334, 1997] was added at a concentration of 10 µg to stimulate the PBMCs. Three wells were prepared. PBMCs were cultured in an incubator (37°C, 5% CO₂). Three days after the beginning of the culture, 100 µl of the culture supernatant was suctioned off and eliminated, and an additional 100 µl/well of lymphocyte medium containing 20 µg/ml of antigen peptide was added to re-stimulate. A similar exchange of medium was performed to re-stimulate at 6 and 9 days after the beginning of culture. At 11 days after the beginning of culture, antigen peptide-specific T cell activity was detected. To C1R-A24 cells in which the HLA-A24 gene was introduced and stably expressed on the B lymphoblast cell line, C1R cells, 10 µg/ml of EB virus derived HLA-A24 restricted antigen peptide or, as a negative control, HIV derived HLA-A24 restricted antigen peptide (sequence of amino acids: RYLRDQQLIGI) [J. Immunol., 159:6242-6252, 1997] was sensitized for two hours, and were added to a 96-well plate at the ratio of 1x10⁴ cells/well to serve as antigen-presenting target cells. Half of the cultured PBMCs were added to each target cell sensitized with each peptide, which were mixed and cultured for 18 hours. The culture supernatant was collected, and the level of IFN-γ in the culture supernatant was quantified by the ELISA method. Accordingly, the difference between the reactivity of PBMCs to target cells sensitized with EB virus derived HLA-A24 restricted antigen peptide and the reactivity of PBMCs to target cells sensitized with HIV derived HLA-A24 restricted antigen peptide as the negative control was more than 300 pg/ml in the production level of IFN-γ. The antigen peptide used herein was prepared by the Fmoc method.

The above examination demonstrated that a culture of 1x10⁵ cells/well of PBMCs in a 96-well plate having wells with U-shaped bottoms, when stimulated with EB virus derived HLA-A24 restricted antigen peptide four times every two days, induced antigen peptide-specific T cells preferably.

### Example 2

### (Analysis of the Frequency of EB Virus Derived Antigen Peptide-specific T cells)

### (1) According to the procedure in example 1, the increase in frequency of EB virus derived HLA-A24 restricted antigen peptide-specific T cells was confirmed by limiting dilution analysis.

The PBMCs were prepared from the same healthy donor as Example 1 by the same procedure, and the following measurement of frequency of antigen specific T cells was performed with or without stimulating a portion of the PBMCs using an antigen peptide. Another portion of PBMCs were added to a 96-well plate having wells with a U-shaped bottom at a concentration of 1x10⁵ cells/well in a method similar to that in Example 1. These were cultured while being stimulated using EB virus derived HLA-A24 restricted antigen peptides four times in total every three days. The cultures were subsequently measured for the frequency of the antigen-specific T cells as described below.

The measurement of the frequency of antigen-specific T cells was performed according to the procedure below. A 96-well plate was prepared for each concentration of 400, 200, 100, 50, 25, and 12.5 PBMCs per well. At this point, radiated allogenic PBMCs (2x10⁵ cells/well) as feeder cells were added, and cloning medium (20% FCS, 1% NEAA, 100IU/ml of IL-2, and 10 µg/ml of PHA were added on top of the mixture of 25% RPMI1640 and 55% AIM-V).

After 13 days of culture, the possibility of the presence of antigen-specific T cells in each well was examined using as a criterion the difference between the level of IFN-γ produced by the reaction of antigen-specific T cells and target cells sensitized by EB virus derived HLA-A24 restricted antigen peptides, and the level of IFN-γ produced by the reaction of negative control target cells sensitized by HIV derived HLA-A24 restricted antigen peptide. The level of IFN-γ in the culture supernatant, produced by mixed culture of the cultured PBMCs and target cells for 18 hours, was measured using ELISA method, and a well was determined to be positive when the difference between target cells was more than 100 pg/ml; otherwise, a well was determined as negative. These data obtained were analyzed by the least χ² method under the condition of a 95% confidential interval. The frequency of antigen-specific T cells was calculated by determining the cell concentration when 37% of wells were negative (J. lmmunol. 126:1614-1620,1981).

As a result, the frequency of EB virus derived HLA-A24 restricted antigen peptide-specific T cells in PBMCs not stimulated with antigen peptide was less than 1/30,000 of the detection limit; and the frequency of EB virus derived HLA-A24 restricted antigen peptide-specific T cells in PBMCs that were stimulated with antigen peptide according to the method in Example 1 was 1/5,639.

Based on the above examination, it was demonstrated that the technique of Example 1 preferably induces T cells specific to an antigen peptide.

### Example 3

### (Peptide stimulated CTLs and their Specificity)

To isolate PBMCs, blood samples collected from healthy donors and patients were heparinized and PBMCs were obtained by density gradient electrophoresis. All donors of the blood samples were serologically HIV negative and HTLV-I negative.

Various densities of PBMCs were cultured by adding them to 96-well microplates having wells with a U-shaped or flat bottom with 200 µl of medium containing 10 µM of an antigen peptide. The medium used consisted of a composition of 45% RPMI-1640, 45% AIM-V, 10% FCS, 100 IU/ml of IL-2, and 0.1 µM of NEAA.

The antigen peptides used were the following:
EB virus derived peptide comprising HLA-A24 binding motif (sequence of amino acids: TYGPVFMCL) (SEQ ID No: 31);
EB virus derived peptide comprising HLA-A2 binding motif (sequence of amino acids: GICTLVAML) (SEQ ID No: 32);
Influenza virus (Flu) derived peptide comprising HLA-A24 binding motif (sequence of amino acids: RFYIQMCYEL);
HIV derived peptide comprising HLA-A24 binding motif (sequence of amino acids: RYLRQQLLGI);
Human T lymphocyte virus (HTLV-1) derived peptide comprising HLA-A2 binding motif (sequence of amino acids: LLFGYPVYV);
SART1₆₉₀₋₆₉₈ (SART1₆₉₀) (sequence of amino acids: EYRGFTQDF) (SEQ ID No: 17);
SART1₉₃₋₁₀₁ (SART1₉₃) (sequence of amino acids: DYSARWNEI) (SEQ ID No: 18);
SART2₁₆₁₋₁₆₉ (SART2₁₆₁) (sequence of amino acids: AYDFLYNYL) (SEQ ID No: 19);
SART2₈₉₉₋₉₀₇ (SART2₈₉₉) (sequence of amino acids: SYTRLFLIL) (SEQ ID No: 20);
SART3₁₀₉₋₁₁₈ (SART3₁₀₉) (sequence of amino acids: VYDYNCHVDL) (SEQ ID No: 21);
SART3₃₁₅₋₃₂₃ (SART3₃₁₅) (sequence of amino acids: AYIDFEMKI) (SEQ ID No: 22);
cyclophilin B (CypB₈₄₋₉₂) (CypB₈₄) (sequence of amino acids: KFHRVIKDF) (SEQ ID No: 23);
CypB_{84-92-2Y} wherein phenylalanine at position 2 of CypB₈₄ is substituted by tyrosine (sequence of amino acids: YHRVIKDF);
CypB₉₁₋₉₉ (CypB₉₁) (sequence of amino acids: DFMIQGGDF) (SEQ ID No: 24);
CypB_{91-99-2Y} wherein phenylalanine at position 2 of CypB₉₁ is substituted by tyrosine (sequence of amino acids: DYMIQGGDF);
Ick₂₀₈₋₂₁₆ (lck₂₀₈) (sequence of amino acids: HYTNASDGL) (SEQ ID No: 25);
Ick₄₈₆₋₄₉₄ (Ick₄₈₆) (sequence of amino acids: TFDYLRSVL) (SEQ ID No: 26);
Ick₄₈₈₋₄₉₇ (Ick₄₈₈) (sequence of amino acids: DVLRSVEDF);
ART4₁₃₋₂₀ (ART4₁₃) (sequence of amino acids: AFLRHAAL) (SEQ ID No: 28); and
ART4₇₅₋₈₄ (ART4₇₅) (sequence of amino acids: DYPSLSATDI) (SEQ ID No: 29).

At 1-6 day intervals, half of the medium was discarded and new medium containing equivalent peptide (20 µM) was added, and the plates were cultured for 13 days. Subsequently, cells were collected to study the level of IFN-γ produced in response to the peptide used.

In addition, the cytotoxicity of PBMC stimulated with EBV derived peptide to EBV transformed B cell lines and phytohaemagglutinin (PHA) activated T lymphocytes was examined by performing a 6-h⁵¹Cr release assay, which is within the public domain in the art (Yang, D., et al., Cancer Res., 59:4056, 1999). The 6-h⁵¹Cr release assay to study cytotoxicity was performed in the presence of 20 µg/ml of anti-CD8 monoclonal antibody, anti-CD4 monoclonal antibody, anti-HLA class I (W6/32) monoclonal antibody, or anti-HLA class II (DR) monoclonal antibody. The phenotype at the surface of cells was measured by the fluorescence antibody technique using anti-CD3 monoclonal antibody, anti-CD4 monoclonal antibody, and anti-CD8 monoclonal antibody bound to FITC, respectively.

Standard culture technique with peptide is described in the literature (Shichijo, S., et al., J. Exp. Med., 187:277, 1998; Yang, D., et al., Cancer Res., 59:4056, 1999; Kawano, K., et al., Cancer Res., 60:3550, 2000). In other words, PBMCs (2x10⁶) were cultured along with corresponding peptides using 2 ml of medium injected into a 24-well micro-culture plate for 6 days. These cells were stimulated on day 6 and day 14 using corresponding peptide, and then re-stimulated with radiated auto-APC and collected on day 21. The obtained cells were examined, by the ELISA method for the ability to produce IFN-γ which responds to a certain peptide.

### (Determination of Culture Protocol.)

To induce peptide-specific CTL, PBMCs were stimulated three times in a 24-well culture plate using corresponding peptide and cultured for 21 days (Kawano, K., et al., Cancer Res. 60:3350, 2000). Cell culture was used to determine the interval of peptide stimulation necessary for the induction of peptide-specific CTL (Table 3). PBMCs of HLA-A24 positive healthy donors were stimulated with EBV peptide or Flu peptide, and the effect of stimulation at an interval of 1-6 days was examined. For peptide re-stimulation, half of the medium was exchanged with new medium containing equivalent peptide (20 µM), and the cells were collected 24 hours after the final stimulation. The obtained cells were examined for production ability of IFN-γ which responded to CIR-A2402 cells pre-sensitized with stimulation peptide or HIV peptide as a negative control. The background level of production of IFN-γ which responded to CIR-A2402 cells, (100-200 pg/ml) was deducted.

### <Table 3>

As shown in Table 3, peptide stimulation at the interval of 3 days (protocol C) induced optimal production of peptide-specific IFN-γ in PBMCs sensitized in vitro. Peptide stimulation at the interval of 5 or 6 days (protocol E and F) generated more cells, but the number of CTLs produced by peptide-specific IFN-γ were smaller than in protocol C. The reactivity of CTL to SART 3 peptide used as a negative control was not induced in any of the experimental conditions applied. In protocol G, the production of peptide-specific IFN-γ was induced and 4x10⁶ cells were created, but 2x10⁶ PBMCs were needed initially and, furthermore, many more auto-PBMCs were needed as feeder cells in order to examine the reactivity to one kind of peptide. Based on these results, protocol C was found to be superior to the other protocols. In protocol G, radiated auto-PBMCs which were cultured in 24-well plates and pre-sensitized with peptide were used to re-stimulate. The result in the table shows the average of three rounds.

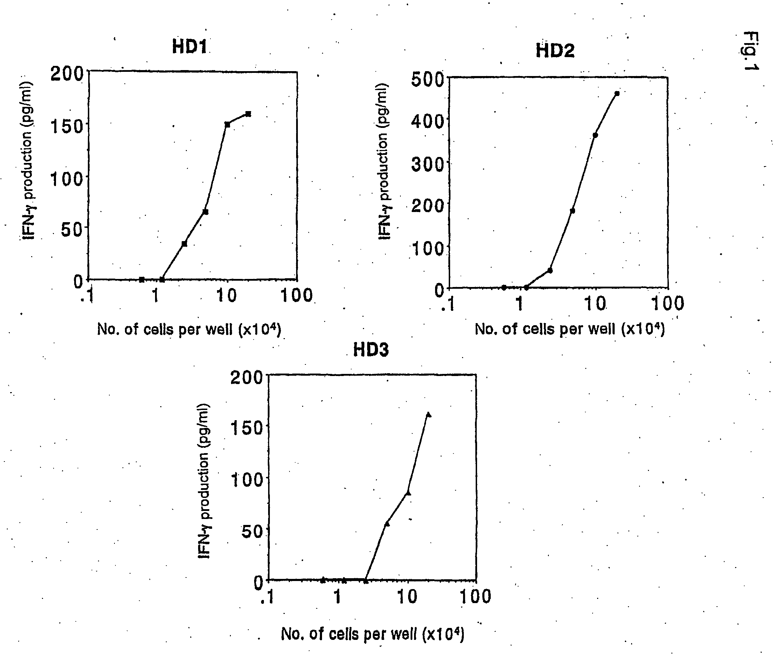

The optimal number of cells added to each well to induce CTLs which produce peptide-specific IFN-γ was determined (Figure 1). In this experiment, PBMCs of three healthy donors (HD1, HD2, and HD3) were used so that each well contained 6,250-200,000 cells. As a result of this experiment, it was found that the responsiveness to EBV peptide was detected when the number of PBMCs in each well was at least 25,000 to 50,000 cells. In all cases, a certain level of production of peptide-specific IFN-γ was observed when the number of cells per well was 100,000.

Furthermore, the efficacies of 96-well microplates having wells with a U-shaped bottom and those with a flat bottom were compared. When similar experiments were performed using both plates, the variation between the three assays was often significant with the 96-well flat-bottomed microplates. Therefore, it is preferable to use the 96-well microplates having wells with a U-shaped bottom.

### Example 4

### (Examination of Frequencies of Antigen Peptide Stimulation)

The influence of frequencies of antigen peptide stimulation was studied in the detection of antigen-specific T cells. Other than the change of stimulation frequencies, stimulation intervals, and culture period of EB virus derived HLA-A24 restricted antigen peptides, the study was performed just as in Example 1.

The conditions and the results of each experimental group are shown in Table 3.

Based on the result, it was demonstrated that stimulation with antigen peptide every two to five days enabled detection of antigen peptide-specific T cells.

### Example 5

### (Examination of the Number of PBMCs Used in Antigen Peptide Stimulation)

The influence of the number of PBMCs used in culture to the detection sensitivity of antigen-specific T cells was investigated.

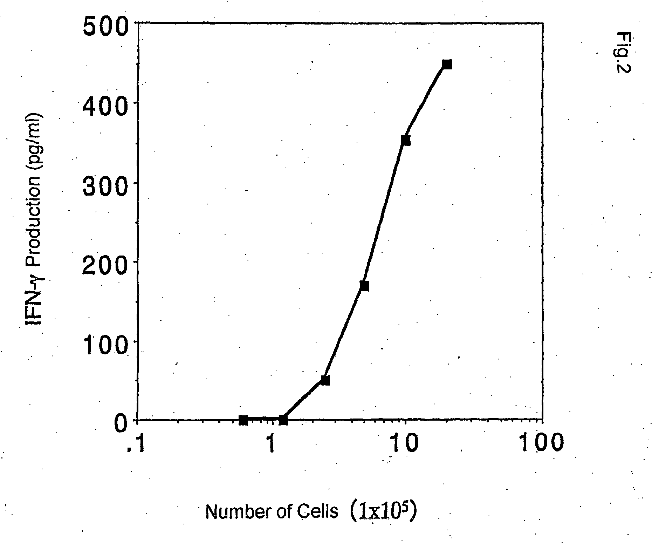

PBMCs were added at the ratios of 2x10⁵, 1x10⁵, 5x10⁴, 2.5x10⁴, 1.25x10⁴, 0.63x10⁴, and 0 cells per well. Radiated PBMCs were added to start the culture at 2x10⁵ cells/well for all batches. Other conditions were set to be similar to Example 1. Figure 2 shows the results. It was noted that the level of IFN-γ increased in proportion to the number of cultured PBMCs, which was detectable even at 2.5x10⁴ cells/well.

As evidenced by Figure 2, the number of PBMCs to be cultured is proportionate to the production of IFN-γ, which indicates that the final production of IFN-γ reflects the number of antigen-specific T cells at the beginning of culture. This means that the quantification of frequency of antigen-specific T cells is difficult in this study, but semi-quantitative measurement is possible.

### Example 6

### (Detection of Antigen-specific T Cells From PBMCs of Multiple Healthy Donors.)

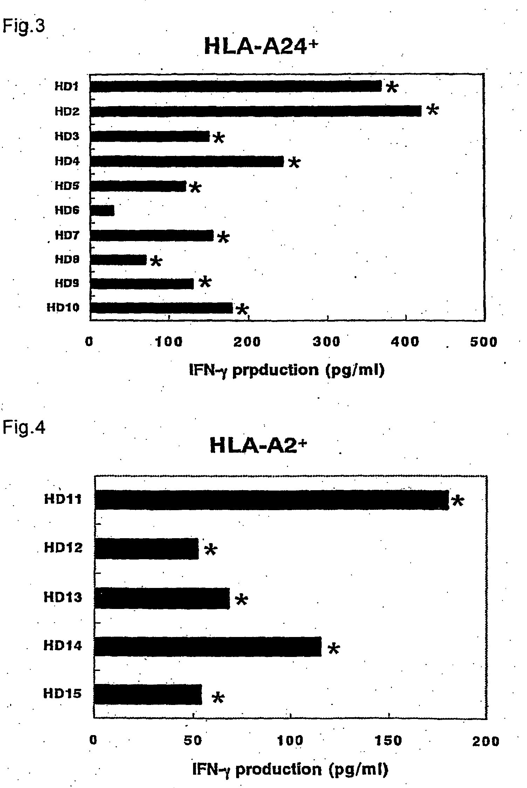

(1) PBMCs were isolated from the peripheral blood of 10 HLA-A24 positive healthy donors (HD1 or HD10), and the detection of peptide-specific T cells reactive to HLA-A24 restricted antigen derived from EB virus was attempted by a procedure similar to Example 1 at 1.5x10⁴ cells/well at the beginning of culture. Figure 3 shows the results. Although there is a difference in the production of IFN-γ, the HLA-A24 restricted antigen peptide-specific T cells from EB virus were detected in all 10 donors measured.
(2) According to the protocol C determined in Example 3, EBV specific CTL was induced from 5 HLA-A2 positive healthy donors (HD11 to HD15). The PBMCs of the donors (1x10⁵ cells) were cultured along with HLA-A2 restricted peptide (10 µM). On day 13, the cultured cells were investigated for the HLA-A2 restricted EBV peptide-specific production of IFN-γ, based on the presence of recognition of T2 cells pre-sensitized with EBV peptide or HTLV-1 peptide. Figure 4 shows the results. The results shown in the figure are the average of three batches of experiment. According to the two-tailed Student's t-test, the significant critical rate was equal to or less than at least 0.05.

Based on the result shown in Figure 4, it was found that EBV specific CTL was obviously induced for all but one blood sample of a donor.

### Example 7

### (Cytotoxic Assay.)

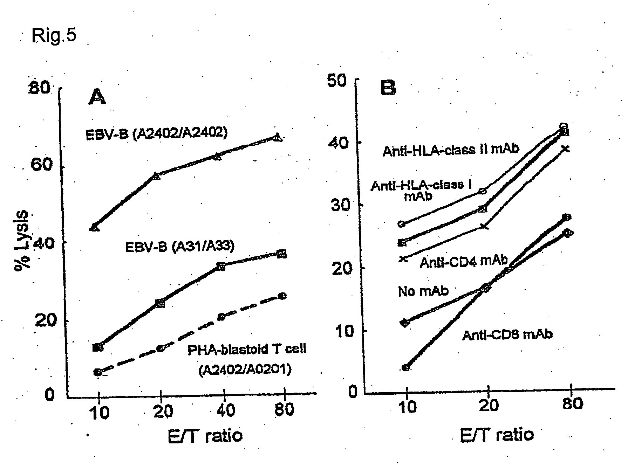

In Example 3, after stimulation by EBV peptide according to protocol C as previously described, PBMCs from three donors were proliferated with radiated allogenic PBMCs, PHA, and 100 IU/ml of IL-2, and the cytotoxicity was studied using ⁵¹Cr release assay. Figure 5 shows a typical result of a donor (HD2).

In Figure 5-A, PBMCs of peptide-stimulated HD2 were cultured for 2 weeks using allogenic feeder cells, PHA, and IL-2. The cytotoxicities to HLA-A24 positive EBV transgenic B cell line (EBV-B), HLA-A24 negative EBV-B, or HLA-A24 positive PHA blastoid normal T cells were then studied by 6-h⁵¹Cr release assay. Figure 5-B shows the case when 20 µg/ml of anti-class I (W6/32) monoclonal antibody, anti-CD8 monoclonal antibody, anti-CD4 monoclonal antibody, or anti-class II (H-DR-1) monoclonal antibody was added to wells at the beginning of the assay.

As is clear from Figure 5, the cytotoxicity to HLA-A24 positive EBV transgenic B cell line (EBV-B) was higher than that to HLA-A24 negative EBV-B or HLA-A24 positive PHA blastoid normal T cells. The cytotoxicity could be inhibited by adding 20 µg/ml of anti-class I (W6/32) monoclonal antibody or anti-CD8 monoclonal antibody, but not by anti-class II (H-DR-1) monoclonal antibody or anti-CD4 monoclonal antibody. Similar results were observed in two other healthy donors.

### Example 8

### (Flow cytometry analysis.)

The flow cytometry of PBMCs cultured according to protocol C determined in Example 3 was analyzed.

The PBMCs freshly isolated from donated blood or the PBMCs cultured with HLA-A24-restricted EBV peptide were examined by FACScan flow cytometry. The cells were stained with various combinations of fluorescence-labeled monoclonal antibodies before flow cytometry. The monoclonal antibodies used were FITC labeled anti-CD3 monoclonal antibody, FITC labeled anti-CD4 monoclonal antibody, PE labeled anti-CD8 monoclonal antibody, FITC labeled anti-CD14 monoclonal antibody, and PE labeled anti-CD86 monoclonal antibody.

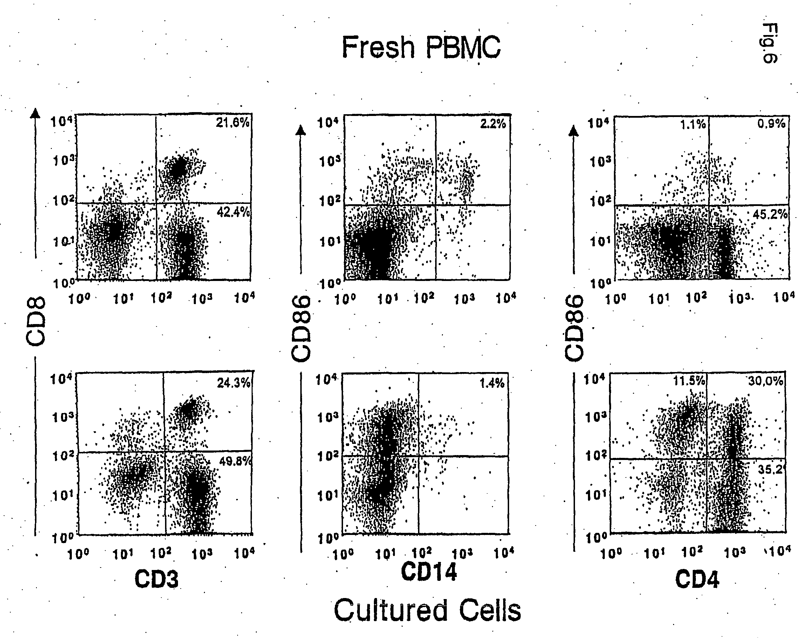

The phenotype of the cultured cells was examined by PBMCs stimulated with HLA-A24-restricted EBV peptide. Figure 6 shows a typical result. The numbers in the figure represent the ratio of subset of positive cells (%). It shows that there is essentially no difference in the positive rates of CD8 positive or CD4 positive T cells between newly isolated PBMCs and cultured PBMCs.

It was then investigated whether live APCs remained during the culture period because protocol C does not require addition of APCs during that time. Flow cytometry analysis was performed using culture cells derived from both attaching and non-attaching cells after culture in a 96-well plate having round-bottomed wells. As a result, it was found that roughly half of the culture cells expressed CD86, known as irritable APCs. A few of these cells were positive to CD14. This means that the monocyte derived cells survived during the culture. Most CD86 positive cells were positive to CD4, while others were negative against CD20 and CD8. These results show that CD86 expressing cells function as APCs in certain culture processes.

### Example 9

### (Tetramer Assay.)

The tetramer (peptide-HLA-A24 complex) was prepared according to the method described in the literature (Garbovzi, D.N., et al., Proc. Natl. Acad. Sci. USA, 89:3429, 1992).

Recombinant HLA-A2402 α chains containing a biotinated portion and recombinant β₂-microglobline were synthesized and used for refolding of soluble HLA molecules in the presence of HLA-A2402 binding peptides. The soluble HLA molecules were prepared for the following epitopes, HIV derived peptide (sequence of amino acids: RYLRQQLLGI) and EBV derived peptide (sequence of amino acids: TYGPVFMCL). Monomer HLA-peptide complex was biotinated with BirA in the α chain, which was isolated form free biotin by gel filtration. The biotinated HLA was tetramerized with 4:1 molar ratio of Strept-avidin-PE. PBMCs sensitized in vitro were re-suspended at the ratio of 5x10⁵ cells per 25 µl of phosphate buffer saline (PBS) containing 20% of human AB serum. The cells were allowed to stand at 37°C for 20 minutes with 1 µg of HLA-A24/EBV-peptide tetramer, and then for 20 minutes with 5 µl of FITC binding anti-CD8 monoclonal antibody (100 µg/ml). After that, the cells were washed with PBS and analyzed using FACScan. Twenty thousand events were obtained.

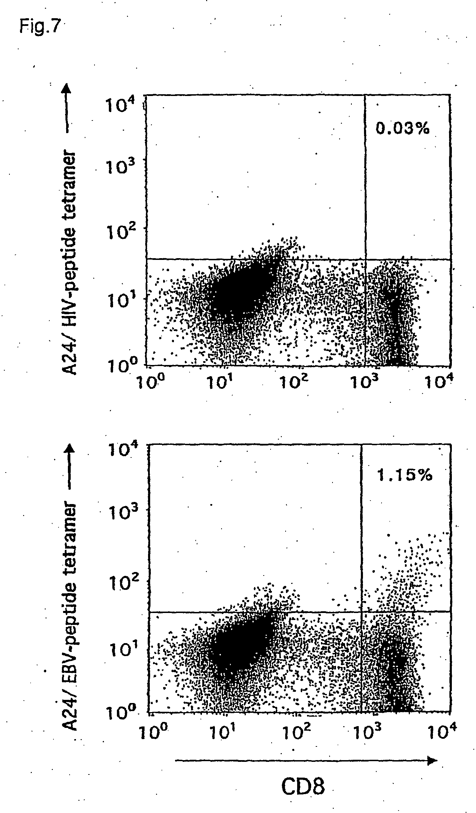

More specifically, PBMCs stimulated according to protocol C determined in Example 3 were analyzed by the tetramer assay as described above. Figure 7 shows the analytical results.

As shown in Figure 7, PBMCs of healthy donors, which were sensitized in vitro, were stained with anti-CD8 monoclonal antibody and anti-HLA-A24/EBV-peptide tetramer. The ratio of the CD8 positive and HLA-A24/EBV peptide tetramer positive subsets was 1.15%. In contrast, the ratio of the CD8 positive and HLA-A24/HIV peptide tetramer positive subsets was 0.03%. The ratio of the CD8 positive and HLA-A24/EBV peptide tetramer positive subsets in freshly isolated PBMCs was equal to or less than 0.01%. As shown in Table 3, these results show that the number of tetramer positive T cells obviously increases, considering that the added cells increased as much as 4.5-fold during the culture period.

### Example 10

### Frequency Analysis of CTL Precursor

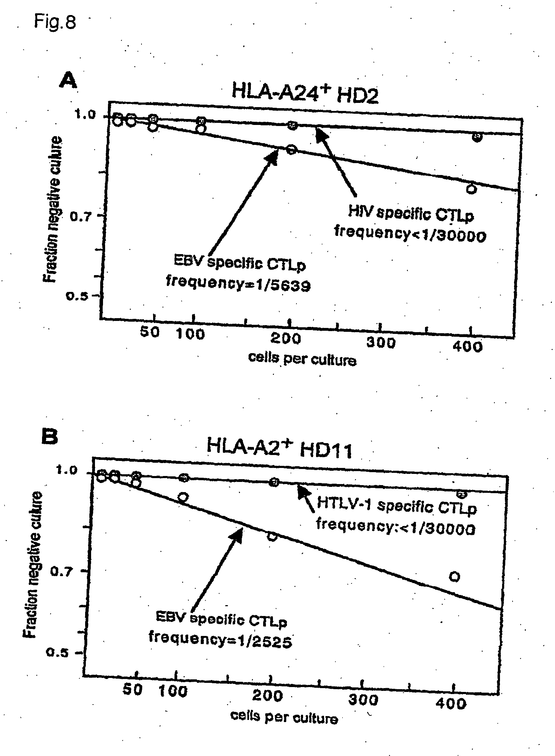

The detailed procedure to analyze the frequency of CTL precursor is described in the literature (Gomi, S., et al., J. Immunol. 163:4996, 1999; Harashima, N., et al., Eur. Immunol. 31:323, 2001).
In this embodiment, PBMCs of HLA-A24 positive healthy donors (HD2) and HLA-A2 positive healthy donors (HD11), sensitized with EBV derived peptide in vitro, were added to plates having U-shaped wells, so that the numbers of cells per well were 400, 200, 100, 50, 25, and 12.5. These were cultured using cloning medium (25% RPMI-1640, 55% AIM-V, 20% FCS, 100 IU/ml of IL-2, and 10 µl of PHA) in the presence of radiated PBMCs as feeder cells according to protocol C. The cells in each well were examined for IFN-γ production in response to target cells, on day 9 or day 15 of culture. Figure 8 shows the test results.

In the analytical results, a well was determined to be positive when effecter cells which produce IFN-γ in response to CIR-A2402 (or T2 cells) pre-sensitized with equivalent EBV peptide were found in a well at a higher level (>100 pg/ml) than that of C1R-A2402 (or T2 cells) pre-sensitized with HIV (or HTLV-1) peptide. The data obtained was analyzed by a minimum χ² test with a 95% confidence interval and the frequency of CTL precursors was calculated by the Taswell method.

### Example 11

### (Detection of Cancer Antigen Derived Peptide-specific CTL Precursor in Blood of Cancer Patients)

The above protocol C revealed that it is possible to detect peptide-specific CTL precursor to a single peptide by using only 3x10⁵ PBMCs in three assays, and this culture system was applied to detect cancer antigen derived peptide-specific CTL precursor in PBMCs of patients with cancer, using a series of peptides which is recognized by HLA-A24 restricted cancer-reactive CTLs (Yang, D., et al., ibid; Kawano, K., et al., ibid; Gomi, et al., ibid; Harashima, N., et al., ibid).

The average number of PBMCs obtained from 10 ml of blood sample was approximately 8 million cells. Therefore, it was possible to detect CTL precursor to at least 20 different peptides in three assays using only this small amount of blood.

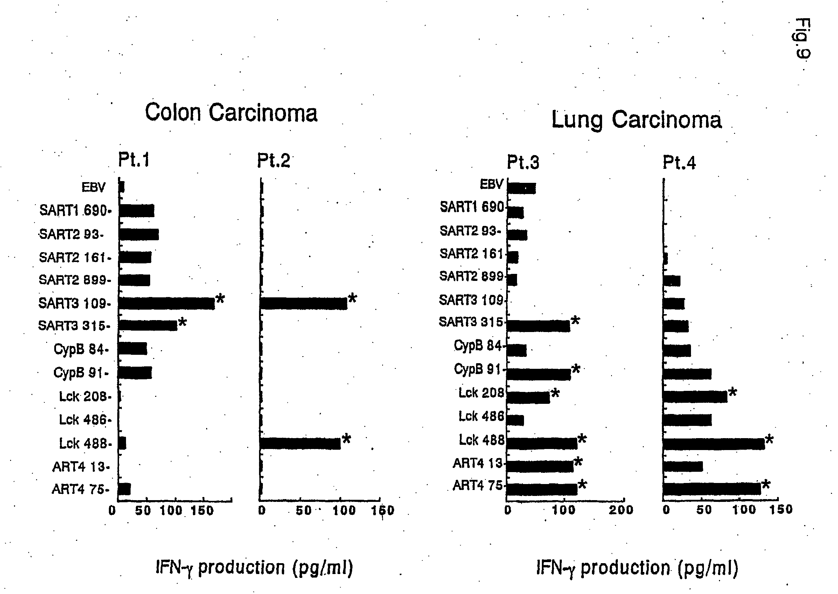

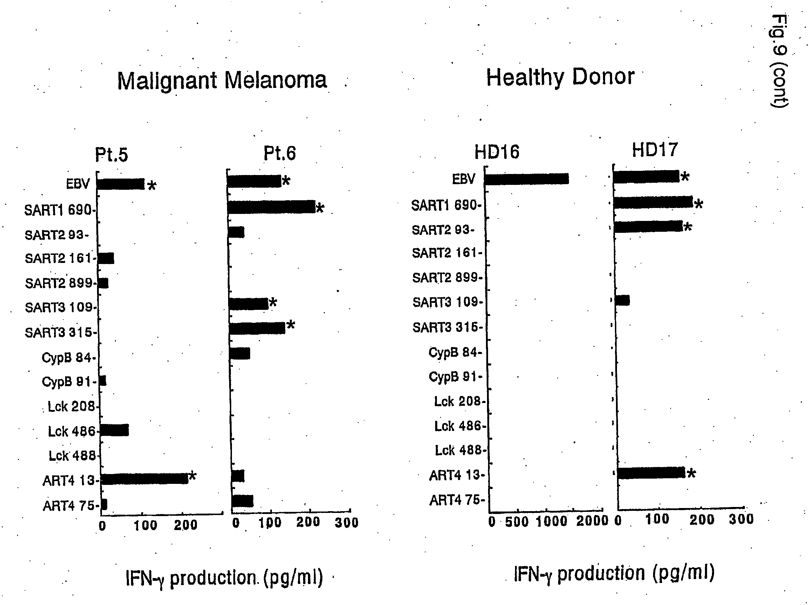

As shown in Figure 9, the PBMCs isolated from approximately 10 ml of blood collected from 6 HLA-A24 positive cancer patients and 2 HLA-A24 positive healthy donors were stimulated with a certain peptide, according to the above protocol C, and the cells cultured for 13 days were examined for the ability to produce IFN-γ in response to the target cells sensitized with the same peptide. Although none of these patients was prescribed a peptide vaccine, CTL precursor, which recognizes cancer antigen-derived peptide, was detected in all patients. In contrast, in a healthy donor (HD16), CTL precursor which responded to either antigen-derived peptide, was not detected; in another healthy donor (HD17), CTL precursor which responded to SART1₆₉₀₋₆₉₈, SART1₉₃₋₁₀₁, or ART4₁₂₋₂₀, was detected. These results showed that it would be possible to detect the presence of CTL precursor against a series of cancer antigen-derived peptide even before vaccination of peptide if there is 10 ml of blood.

### Example 12

### (Examination of Micronization of Study Procedure)

In the above embodiment, the use of a 96-well plate having U-shaped wells was examined, but 384-well plate was later used and examined to build a system which can measure more samples.

PBMCs of HLA-A24 positive healthy donors were added to the 384-well plate along with 80 µl/well of medium at the rate of 5x10⁴ cells/well, and stimulated with a peptide by the exchange of half of the medium (peptide stimulation, five times in total), and the detection of EB virus derived HLA-A24 restricted antigen peptide-specific T cells was attempted based on the same procedure as Example 1. As a result, the difference of reactivities to target cells sensitized with EB virus derived HLA-A24 restricted antigen peptide and target cells sensitized with HIV derived HLA-A24 restricted antigen peptide as a negative control was 2775 pg/ml of IFN-γ production. Thus, it was shown that the antigen-specific T cells are detectable even if the assay system is micronized.

### Example 13

### (Quantification of IFN-γ by FLISA Method.)

### 1. System

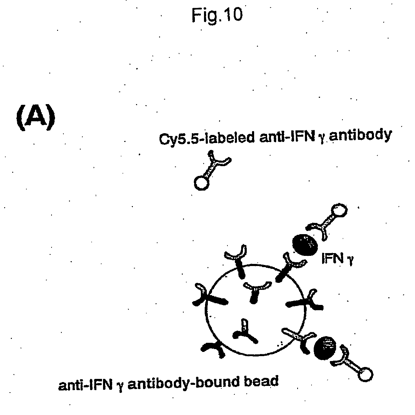

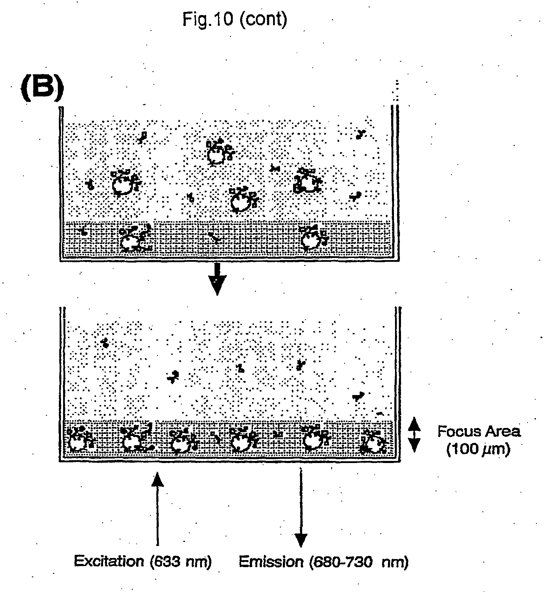

An FMAT system from Applied Biosystems equipped with a bar-code reader and robot plate handler (Zymark), and capable of handling 60 plates was set up. A helium/neon laser (633 nm) was used and the topology of the bottom of a microplate was mapped. The laser scanned the region of 1 mm x 1 mm x 100 µm (thickness) 256 times and was designed so that the generated fluorescence passes along the same optical path as the excitation beam and beam splitter for the detection by the photomultiplier in the filter (Figure 10).

### 2. Antigen Coating Beads

4 µg of anti-human IFN-γ monoclonal antibody (M700A, ENDGEN) was diluted with 800 µl of PBS solution (0.01% NaN₃). To this solution, 200 µl of goat anti-mice IgG (Fc) polystyrene beads (0.5% w/v; 6 µm diameter beads, Spherotech) was added, and the whole was incubated for 18 hours at room temperature while agitating slowly. After the solution was centrifuged at 8000 x g for 2 minutes, the supernatant was eliminated, and the beads coated with the resultant monoclonal antibody were re-suspended in 1 ml of the above PBS solution. The resultant beads were repeatedly washed and stored in the above PBS solution (8 x 10⁶ beads/ml) at 4°C.

### 3. Binding of Cy5.5 to Anti-human IFN-γ Monoclonal Antibody

Anti-human IFN-γ monoclonal antibody M-701 separate from the above was labeled using a Cy5.5 labeling kit (Amersham Pharmacia Biotech). In other words, 1 mg of the above anti-human IFN-γ monoclonal antibody was dissolved in 1 ml of 50 mM phosphate buffer (pH 7.0), and then 5 µl of coupling buffer was added to 100 µl of the above antibody solution and the mixture was agitated completely. To the resultant antibody solution, Cy5.5 fluorescent dye was added and the whole was left to stand for one hour at room temperature, and subsequently the free dye was isolated by gel filtration column chromatography.

### 4. Fluorometric Microvolume Immunoassay of IFN-γ

50 µl of the sample and IFN-γ standard solution were respectively placed on a 96-well plate, to which 50 µl of the above bead mixture (antibody beads: Cy5.5 antibody: FLISA buffer (PBS, 1% BSA, 0.35 M NaCl, 0.1% Tween-20, 0.01% NaN₃) = 70:5:5000) was added, in which the sample or IFN-γ standard solution was added and mixed. The resultant mixture was allowed to stand in a dark place for 4 hours at room temperature. After the standing, the 96-well plate was scanned with an FMAT scanner and the average fluorescence per bead was recorded.

### 5. Preparation of Standard Curve of IFN-γ

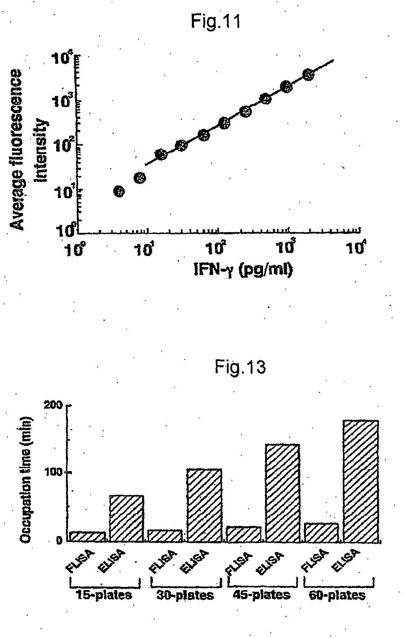

Cy5.5-binding anti-human IFN-γ monoclonal antibody and anti-human IFN-γ monoclonal antibody-binding beads were mixed with each sample and left to stand in a dark place for 3.5 hours at room temperature. The beads were bound to fluorescent dye through IFN-γ and moved at the bottom of each plate. An FMAT scanner was used to detect fluorescence bound to the beads by laser scanning. The standard solution of IFN-γ was gradually diluted twice with a mixture of RPMI-1640 and 10% FCS, and the standard curve of IFN-γ was created using an FMAT scanner (Figure 11A). The linear dynamic range of IFN-γ by FLISA was 15.6 - 2,000 pg/ml (2.1 log range). As stated above, the FLISA method gives high stability in a range of relatively high concentrations, while the ELISA method gives high stability in a range of relatively low concentrations (10 - 1,000 pg/ml). Furthermore, the strength of bead-binding fluorescence increased in accordance with the concentration of IFN-γ (Figure 11B).

### 6. Measurement of IFN-γ in Culture Samples

To study whether IFN-γ in a culture sample can be accurately quantified, the level of IFN-γ produced by cytotoxic T lymphocytes (CTLs) stimulated by various cancer related peptides was measured by both the FLISA and ELISA methods. Figure 12 shows the result. The average standard deviations (SD) of the same sample measured by ELISA and ELISA methods were 88 and 65, respectively. No significant difference was observed for either result. The correlation coefficient between FLISA and ELISA methods was 0.967. As a result, the FLISA method was able to quantify IFN-γ accurately in a biological sample like IFN-γ in CTL medium when CTL medium was used. However, the sensitivity of measurement was higher with FLISA buffer (1% BSA, 0.01% NaN₃ in Tween-PBS) than with RPMI-1640 mixture (RPM-1640 + 10% FCS). In the FLISA method, the results of similar treatment in a 384-well plate were essentially the same as the results in a 96-well plate. Changing the final volume of mixture of the sample and beads to 25 µl reduced time needed to culture to 2 hours.

### 7. Comparison of Operation Time Needed for the IFN-γ Measurement by FLISA and ELISA Methods

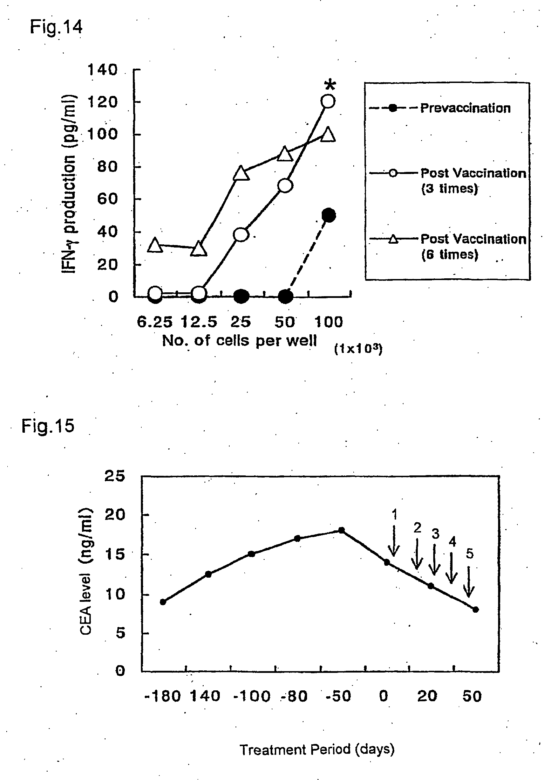

Figure 13 shows the result of comparison between FLISA and ELISA methods on operation time needed for the measurement of IFN-γ. In the comparison experiment, the time spans needed to measure 15, 30, 45, and 60 plates by FLISA and ELISA methods (except reaction time) were compared. As is clear from Figure 13, the measurement time of the FLISA method was roughly 1/6 that of the traditional ELISA; it was found that the FLISA method is more effective in reducing measurement time markedly and improving operation efficacy compared to the ELISA method. FLISA also significantly reduced the measurement time per plate compared to ELISA; it was found to contribute to significant improvement in the efficiency of measurement. Such a difference in measurement time can be attributed to the fact that FLISA is a homogeneous assay which does not require a washing process, while ELISA is a non-homogeneous assay which requires a washing process.

Furthermore, the FLISA method allows further reduction of culture time by using a 384-well plate, contributing to a significant overall reduction of measurement time of IFN-γ. When a 384-well plate is used, the volume of a mixture of a sample and beads in each well is just 20 µl.

### Example 14

### (Kinetic Analysis of Peptide-specific CTL Response)

In patients with HLA-A24 positive lung cancer, IFA (montanide ISA-51, Seppic, France) emulsion containing 1 mg of CypB91-92-2Y peptide was subcutaneously injected into the anterior femoral region at intervals of 2 weeks. To investigate the immune response, approximately 10 ml of peripheral blood was collected before initial vaccination and 7 days after the 3rd and 7th vaccination. PBMCs were isolated by the Ficoll-Hypaque isolation method and refrigerated until they were used in the assay. After being thawed, the PBMCs were stimulated with CypB91-92-2Y peptide in vitro, and it was investigated whether they had produced IFN-γ in response to HLA-A24 positive 11-18 lung adenocarcinoma cells and HLA-A24 positive QG56 lung squamous cells. Figure 14 shows the results.

As shown in Figure 14, HLA-A24 restricted and lung cancer reactive CTL precursor was easily detected in the PBMCs of patients with lung cancer by vaccinating with the peptide three or six times. These results show that the culture protocol of this invention is useful in the kinetic analysis of the CTL precursor in cancer patients.

### Example 15

### (Cancer Vaccine Treatment of Patients With Cervical Cancer - Part 1)

### (1) Selection of a Peptide to Be Used

Peripheral blood mononuclear cells were isolated from HLA-A2 positive patients with cervical cancer and frequently stimulated with 13 of 16 antigen peptides shown in Table 4 in the same manner as described in Example 1, and the reactivity of specific T cells against each antigen was studied.

### <Table 4>

Lymphocytes were cultured in 4 wells per each peptide, and the cells in each well were divided equally into two portions for the measurement of IFN-γ production to measure 8 samples. The production level of IFN-γ produced by the cultured lymphocytes in response to HLA-A2 positive T2 cells sensitized with each peptide (ATCC number, CRL-1992) (hereinafter called as antigen-peptide-T2 cells) and T2 cells sensitized with HLA-A2 restricted HIV derived antigen peptide (sequence of amino acids: SLYTYATL) (hereinafter called HIV-T2 cells), was quantified, and a *t*-test was used to determine if the difference is significant. Figure 5 shows the results.

### <Table 5>

The production level of IFN-γ in the table is the average of the value of two wells wherein the level of IFN-γ produced in response to HIV-T2 cell is subtracted from the level of IFN-γ produced in response to each antigen peptide-T2cells. The values are represented as <0 pg when the difference is smaller than 0 pg/ml. The criteria to select a peptide to be treated as a cancer vaccine are the following. A peptide was selected as a cancer vaccine when the level of IFN-γ produced in response to each antigen peptide-T2 cell was greater than the level of IFN-γ produced in response to HIV-T2 cells, and the level of IFN-γ produced in response to the antigen peptide-T2 cells, wherein more than one well was p<0.05 in a significant difference test, or the level of antigen peptide-T2 cells was greater than the level of IFN-γ produced in response to HIV-T2 cells, and the difference of IFN-γ production in response to the antigen peptide-T2 cells or HIV-T2 cells is more than 100 pg/ml in a well. In this patient with cervical cancer, 4 kinds of peptides, ppMAPkkk₂₉₄, ppMAPkkk₄₃₂, WHSC2₁₀₃, and HNRPL₅₀₁, which present frequently in antigen peptide-specific T cells and react preferably, were selected based on the above criteria.

### (2) Clinical Treatment Effect

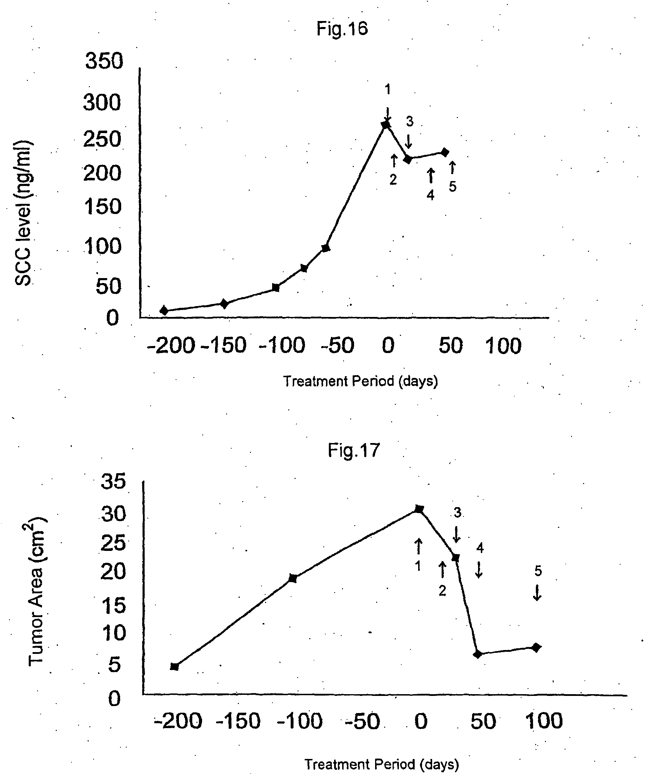

W/O emulsions of the 4 peptides selected above were prepared using Montanide ISA52 (Seppic), and 3 mg of each emulsion was subcutaneously administered to patients with cervical cancer five times in total every two weeks. Figures 15 and 16 show the shift in the measurements of tumor markers, carcinoembryonic antigen (CEA) and squamous cell carcinoma (SCC) related antigen, before and after the treatment. Figure 17 shows the reduction rate of the tumors. A trend was noted that the treatment with cancer vaccine decreased and stabilized the level of CEA and inhibited and stabilized the increase in tumor markers at high level. It was observed that tumors decreased 70% with vaccination. These results showed that a cancer vaccine using an antigen peptide selected by the new cell-mediated immune assay of the present invention is effective in the treatment of cancer.

### Example 16

### (Cancer Vaccine Treatment of Patients With Cervical Cancer - Part 2)

### (1) Selection of a Peptide to Be Used

Peripheral Blood mononuclear cells were isolated from HLA-A24 positive patients with cervical cancer, and the reactivity of 13 of 14 antigen peptides shown in Table 6 was studied in the same manner as described in Example 15.

### <Table 6>

Three wells of lymphocytes per peptide were cultured and cells of each well were divided equally into three portions for the measurement of the production of IFN-γ to measure 9 samples. The level of IFN-γ produced by the cultured lymphocytes in response to HLA-A24 positive C1R-A24 cells (hereinafter called antigen peptide-C1R-A24 cells) sensitized with each antigen peptide and C1R-A24 cells sensitized with HLA-A24 restricted HIV derived antigen peptide (sequence of amino acids: RYLRDQQLLGI; SEQ ID NO: 32) was quantified, and a t-test was used to calculate the p value to determine if the difference was significant,. Table 7 shows the results.

### <Table 7>

The production level of IFN-γ in the table is the average of the value of three wells wherein the level of IFN-γ produced in response to each HIV-C1R-A24 cell is subtracted from the level of IFN-γ produced in response to antigen peptide -C1R-A24 cells. The values are represented as <0 pg when the difference is smaller than 0 pg/ml. The criteria to select a peptide to be used as cancer vaccine are similar to those of Example 15. In the case of this cervical cancer patient, 4 kinds of antigen peptides, SART2₁₆₁, SART2₈₈₉, Ick₂₀₈, and ART4₃₄ were selected based on the above criteria. Furthermore, after more than 5 doses of cancer vaccine using these antigen peptides, peripheral blood mononuclear cells were isolated and the reactivity to 14 antigen peptides in Table 6 was examined according to the same method described above. Table 8 shows the results.

### <Table 8>

Based on these results, 4 kinds of antigen peptides, SART1₆₉₀, SART3₁₀₉, lck₂₀₈, and lck₄₈₈ were selected and used for the 8^{th} or later doses of cancer vaccine.

### (2) Clinical Treatment Effect

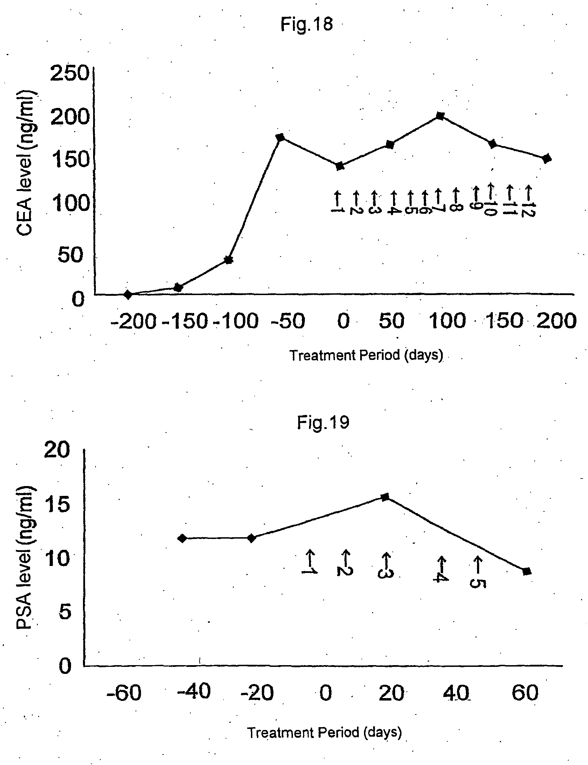

W/O emulsions of the 4 peptides were prepared using Montanide ISA51 (Seppic) in the same manner as described in Example 7, and 3 mg of the emulsion was subcutaneously administered to patients with cervical cancer every two weeks, respectively. Figure 18 shows the shift in measurement s of a tumor marker, carcinoembryonic antigen (CEA) before the treatment until the 12^{th} dose. A trend was noted that the administration of cancer vaccine inhibited the increase of CEA and stabilized it, albeit at higher level. The reactivity of peripheral blood mononuclear cells of patients before vaccination and after the 3^{rd} and 6^{th} dose to antigen peptide using vaccination was measured according to the method in Example 1. Table 9 shows the results.

### <Table 9>

There was no change in the reactivity to SART2₈₉₉ among the 4 antigen peptides, but an increase in reactivity to SART1₁₆₁, Ick₂₀₈, and ART4₇₅ was noted; it was suggested that the vaccination increased the frequency of antigen peptide-specific T cells. As stated above, the method of detecting cellular immunity is shown to be useful not only in the selection of antigen peptide used in cancer vaccine, but also in obtaining data to select a more effective antigen peptide or monitoring the T cell induction specific to the antigen peptide after the vaccination.

### Example 17

### (Cancer Vaccine Treatment of Patients With Prostate Cancer)

### (1) Selection of a Peptide to Be Used

Peripheral blood mononuclear cells were isolated from HLA-A2 positive patients with prostate cancer, and the reactivity of 13 of 16 antigen peptides shown in the above Table 4 was studied in the same manner as described in Example 15. Table 10 shows the results.

### <Table 10>

In this case, the overall reactivity to antigen peptides was low, but the antigen peptide was selected to administer only ppMAPkkk₃₂.

### (2) Clinical Treatment Effect

W/O emulsions of the peptide were prepared using Montanide ISA51 (Seppic) in the same manner as described in Example 15, and 3 mg of the emulsion was subcutaneously administered to patients with prostate cancer every two weeks. Figure 19 shows the shift in measurements of a tumor marker, prostate-specific antigen (PSA). The upward trend in PSA level was reversed after the 2nd administration of the vaccine, and the level was lower than that before treatment after the 5th vaccination. As stated above, the novel determination method of cellular immunity of this invention was shown to be useful, and to be easily able to select T cells which are effective in the treatment of patients and presented frequently in the bodies of patients from among many candidate peptides.

### Example 18

### (Treatment of Patients With Prostate Cancer)

In the progress of prostate cancer, hormone refractory prostate cancer (HRPC) represents the final stage, and effective treatment approaches are limited at this stage. Average life expectancy of HRPC patients is still approximately one year, and there is as yet no pharmaceutical composition to prolong this, so there is an urgent need to develop a new drug to treat HRPC.

To date, many cancer antigens recognized by CTL have been identified, and specific immune therapy using these peptides was performed in a patient with metastatic melanoma. New approaches to HRPC using cancer vaccines are also being investigated.

### (Selection of a Peptide)

Table 11 shows the peptides used in this embodiment.

### <Table 11>

Before the first vaccination and 7 days after every three vaccinations, 30 ml of peripheral blood was collected, and the PBMCs were isolated by Ficoll-Conray density-gradient centrifugation. After that, peptide-specific CTLs in the PBMCs were detected using the method of this invention. More specifically, PBMCs (1x10⁵ cells/well) were cultured along with 10 µM of each peptide in a 96-well microculture plate having U-shaped wells containing 200 µl of medium. The composition of the medium used consists of 45% RPMI-1640, 45% AIM-V, 10% FCS, 100 U/ml of IL-2 and 0.1 mM of NEAA. Half of the medium was drawn out every three days, and new medium containing the corresponding peptide (20 µM) was added. This operation was repeated until day 12. Twenty-four hours after the final stimulation on culture day 12, the resultant cells were collected and washed three times. The production of IFN-γ was subsequently determined in the stimulated HLA-A24 PBMCs which responded to CIR-A2402 cells pre-sensitized with either corresponding peptides or HIV peptides as a negative control. The target cells (CIR-A2402, 1x10⁴/well) were sensitized with each peptide (10 µM) for 2 hours, and then effecter cells (1x10⁵/well) were added to each well to make the final volume 200 µl. After 18 hours of culture, the supernatant (100 µl) was collected to measure the amount of IFN-γ by the ELISA method (sensitivity limit: 10 pg/ml). In all experiments, 4 wells were used and each assay was repeated twice.

All 14 peptides used were shown to be able to induce HLA-A24 restricted and cancer-specific CTL activity in the PBMCs of a cancer patient. The expression of 30 kinds of surface antigens of prostate cancer cells was screened to identify the 9 most extensively expressed antigens. Studies showed that the most suitable peptides for the use of a CTL precursor as a peptide vaccine vary according to the patient, because the profiling of positive peptide varies significantly from patient to patient. As an approach to immune therapy in patients with HRPC (Hormone-Refractory Prostate Cancer), the administration of up to 4 peptides was employed, which were positive in pre-vaccination measurements of peptide-specific CTL precursor in the circulation of cancer patients, and subsequently in pre-vaccination measurements among 14 vaccine candidates. In this embodiment, it was determined that peptide-specific CTL precursor was detected in 9 of 10 patients before vaccination with peptide, and cellular immune responses to HLA-A24 positive prostate cancer cells and to the peptide sensitized target cells expressed in 5 of 10 patients and 3 of 6 patients, respectively. The reason why the cancer-specific CTL precursor was preferentially induced in cancer patients may be that the level of this precursor in the circulation is different.

### (Screening of Peptide-Specific CTL Precursor)

Pre-vaccinated PBMCs were used for the screening of CTL precursor having reactivity to 14 peptides. The screening was carried out using 4 different wells per peptide and performing 2 assays for each. The result for each well was categorized in the following seven groups according to the p value and the amount of IFN-γ: Armed response (Ar): p <= 0.1 and 500 <= net; response level A (A): p<=0.05 and 50 <= net; response level B (B): p<=0.05 and 25 <= net <50; response level C (C): 0.05 < p <= 0.1 and 50 <= net; response level D (D): 0.05 < p <= 0.1 and 25 <= net <=50; response level E (E): 0.1 < p <=0.2 and 100 <= net; response level E' (E'): 0.2 < p <= 0.3 and 100 <= net. The peptide was selected according to the above order based on all evaluations of 4 wells. As a result, peptide-specific CTL precursor was detected in all 10 patients, and the average number of positive peptides in the 4 peptides was 2 to 5 peptides per patient (Table 12). All of these peptide candidates were submitted to immediate hypersensitivity reaction study when the result was negative, and up to 4 peptides were selected to vaccinate in vivo based on the above method. As a result, the peptide which was most frequently detected in the primary screening was SART2₉₃ (4/10), followed by ART1₁₇₀ (3/10), SART2₈₉₉ (2/10), SART3₁₀₉ (3/10), and Ick₂₀₈ (3/10).

### <Table 12>

### (Footnote of Table 12)

a ⓞ: The peptide was positive in the CTL precursor induction assay (Ar, A, and B are positive.)
b ( ): The peptide was positive in the CTL precursor induction assay, but was not administered due to the immediate hypersensitivity reaction in the skin test.
c Quadricate assay was performed to assess according to the following criteria (Up to 4 kinds of peptides were administered).
   Ar: p* ≤ 0.1 and 500 ≤ net**; A: p≤0.05 and 50 ≤ net;
   B: p≤0.05 and 25 ≤ net <50; C: 0.05 < p ≤ 0.1 and 50 ≤ net;
   D: 0.05 < p ≤ 0.1 and 25 ≤ net ≤50; E: 0.1 < p ≤0.2 and 100 ≤ net;
   E': 0.2 < p ≤ 0.3 and 100 ≤ net
   *p value (Student T)
   **The production level of specific IFN-γ was calculated by subtracting the response to HIV-derived peptide.
   AAAA: All 4 wells are positive in quadricate assay.
   AAA: Three wells are positive in quadricate assay.
   AA: Two wells are positive in quadricate assay.
   A: One well is positive in quadricate assay.

### (Vaccination Schedule)

For the skin test, 10 µg of 5 kinds of peptides was subcutaneously administered to each patient. Saline was used as the negative control. 20 minutes and 24 hours after injection, immediate and delayed type hypersensitivity (DHT) reactions were looked for. The skin test reaction was determined as positive if erythema and sclerosis were more than 10 mm in diameter. If the immediate hypersensitivity reaction was negative, a peptide was administered. Each peptide solution (3 mg/ml) was mixed with the same amount of IFA (Montanide ISA-51) and emulsified in a 5-ml plastic syringe, and 3 ml of the emulsion was subcutaneously administered to the lateral femoral region every two weeks. Serum PSA level was measured 4 weeks after vaccination. The time passed was defined as the time from the registration day to the date when the progress of the target disease was recorded. For patients who chose to discontinue the study without any progress of the target disease (e.g., increase in PSA) it was determined that the disease had progressed at the time the study was stopped.

### (Immune Response)

To investigate immune response, 30 ml of peripheral blood was collected before the 1^{st} vaccination and 7 days after each 3^{rd} vaccination, and the PBMCs were isolated and refrigerated at -195.8°C. Cell feature activity was measured by the standard 6-h⁵¹Cr-release assay method similar to the above embodiment. In other words, the refrigerated PBMCs were thawed and cultured in a medium (45% RPMI-1640, 45% AIM-V, 10% FCS, 100 U/ml IL-2, and 0.1 mM NEAA). On day 7 of the culture, the cells were collected for assay. To avoid fluctuation of bioassay, PBMCs collected at different points in the same patient were thawed simultaneously. The target cancer cell lines used in this embodiment were HLA-A2402 gene transfect CIR (CIR-A2402) cells, PC93 prostate cancer cell line, and HLA-A2402 gene transfect PC93 prostate cancer cells (PC93-A2402). In the same manner as the above embodiment, ELISA was used to detect the level of serum IgG specific to the peptide treated. The optical density (OD) of each sample was compared to those of standard samples sequentially diluted to estimate the level of peptide-specific IgG.

In this embodiment, immediate hypersensitivity reaction to one of the peptides selected was observed in 5 of 10 patients in the skin test before the 1^{st} vaccination. Peptide-specific delayed type hypersensitivity (DTH) reaction was observed in 6 patients after vaccination. Interestingly, 4 of 6 patients whose skin test to DTH reaction was positive exhibited a decrease in PSA level after vaccination, while only one patient whose skin test was still negative exerted no decrease in PSA.

Anti-peptide IgG was not detected in the pre-vaccinated serum of most patients (9 of 10 patients), but Cyp_{B84-92} was detected at low level in the post-vaccinated serum of only one patient. Anti-ART1₁₇₀₋₁₇₉IgG was detected at significant level in the post-vaccinated serum of 2 patients after the 6^{th} vaccination, and anti-SART3₁₀₉₋₁₁₈IgG was detected at high level in the post-vaccinated serum of 3 patients after the 3^{rd} or 6^{th} vaccination.

### (Clinical Response)

The clinical response of 10 patients was studied (Table 13). Only one patient responded partially with an 89% decrease in PSA. Table 13 shows the PSA level of the patient in this embodiment and during the observation period. 5 of 10 patients showed a stable condition during an average period of 2 months (2 to 5 months). Detectable lesions did not respond objectively.

This embodiment shows that the activated immune therapy using peptide vaccine to CTL precursor may be a new approach to immune therapy for prostate cancer. The result of the clinical study of this embodiment shows that this treatment approach is feasible, and the treatment is safe and immunologically active, and furthermore, it is clinically active.

### <Table 13>

### Example 19

### (Detection of Peptide, which inducible peptide Specific CTL, Used in Specific Immunotherapy of Pancreatic Cancer)

### (1) Patients and Cell Lines

Twenty ml of peripheral blood was collected from 10 patients with HLA-A24 positive pancreatic cancer and 5 patients with HLA-A2 positive pancreatic cancer (none of the patients were infected with HIV or HTLV-1), and PBMCs were obtained by the Ficoll-Conray density gradient centrifugation method. PBMCs were also obtained by collecting the blood of 5 HLA-A24 positive healthy donors and 5 HLA-A2 positive healthy donors. HLA-class I typing was performed by a traditional serologic method. The pancreatic cancer cell lines used in this embodiment were Panc-1 (HLA-A0201), paca-2 (HLA-A2402), YPK-1 (HLA-A2402), YPK-2 (HLA-A2402), YPK-3 (HLA-A0201), YPK-4 (HLA-A2601), HPAKII, SUIT2, and BxPC3. KE4 esophagus SCC cell line (HLA-A2402/A2601), from which SART1₂₅₉, SART2, and SART3 genes was cloned, was used as a positive control. HT1376 bladder carcinoma cell line (HLA-A2402), from which ART4 gene was cloned, was also used as a positive control. MKN-45 gastric adenocarcinoma cell line (HLA-A2402), which expressed SART2, was used as a negative control. SW 620 colon carcinoma cell line (HLA-A2402/0201) and phytohemagglutinin (PHA) blastoid T cells were also used as target cells.

### (2) Detection of Cancer Antigen

The expression of SART1₂₅₉, SART2, SART3, and ART4 of pancreatic cancer cell line, pancreatic cancer tissue, and non-cancerous pancreatic tissue was studied at the protein level by Western blot analysis using polyclonal antibody as known in the literature (Shichijo, S., et al., J. Exp. Med. 187:277-88, 1998 and others). Table 14 shows the results. The figures in the table represent the number of positive samples/total number of study samples.

### <Table 14>

Based on the results shown in Table 14, SART1₂₅₉ antigen was expressed in half of the examined cytoplasmic fraction of pancreatic cancer cell line and pancreatic cancer tissue, but not expressed in the non-cancerous pancreatic tissue at all. In contrast, SART1₂₅₉ antigen was not expressed in the cell nucleus fraction at all. These results agreed with other results on cancer (Shichijo, S., J. Exp. Med., 1999; 59:4056-63). SART2, SART3, and ART4 tumor rejection antigens were expressed in the tested cytoplasmic and cell nucleus fractions of most pancreatic cancer cell lines and pancreatic cancer tissue, but not in non-cancerous pancreatic tissue at all. These results also agreed with results found for other cancers (Nakao, M., at al., J. Immunol., 2001; 165:2565-74).

### (3) Peptide

Tables 15 and 16 show the peptides used in this embodiment. Table 15 exemplifies the peptides which induce peptide-specific PBMCs in HLA-A2 positive pancreatic cancer patients; and Table 16 exemplifies the peptides which induce peptide-specific PBMCs in HLA-A24 positive pancreatic cancer patients. All the peptides exemplified below except EBV derived peptide code tumor rejection antigen have the ability to induce HLA-A24 or HLA-A2 restricted CTL specific to cancer cells in PBMCs of cancer patients. HIV derived peptide comprising HLA-A24 binding motif (sequence of amino acids: RYLQQLIGI; SEQ ID NO: 32) and HTLV-1 derived peptide comprising HLA-A2 binding motif (sequence of amino acids: SLYNTYATL; SEQ ID NO: 33) were used as negative controls.

### <Table 15>

### <Table 16>

### (4) Detection of Peptide-specific CTL Precursors

According to protocol C shown in the above Example 3, peptide-specific CTL in PBMCs were detected as follows:

PBMCs (1x10⁵ cells/well) were cultured with each peptide using a 96-well micro-culture plate having U-shaped wells containing 200 µl of medium. The culture medium consisted of 45% RPMI-1640, 45% AIM-V, 10% FCS, 100 IU/ml of IL-2, and 0.1 M NEAA. Half of the medium was eliminated every three days for 12 days, and new medium containing the equivalent amount of a peptide (20 µM) was added. The cells were collected 24 hours after the final stimulation on day 12 of the culture and washed three times. The production of IFN-γ of HLA-A24 positive PBMCs in response to CIR-A2402 cells pre-sensitized with the corresponding peptide or in response to C1R-A2402 cells pre-sensitized with HIV peptide as a negative control was examined. When HLA-A2 was positive, T2 cells pre-sensitized with corresponding peptide or HIV peptide were used as target cells. The target cells (C1R-A2402 or T2, 1x10⁵ cells/well) were sensitized for 2 hours with each peptide (10 µM), and subsequently, effecter cells were added to each well to a total volume of 200 µl. After 18 hour of culture, the supernatant (100 µl) was collected to measure the production level of IFN-γ by ELISA (sensitivity limit 10 pg/ml). All experiments were repeated 4 times and a 2-tailed Student's t-test was used for statistical analysis. The detectable level of CTL precursor was determined as positive when the average production level of IFN-γ in response to the corresponding peptide was significantly higher (p<0.05) than that in response to HIV peptide.

Tables 17 and 18 show the results. The figures in the table are the average of 4 experiments with the production level of IFN-γ in response to HIV peptide subtracted as background. Pt stands for "patient" and HD stands for "healthy donor". The numbers double-underlined are the values determined as positive.

### <Table 17>

### <Table 18>

### (5) Cytotoxic Assay

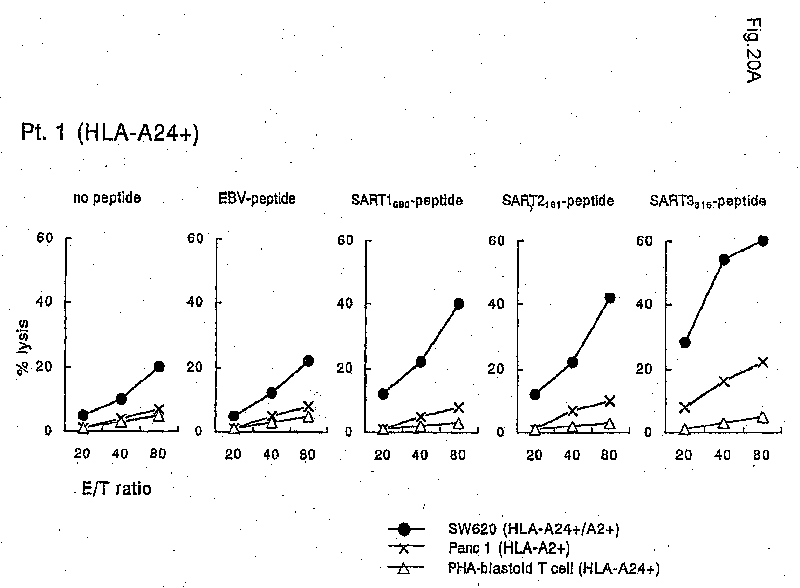

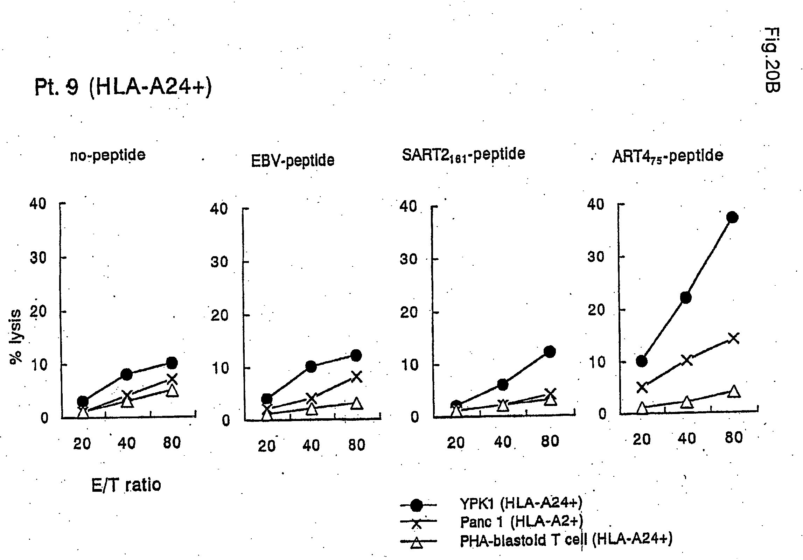

PBMCs stimulated with peptide were further proliferated in the presence of feeder cells, studied for cytotoxicity to various target cells by the above 6-h⁵¹Cr release assay, and measured for CTL activity in the presence of 20 µg/ml of monoclonal antibody (anti-CD8, anti-CD4, anti-HLA class I (w6/32), or anti-HLA class II (DR) monoclonal antibody). The phenotype of the surface of cells was measured by the fluorescence antibody technique using anti-CD3, anti-CD4, or anti-CD8 monoclonal antibody. Figure 20 shows the results.

In this embodiment, see the literature which was introduced above (Suzuki, N., et al., Int. J. Cancer: 98, 45-50, 2002) for details of the experimental data and analytical results.

### Example 20

### (Induction of Cellular Immune Response to Cancer Cells and Peptide in Patients with Colon Cancer Using SART3 Peptide Vaccine)

### (1) Selection of Peptide

The peptides used in this embodiment were SART3₁₀₉ and SART3₃₁₅. These two peptides have the ability to induce HLA-A24 restricted CTL activity in PBMCs of most cancer patients studied.

These peptides were dissolved in 6.7 mg/ml of DMSO and 200 mg/ml of trifluoroacetate, respectively, and stored at -80°C. For these stock solutions, SART3₁₀₉ was diluted with saline and SART3₃₁₅ was diluted with 0.12N NaOH-saline solution before use. The pH of SART3₃₁₅ solution was adjusted to between 8.0 and 8.2. HIV derived peptide and Ick₄₈₈ comprising HLA-A24 binding motif, which was shown to be HLA-A24 restricted and induce cancer-specific CTL, were used as negative controls.

### (2) Clinical Protocol

SART3 peptide vaccine was administered to 12 patients with progressive colon cancer (all adenocarcinoma: stage IIIa, IIIb, and IV, where surgery is impossible, and recurrence stage). Table 19 shows the results.

### <Table 19>

a) SART3 peptide was subcutaneously administered to each patient.
b) Number of vaccinations after sampling
c) Union International Contre Cancer -tumor Node Metastasis classification for malignancy was used to determine the clinical stage.
d) Toxicity Class Criteria of the Japanese Clinical Oncology Group was used for the classification of adverse events.
e) SD = Stable disease

All patients who entered to this study did not respond to chemotherapy using fluorouracil. The areas of cancer were located at the ascending colon (patients number 008 and 012), sigmoid colon (patient number 001), and rectum (the other 9 patients). All patients were confirmed to be HLA-A24 positive by staining PBMCs using traditional serological methods and anti-A24 monoclonal antibody. None of the patients had received treatment with steroid or other immunosuppressive drugs for 4 weeks before this experiment.

The following doses of peptide were administered: group 1 (4 patients) each received 0.3 mg of 2 peptides; group 2 (5 patients) each received 1 mg of 2 peptides; and group 3 (3 patients) each received 3 mg of 2 peptides. In group 1 and 2, 1.5 ml of the peptide (1 mg/ml) solution was mixed with the same volume of IFA (montanide) and emulsified in a 5-ml glass syringe for preparation. 0.6 ml and 2 ml of these peptide solutions were subcutaneously injected into the anterior femoral region of the patients in groups 1 and 2. In group 3, 2 ml of peptide (2 mg/ml) solution was similarly mixed with the same volume of IFA (montanide) and emulsified for preparation. 3 ml of this peptide solution was subcutaneously injected into the anterior femoral region of each of the patients in group 3. In this embodiment, the situation was regulated so that all patients received at least 3 doses of vaccine at intervals of 2 weeks.

To investigate immune response, 30 ml of the peripheral blood was collected before the 1^{st} vaccination and 7 days after the 3^{rd} vaccination. PBMCs were isolated from the collected blood and refrigerated.

### (3) Assessment of Responsiveness

All-known lesions were assessed 7 days after 3 doses of vaccination, and the findings were compared to those before vaccination. The responsiveness was categorized according to the responsiveness assessment criteria for solid cancer (revised WHO criteria).

### (4) Detection of Serum IgE and IgG Levels

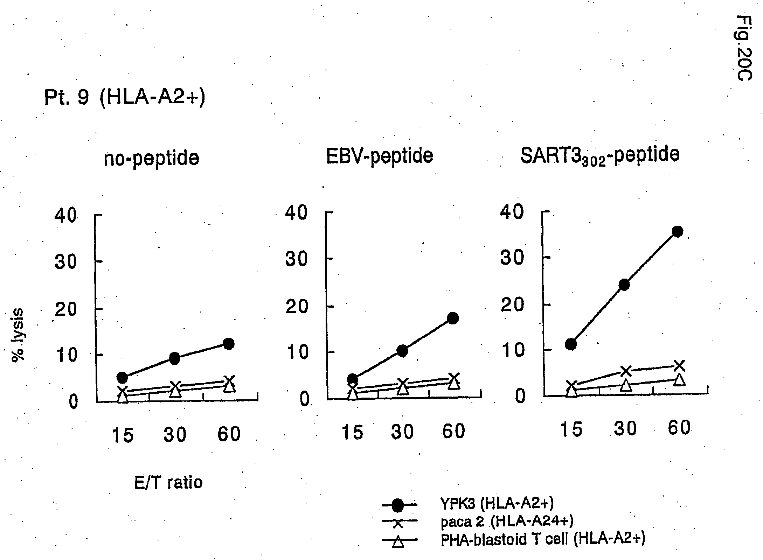

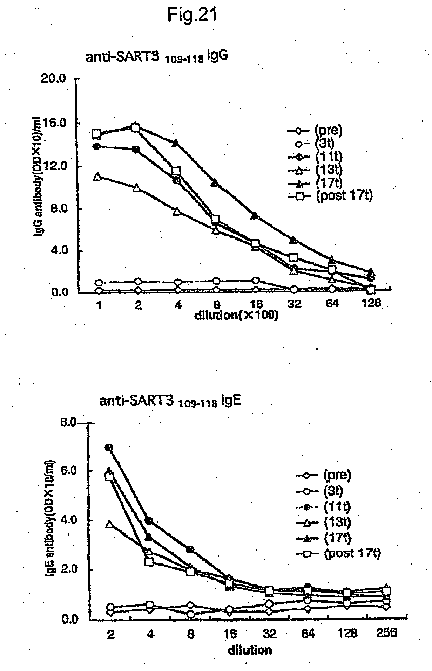

Serum IgE and IgG levels specific to SART3 derived peptide were detected using ELISA (Figure 21). In this Figure, OD:=optical density; pre=pre-vaccination; t=after sampling of vaccine (e.g., 3t=3^{rd} dose of vaccination; post17t=after 17^{th} dose of vaccination); post=post-vaccination.

A SART3 peptide fixed plate (20 µg/well) was blocked with Block Ace, washed with 0.05% Tween 20-PBS (PBST) to which serum or plasma sample diluted with 0.05% Tween 20-Block Ace was added. After letting it stand for 2 hours at 37°C, the plate was washed with PBST. Then 1:1000 diluted rabbit anti-human IgE (ε specificity: DAKO) or anti-human IgG (γ specificity: DAKO) was added and the plate was left to stand for 2 hours at 37°C. The plate was washed 9 times, and 100 µl of 1:100 diluted goat anti-rabbit immunoglobulin binding horseradish peroxidase-dextran polymer (EnVision; DAKO) was added to each well. The plate was then left to stand for 40 minutes at room temperature and washed. Subsequently, 100 µl of tetramethylbenzine (KPL, Guildford) was added to each well. After this, the reaction was terminated by adding 1M phosphoric acid. To estimate the level of peptide-specific IgE, the optical density of each sample was compared with those of standard samples consecutively diluted, and the values were recorded as the optical density. The level of peptide-specific IgG was estimated in the same way.

As shown in Figure 21, anti-peptide IgG was not detected in either pre- or post-vaccinated serum from most (10) of the 12 patients. Low levels of SART3₁₀₉IgG and SART3₃₁₅IgG were detected in the pre-vaccinated serum of only one patient (number 011). This value was not significantly changed in post-vaccinated serum (after 3 doses of vaccination). In the post-vaccinated (after 11^{th} and 13^{th} vaccination of two kinds of vaccines) serum of another patient (number 006), high levels of SART3₁₀₉IgG and SART3₃₁₅IgG were detected. Anti-IgE was not detected in either pre- or post-vaccinated blood in 7 of 12 patients. Low levels of SART3₁₀₉IgG and SART3₃₁₅IgG were detected in pre- and post-vaccinated serum of one patient (number 009). In 4 patients (numbers 001, 002, 006, and 008) low levels of SART3₁₀₉IgG and in 2 patients (numbers 001 and 008), low levels of SART3₃₁₅IgG were detected in only post-vaccinated serum.

### (5) Assay for Cell-Mediated Immunity

Refrigerated (Frozen) PBMCs were thawed in complete medium consisting of 45% RPMI-1640, 45% AIM-V, 10% FCS containing 100 U/ml of IL-2, and 0.1 mM NEAA. To avoid deviation from bioassay, PBMCs collected from one donor at various points were simultaneously thawed. Peptide induced cellular immune response was measured using 3 different methods. In the first 2 methods, PBMCs were washed once, re-suspended to a cell concentration of 10⁶ cells/ml, and cultured in vitro for 7 days, with or without the corresponding peptide used in in-vivo vaccine injection, in the wells of a 24-well culture plate (2 ml/well). For 6-h⁵¹Cr release assay, cells were further cultured in the wells of a 96-well micro-culture plate having U-shaped wells (Nunc) in the presence of feeder cells comprising radiated HLA-A24 positive allogeneic PBMCs (2x10⁵ cells/well) (from three healthy donors), without any peptide. On day 21 or 26 of the second culture, the surface phenotype and cytotoxic activity of these proliferated effecter cells were examined by 6-h⁵¹Cr release assay. The target cells were HLA-A24 positive SW620, HLA-A24 negative Colo201 tumor cells, VA13 fibroblasts, and HLA-A24 positive PHA activated normal T cells. HLA-A24 positive KE4 esophageal cancer cells, from which the SART3 gene was isolated, and HLA-A24 negative QG56 lung cancer cells were also used as target cells. The phenotype of PBMCs was determined by the fluorescence antibody technique using FITC binding anti-CD3, anti-CD4, or anti-CD8 monoclonal antibody. For the inhibition test, 20 µg/ml of anti-HLA class I (W6/32, IgG2a) monoclonal antibody, anti-CD8 (NUTs/c, IgG2a) monoclonal antibody, anti-HLA class II (H-DR, IgG2a) monoclonal antibody, and anti-CD4 (NU-Th/l, IgG1) monoclonal antibody were used.

The frequency of CTL precursor of the same PBMCs was analyzed on day 7 of in vitro culture, according to the method described in the literature (Harashima, N., et al., Eur. J. Immunol., 31:323-332, 2001). In this method, various numbers of PBMCs (1-400 cells/well) were added to each well of a 96-well micro-culture plate having U-shaped wells, and cultured using cloning medium (25% RPMI-1640, 55% AIM-V, 20% FCS, 100 U/ml of IL-2, 10 µg/ml of PHA, 0.1 mM NEAA9, in the presence of HLA-A24 positive allogeneic PBMCs donated from three healthy donors as feeder cells, without any peptide. The production level of IFN-γ in response to target cells in each well was assayed three times. To detect cancer responsive CTL precursor, a well was determined as positive under the following conditions: (a) both wells contained effecter cells produced at a higher level (>50 pg/ml) of IFN-γ in response to HLA-A24 positive SW 620 cancer cells than the level indicated when cultured in response to HLA-A24 negative Colo201 cancer cells and when cultured without cancer cells; and (b) the average IFN-γ level produced in response to SW602 cancer cells was significantly higher than the average the IFN-γ level produced in response to Colo201 cancer cells and the level cultured without cancer cells (two-tailed Student's t-test). To detect peptide responsive CTL precursor, a well was determined as positive under the following conditions: (a) both wells contained effecter cells at a higher level (>50 pg/ml) of IFN-γ produced in response to CIR-A2402 cells sensitized with either the corresponding SART3₁₀₉, SART3₃₁₅, and Ick₄₈₈ than the level produced in response to C1R-A2402 cells sensitized with irrelevant peptide, or in response to the C1R-A2402 cells sensitized with HIV peptide as a negative control; and (b) the average IFN-γ level produced in response to CIR-A2402 cells sensitized with either the corresponding SART3₁₀₉, SART3₃₁₅, and lck₄₈₈ was significantly higher (p,0.05) than the levels seen when one of the other two irrelevant peptides was used for the sensitization, or in case of HIV peptide.

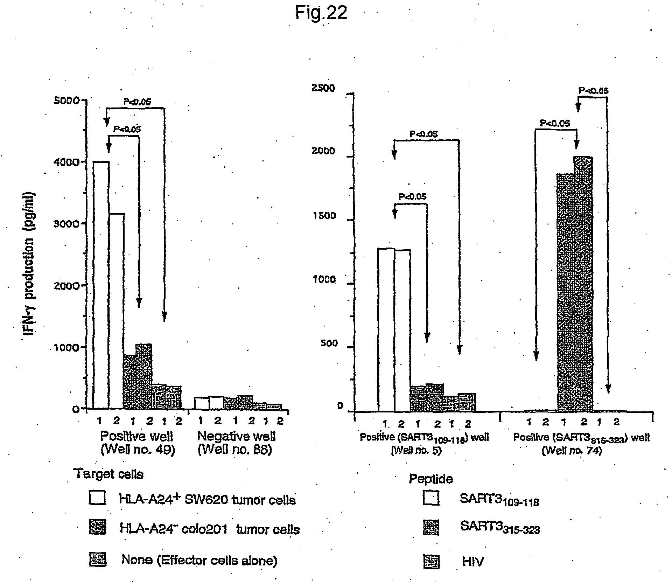

The following are the details of the assessment of the analytical results of CTL precursor frequency: For example, the production levels of IFN-γ due to recognition of cancer cells was measured in post-vaccinated PBMCs (after 3 doses of vaccination) in a patient (number 006). Figure 22A shows a typical example of positive well 49 and negative well 88.

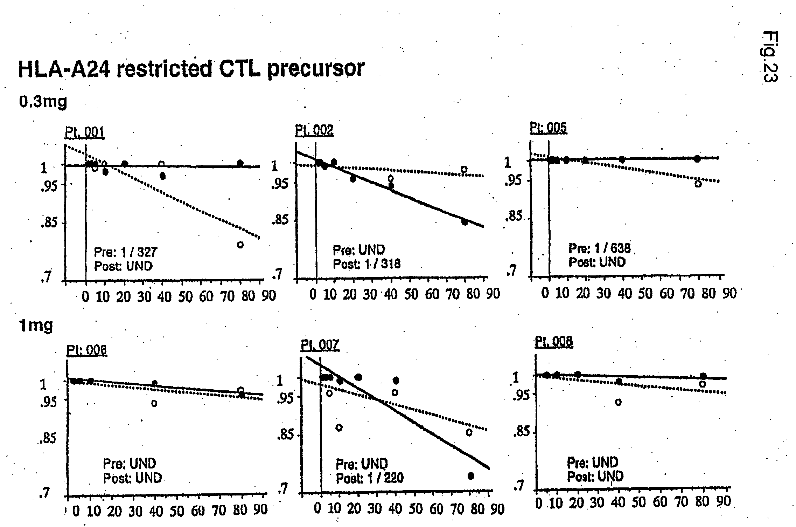

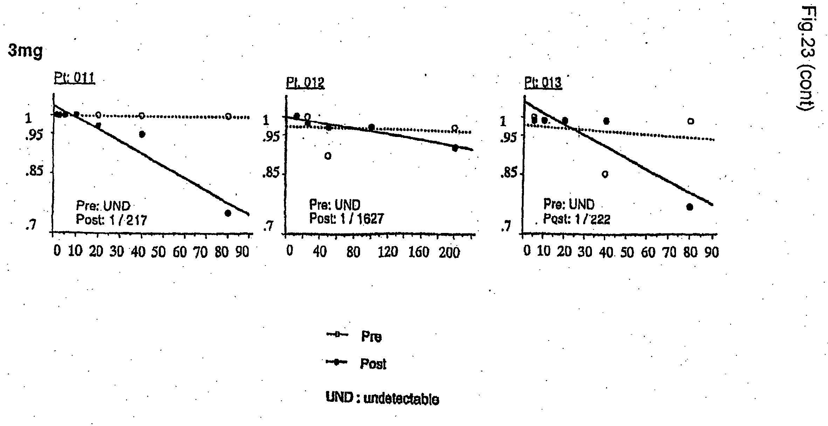

The statistical analysis and the evaluation of positive wells per plate were performed from the overall results of 1344 wells in total (96 well/plate x 7 different cells per well x 2 = 1344 wells). The numbers of positive wells of post-vaccinated PBMCs (after 3 doses of vaccination) were 24, 5, 3, 0, 0, 0, and 0 when the numbers of cells per well of a plate were 80, 40, 20, 10, 5, 2.5, and 1.25, respectively. In contrast, there were no positive wells in the pre-vaccinated PBMCs. These scores were plotted for the analysis of CTL precursor frequency (left of lower column in Figure 23), and the CTL precursor was calculated by the Taswell method with <1/5237 of pre-vaccinated PBMCs and 1/217 of post-vaccinated PBMCs (sensitivity limit 1/5237). See the literature incorporated as a part herein as a reference for the details of this result (Miyagi, Y., et al., Clin. Cancer Res., 3950: Vol. 7, 3950-5962, Dec. 2001).

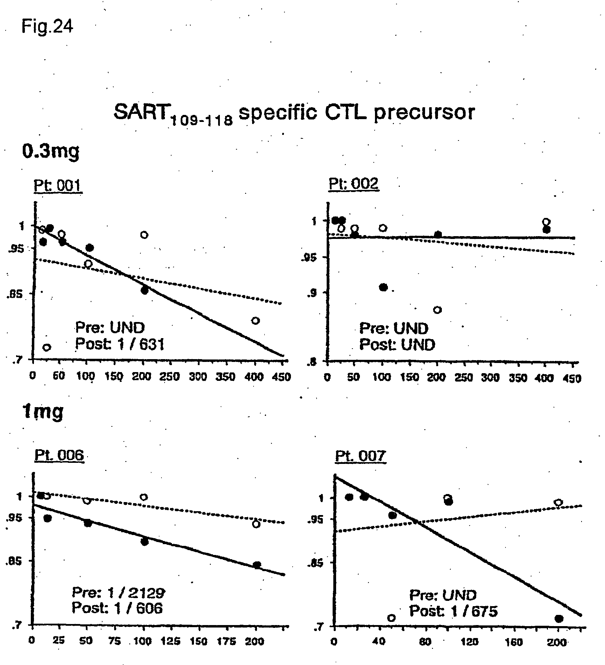

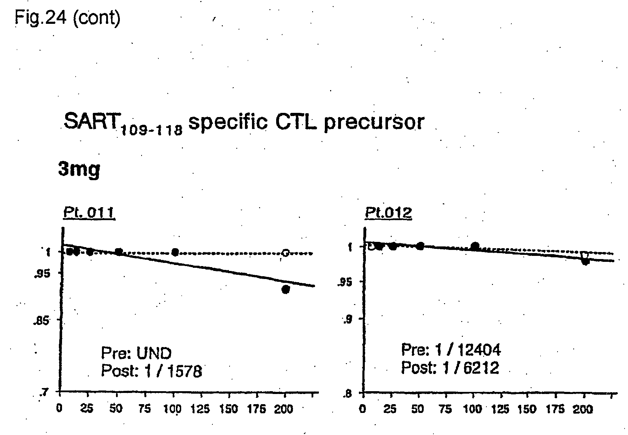

Figure 22B shows an example of frequency analysis of peptide-specific CTL precursor. In this example, the ability of post-vaccinated PBMCs (after 3 doses of vaccination) of a patient (number 006) to produce IFN-γ after 3 doses of vaccination was investigated, and wells 5 and 74 were rated as positive for the SART3₁₀₉ and SART3₃₁₅ peptide-specific CTL precursor (Figure 24). SART3₃₁₅ peptide-specific CTL precursor is not illustrated. For example, in post-vaccinated PBMCs (after 3 doses of vaccination) of a patient (number 013), the numbers of positive wells containing CTL precursor reactive to SART3₃₁₅ peptide were 21, 7, 6, 4, 1, and 0 in plates containing 200, 100, 50, 25, 12.5, 6.25 cells per well, respectively. CTL precursor responsive to SART3₃₁₅ peptide was determined as non-detectable (<1/12,407) before vaccination and calculated as 1/631 after vaccination.

Furthermore, cellular immune response of post-vaccinated PBMCs were post-kinetically detected at various points using a method described in the literature (Schichijo, S., et al., J. Exp. Med., 187:277-288, 1998). In other words, the post-vaccinated PBMCs were cultured with 10 µM of peptide in 200 µl of medium (complete medium) containing various numbers of effecter cells (100,000, 50,000, 25,000, 12,500, 6,250) in each well of a 96-well micro-culture plate having U-shaped wells. Half of the medium was discarded and exchanged with fresh medium containing a corresponding peptide (20 µM). This procedure was repeated every three days for 12 days of culture. Twenty-four hours after final stimulation, in these effecter cells, the IFN-γ production ability of the cells recognized by SW620 and Colo2402 cancer cells and the IFN-γ production ability in the absence of cancer cells as a negative control were examined by ELISA. In the same effecter cells, the IFN-γ production ability which responded only to CIR-A2402 cells sensitized with corresponding peptide or CIR-A2402 cells sensitized with HIV peptide or CIR-A2402 cells without sensitization was examined as a negative control. This assay was repeated three times.

### (Cellular Immune Response)

Figure 23 shows the frequency of CTL precursor responsive to HLA-A24 positive SW620 cancer cells in all 11 patients. In 7 of the 11 patients (patient numbers: 002, 007, 009, 010, 011, 012, and 013), significant increase in post-vaccinated PBMCs was observed. In the remaining 4 patients (number 001, 005, 006 and 008), detectable levels of post- vaccinated PBMCs could not be observed. Table 20 shows the summary of these results.

### <Table 20>

In the PBMCs stimulated with peptide, cytotoxicities to HLA-A24 positive SW620 cancer cells or HLA-A24 negative Colo201 cancer cells, VA-13 fibroblast, and HLA-A24 positive PHA activated normal T cells were measured in 6-h⁵¹Cr release assay. The procedure was repeated twice. Table 21 shows some representative results.

### <Table 21>

### (Kinetic Measurement of Cellular Immune Response)

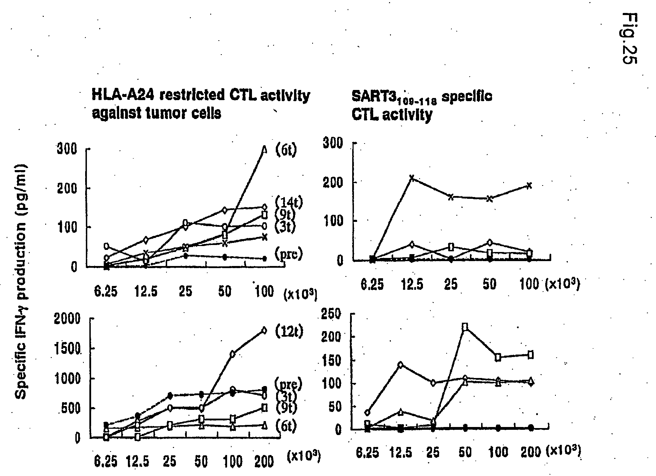

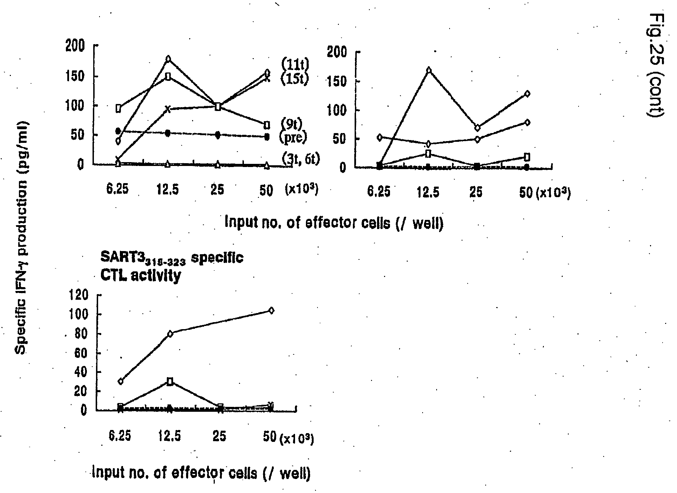

The kinetics of cellular immune response of PBMCs collected from three patients (number 001, 002, and 006) vaccinated with SART3 peptide more than 15 times was measured. Figure 25 shows the results.

As shown in Figure 25, there were almost no cells which recognized cancer cells and produced IFN-γ in the pre-vaccinated PBMCs from two patients (number 001 and 006). In one patient (number 001), a significant level of IFN-γ was noted in the PBMCs in response to HLA-A24 positive cancer cells when SART3₁₀₉ peptide vaccine was injected more than 6 times (0.3 mg per dose), and a significant level of IFN-γ producing CTL was induced in response to SART3 peptide when the vaccine was administered 18 times. In one of these patients (number 006), a significant level of IFN-γ producing CTL was induced in PBMCs by vaccinating with 1mg of 2 peptides per dose in response to HLA-A24 positive cancer cells after the 10^{th} vaccination, as well as in response to the corresponding peptide after the 11^{th} vaccination. In the pre-vaccinated PBMCs collected from another patient (number 002), treatment with 0.3 mg of SART3₁₀₉ peptide induced a significant level of IFN-γ production in response to HLA-A24 positive cancer cells in the concentration of more than 25,000 cells. These levels of IFN-γ production in PBMCs decreased if only 6 or 9 doses of vaccination were administered and increased with more doses (e.g. 12 doses). In the vaccination of a patient (number 002) with SART3₁₀₉ peptide a significant level of IFN-γ was produced in response to the corresponding peptide.

### (Clinical Responsiveness)

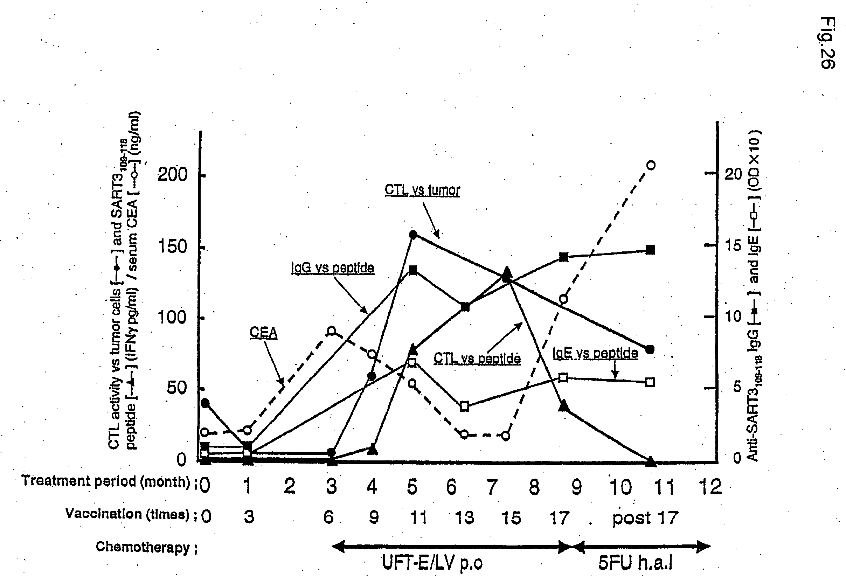

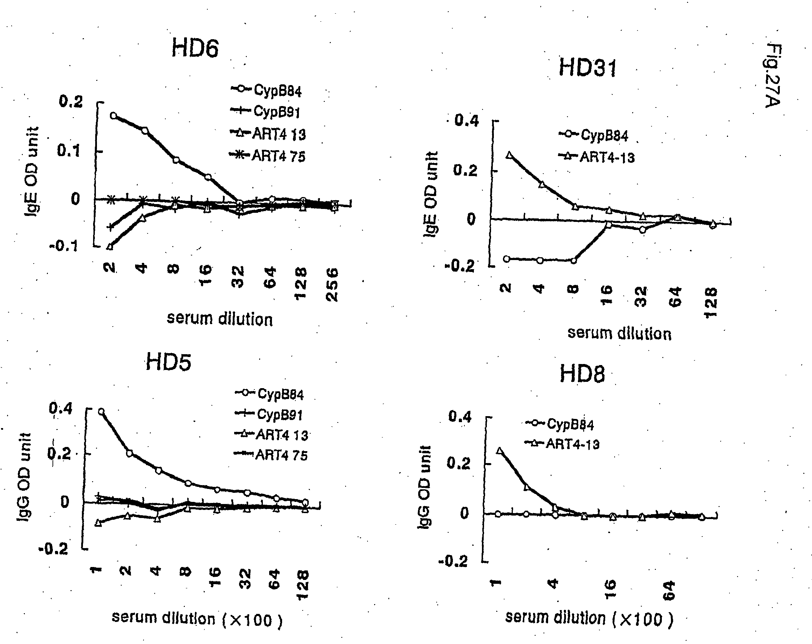

Figure 5 shows the clinical course of a patient (number 006) whose condition was determined to have been stable for 9 months. In this patient, only SART3 peptide was administered during the initial vaccination; and then treatment with SART3 peptide (7^{th} to 17^{th} dose of vaccination) and chemotherapy (oral administration of UFT-E (Taiho pharmaceutical Co., Ltd.) 450 mg/day and leucovorin (Takeda pharmaceutical Co., Ltd.) 40 /day) followed by only chemotherapy (injection of 5-fluorouracil 2250 mg into the hepatic artery and oral administration of leucovorin (Takeda pharmaceutical Co., Ltd.) 40 /day) was performed. The condition of this patient was stable for 9 months and then the patient experienced a relapse of metastases in the liver. In this patient, temporal decrease of serum CEA level between the 9^{th} and 15^{th} doses of vaccination was observed, and the diameter of cancer lesion diminished from 12.5 cm (after the 3^{rd} vaccination) to 10 cm (after the 12^{th} vaccination). It is noteworthy that the kinetics of cellular immune response reversely correlates to the kinetics of serum CEA level (Figure 26). In other words, the cellular immunity, which was not detected in either pre- or post-vaccinated PBMCs from the 1^{st} vaccination to the 10^{th} vaccination, became detectable in between the 11^{th} and 15^{th} vaccinations, and then gradually disappeared after the batch. The decrease of CEA also corresponded to the beginning of chemotherapy. The CEA level increased with roughly the 6^{th} vaccination, decreased until the 15^{th} vaccination, and then rapidly increased.

### Example 22

### Treatment for Colorectal Cancer

Ten patients with colorectal cancer were treated using peptide vaccine in the same manner as described in Example 18.

In this embodiment, CTL precursor which responded to 14 peptide candidates was screened using pre-vaccinated PBMCs, and subsequently, up to 4 kinds of peptides were selected as candidates according to the criteria described below: Criterion A: the production of peptide-specific IFN-γ was greater than 50 pg/ml, and the p value was smaller than 0.05 compared to recognition of the control HIV peptide; Criterion B: the production of peptide-specific IFN-γ was greater than 25 pg/ml, and the p value was smaller than 0.05; Criterion Z: the production of peptide-specific IFN-γ was greater than 50 pg/ml without a statistically significant difference. When an immediate hypersensitivity reaction positive in the skin test of one of these peptides, that peptide was vaccinated if the fifth peptide of criteria A, B, or Z was negative in the skin test. For SART2₈₉₉, CypB₉₁, ART1₁₇₀, and ART4₁₃, the immediate hypersensitivity reaction was positive in all patients and the vaccine was not used. As a result, as shown in Table 14, 4 peptides were injected into 5 patients; 3 peptides were injected into 3 patients; and 2 peptides were injected into 2 patients. In Table 14, o signifies vaccine peptides given to each patient. It is noteworthy that the profile of vaccine peptides for the 10 patients is almost completely different. The frequency of the vaccine peptides used is as follows: SART3₁₀₉ and Ick₂₀₈ (7 patients), CypB₈₄ and Ick₄₈₆ (4), ICk₄₈₈ (3), SART1₆₉₀, SART2₁₆₁, SART3₃₁₅, and ART₄₇₅ (2), and SART2₉₃ (1) were administered to the designated number of patients.

### (Toxicity)

The toxicity was assessed in all patients. The vaccine caused grade I or II local redness and swelling at the injection site in almost all patients (8 of 10 patients). Four patients (grade I and II) experienced fever with weak influenza-like symptoms, although the symptoms were temporary and did not need any treatment. In 2 patients, grade I fatigue and nausea were observed; in one patient, grade I anorexia, diarrhea, and nausea was observed. Grade III or IV toxicity related to the vaccine was not observed. There was no clinical evidence of autoimmune reaction that could be determined by symptoms, physical tests, or laboratory tests.

### (DTH Skin Test)

DTH (delayed type hypersensitivity) to the peptides was not observed in any of the patients before vaccination. Peptide-specific DTH reaction was observed in three patients after vaccination. In patient 1, DTH reaction to Ick₂₀₈ and Ick₄₈₈ was observed after the 6^{th} vaccination, and DTH to SART3₁₀₉ was detected after the 9^{th} vaccination. Patient 4 exhibited DTH reaction to Ick₂₀₈, Ick₄₈₆, and Ick₄₈₈ after the 3^{rd} vaccination. Patient 7 exhibited DTH reaction to SART3₁₀₉ after the 3^{rd} vaccination.

### (Cellular Immune Response)

In post-vaccinated (6^{th} dose) PBMCs, the production of peptide-specific IFN-γ was increased compared to pre-vaccinated PBMCs in 5 of 10 patients (patient numbers 1; 2, 5, 6, and 10). It was observed that at least one vaccinated peptide increased the production of IFN-γ (criteria A: >500 pg/ml) in 4 patients (Ick₂₀₈ and Ick₄₈₈ in patient 1, SART3₁₀₉ in patient 5, SART3₁₀₉ in patient 6, SART3₁₀₉ in patient 10). Furthermore, 5 patients (Ick₂₀₈ in patients 1 and 2, SART3₁₀₉ in patients 5 and 6, SART3₃₁₅ in patient 10) caused an increase in positive well frequency. In contrast, CTL activity to other peptides was not detected in the PBMCs of these 5 patients after vaccination. The immune response to all vaccinated peptides did not increase in the post-vaccinated PBMCs in other patients in C (numbers 3, 4, and 7-9).

CTL activities of pre- and post- vaccinated (3^{rd}, 6^{th}, and 9^{th} dose) PBMCs to SW620 (HLA-A24 positive colon cancer cells), Colo201 (HLA-A24 negative colon cancer cells), and PHA activated T cells (HLA-A24 positive) were evaluated. To avoid variances in bioassay, PBMCs collected from the same patient at various points were thawed simultaneously, and cultured for 14 days with only IL-2, and effector cells were used. None of the effecter cells tested exhibited cytotoxicity to PHA blastoid T cells. These cells showed low or intermediate levels of cytotoxicity to Colo201 cancer cells comprising a certain level of cytotoxicity among samples collected at various points. The increase of cytotoxicity to SW620 cancer cells was not observed in the post-vaccinated PBMCs in 4 patients (numbers 4- 7). Conversely, cytotoxicity to SW620 cancer cells was observed in another 5 patients as follows: 3^{rd} and 6^{th} post-vaccinated PBMCs in patient 1 as shown in Figure 4B; 6^{th} and 9^{th} post-vaccinated PBMCs in patient 2; 3^{rd} and 6^{th} post-vaccinated PBMCs in patient 7; and 6^{th} post-vaccinated PBMCs in patient 9.

### Example 23

### (Type I Allergy of Non-mutating Tumor Rejection Antigen in Healthy Donors)

Autoantibodies to cell surface antigen or nuclear components of DNA or histone are often detected in the serum of patients with autoimmune diseases. Low levels of autoantibodies have also been detected in the serum of healthy donors, and most of these donors have IgM or IgG isotype (Naparstek, Y., et al. Annual. Rev. lmmunol., 11:79, 1993; Rossi, F., et al., Immunol. Rev. 110:135, 1989 and others).

It is also known that immune effecter mechanism may lead to serious tissue damage when the mechanism affects a host (Naparstek, Y., et al. Annual. Rev. Immunol., 11:79, 1993). In effect, several autoimmune peptides were shown to induce anaphylactic shock in mice exhibiting experimental autoimmune encephalomyelitis (EAE) (Pedotti, R., et al., Nature Immunol., 2:216, 2001).

Furthermore, in the past 10 years, many autoantibodies recognized by serum CTL and IgG of cancer patients have been identified by the cDNA expression cloning method and SEREX method (Yang, D., et al., Cancer Res. 59:4056, 1999; Shichijo, S., et al., J. Exp. Med. 187:277, 1998; Gomi, S., et al., J. Immunol. 163:4994, 1999; Nishizaka, S., et al., Cancer Res. 60:4830, 2000; Ito, M., et al., Cancer Res. 61:2038, 2001; Harashima, N., et al., Eur. J. Immunol. 31:323, 2001; Kawano, K., et al., Cancer Res. 60:3550, 2000; Nakao, M., et al., J. Immunol. 164:2565, 2000 and others).

These molecules were determined to be non-mutating autoantibodies by cloning and sequencing cDNA, which codes allergic molecules, and now, evidence is accumulating that IgE mediated reaction to autoantibody plays an important role in serious atopic diseases (Natter, S., et al., FASEB J. 12: 559, 1998 and others). The inventors identified many tumor rejection antibodies from the cDNA library of epithelial cancer using a similar molecular technique (Yang, D., et al, ibid; Shichijo, S., et al, ibid; Gomi, S., et al, ibid; Nishizaka, S., et al, ibid; Ito, M., et al, ibid; Harashima, N., et al, ibid; Kawano, K., et al, ibid; and Nakao, M., et al, ibid). Most of these antigens recognized by host CTL play various roles in cell proliferation, and thereby were non-mutating autoantibodies preferably expressed in both malignant cells and normal proliferating cells. The non-specific autoantibody recognized by IgE of atopic dermatitis patients and that recognized by CTL of cancer patients have many common characteristics including intranuclear preferential cell-to-cell arrangement. Two non-specific autoantibodies recognized by a group of tumor rejecting antibodies, SART1, and cyclophilin (CypB) (Shichijo, S., et al., J. Exp. Med. 187:277, 1998; Gomi, S., et al., J. Immunol. 163:4994, 1999) have been also identified as antigens which are involved in the anti-allergic process of atopic dermatitis (Seiberler, S., et al., Int. Arch. Allergy Immunol. 120:108, 1999; Van der Zee, JS., et al., Eur. Respir. J. Suppl., 13:91s, 1991).

The inventors discovered type I allergy to a non-specific auto-peptide in healthy donors in the process of developing peptide-specific immunotherapy for cancer patients during the pre-vaccine skin test to help avoid anaphylactic shock.

### Experimental Method

### (Subjects)

The subjects of this embodiment were 51 healthy donors aged 19 to 54 who donated their blood. None of the donors had a history of allergic diseases and all had normal serum IgG (≤250 IU/ml in RIST). The serum of cancer patients who underwent phase I clinical study of peptide cancer vaccine was also used.

### (Peptide and Skin Test)

All peptides used in this embodiment exhibited binding ability to HLA-A24 and CTL inducing ability; in this embodiment, SART2₁₆₁, SART2₈₉₉, SART3₁₀₉, SART3₃₁₅, CypB₈₄, CypB₉₁, ART4₁₃, and ART4₇₅ were used as peptides.

The peptides used were dissolved in dimethylsulfoxide (DMSO), stored at -80°C, and diluted with saline just before the treatment. This peptide (50 µl) solution (10 mg) was subcutaneously injected and allergic skin reaction 15 minutes after the injection was assessed. Physiological saline containing the same amount of DMSO was used as a control.

### (Quantification of Peptide-specific IgE and IgG)

Serum IgE and IgG levels were measured by ELISA method. The peptide was fixed with disuccinimidyl suberate to a 96-well flat plate. The peptide (20 µg/well)-fixed plate was blocked with Block Ace, and washed with 0.05% Tween 20-PBS (PBST). Subsequently, serum or plasma sample diluted with 0.05% Tween 20-Block Ace (100 µl/well) was added to this plate and it was left to stand for 2 hours at 37°C. The plate was then washed with PBST and left to stand for another 2 hours at 37°C along with 1:1000 diluted rabbit anti-human IgE (ε chain specific), anti-human IgG (γ chain specific), and anti-human IgG subclass-specific antibody (Zymad Laboratories). After standing, the plate was washed 9 times, and 1:100 diluted goat anti-rabbit Ig binding horseradish peroxidase-dextran polymer (EnVision) was added to each well, and then cultured for 40 minutes. Next, the plate was washed, 100 µl/ml of tetramethylbenzine substrate solution (KPL), and 1 M phosphoric acid were added to terminate the reaction. To estimate peptide-specific IgE level, the optical density (OD) of each sample was compared to a standard sample which was sequentially diluted, and the resultant values were indicated as OD unit/ml. Peptide-specific IgG was similarly estimated. In standard wells, 1:100 diluted anti-human IgE monoclonal antibody or anti-human IgG monoclonal antibody was fixed instead of a peptide. HIV peptide was used as a solid phase antigen, and the cutoff point of peptide-specific IgE or IgG was determined. The HIV-specific IgE and IgG of 41 healthy donors were 0.01 and 0.02 OD units respectively, and these values were used as the cutoff point. Total IgE of samples containing the standard sample and also Aspergillus-antigen specific IgE level were measured. It is not easy to compare OD units measured in this embodiment and LC units of MAST directly because the two systems are completely different. However, this embodiment provides sample data on Aspergillus-antigen specific IgE levels of atrophy patients measured by these two assay systems. In this case, 1.73LC of MAST was comparable to 2.35 OD units of the system of this embodiment. However, these values were against multi-epitope specific IgE, and the values on single epitope or short peptide specific IgE were several times smaller.

### (Vaccination Protocol)

Vaccine was administered to HLA-A24 positive patients with progressive cancer as following. CypB₉₁ was given to lung cancer patients, ART4₇₅ to ovarian cancer patients, and SART3₁₀₉ to colorectal cancer patients. None of the patients received any steroids or other immunosuppressants from 4 weeks before the beginning of this study to the end of the study. When the skin test was negative, 1 ml of peptide solution (1 mg/ml) was emulsified with the same amount of Freund's incomplete adjuvant (Montanide ISA-51) and injected subcutaneously. In this embodiment, all patients were vaccinated at least three times every two weeks, and up to seventeen times, depending on the patient's wishes.

### Result

### (Type I Allergy to Autopeptide in Healthy Donors)

Among the peptides used in this embodiment, 2 kinds of cyclophilin (CypB) derived peptides, CypB₈₄ and CypB₉₁, have been identified as tumor rejection antigen peptides recognized by HLA-A2402 restricted CTL. The possibility of using these peptides as a cancer vaccine was investigated in a Phase I clinical trial. In this clinical trial, CypB₈₄ induced immediate hypersensitivity reaction in 8 of 10 vaccinated patients with lung cancer in a pre-vaccination skin test. 3 of 6 peptides (ART4₁₃, SART2₈₉₉, and SART3₃₁₅) also induced allergic skin reaction in a pre-vaccination study (Miyagi, S., et al., Clin. Cancer Res., 7:3950, 2001). All peptides used were derived from non-mutating autoantibody and elicited HLA-A24 restricted and cancer -specific CTL activity from PBMCs of cancer patients.

For a better scientific understanding of this unexpected phenomenon, we examined whether an immediate hypersensitivity reaction to these 3 peptides is also observed in healthy donors (41 donors; aged 19-54; average 29.2 years). As a result, the rates of skin reaction occurrence were 39/41 (95%), 38/40 (95%), 10/40 (25%), 7/41 (17%), and 18/40(45%) for CypB₈₄, ART4₁₃, SART2₈₉₉, SART3₁₀₉, and SART3₃₁₅ respectively in healthy donors. Conversely, there was no such response in CypB₉₁, ART479, and SART2₁₆₁. The allergic reactions, ranging from wheal to redness, occurred within one minute of subcutaneous injection of the peptide. In most cases, the reaction reached a peak within 5 to 20 minutes, then gradually diminished between 60 and 90 minutes. These findings show that such skin reactions induced by peptide are of typical type I allergies. Systemic anaphylactic reaction, including Arthus reaction, delayed type hypersensitivity and delayed phase response were not observed in any patients. Table 1 shows the results of skin tests. We could not find any correlation between allergic skin reaction and HLA-A or HLA-DR-type of the subject, or between the allergic skin response of the subjects and their sex.

### (Peptide-specific IgE and IgG in Healthy Donors)

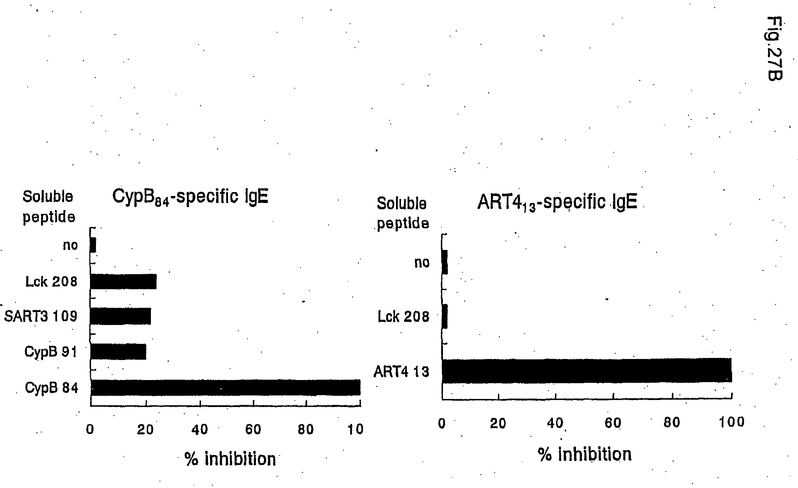

To better understand the molecular basis of allergic skin reaction, the serum levels of IgE and IgG were measured. Figure 27A shows the results of measurement by the ELISA method.

Table 1 shows the test results on serum IgE and IgG specific to each peptide. CypB₈₄- or CypB₉₁-specific IgE was detected in 12 or 6 of 41 donors respectively, and CypB₈₄- or CypB₉₁-specific IgG was detected in 4 or 5 of 37 donors respectively. Of 39 donors who showed skin reaction to CypB₈₄, the IgE level to the corresponding peptide in the serum of 12 donors was detectable, but there was no correlation between the degree of skin reaction and serum IgE level. ART13-specific IgE was detected in 5 of 41 donors, and ART75-specific IgE was detected in 0 of 40 donors. ART13-specific IgG and ART75-specific IgG were detected in 2 and 6 of 37 donors, respectively. SART2₁₆₁-specific IgE was detected in 1 of 39 donors, and SART2₈₉₉-specific IgE in 0 of 39 donors. SART2₁₆₁-specific and SART2₈₉₉-specific IgGs were detected in 3 and 7 of 40 donors, respectively. SART3₁₀₉-specific and SART3₃₁₅-specific IgEs were detected 11 and 6 of 41 donors, respectively, and IgGs specific to these two were detected in 8 and 4 of 37 donors, respectively. Overall, there was no correlation between the level of in vivo skin reaction and the level of serum IgE or IgG specific to the corresponding peptide. Given that good correlation is usually found when responsiveness of these two parameters is compared with a common allergen, these findings were unexpected. Furthermore, it is noteworthy that, in the serum of 2 donors who did not show skin reactions to CypB₈₄ (HD5 and HD37), IgG to the corresponding peptide was at a detectable level, although the level of IgE was also detectable.

To better understand the relative contribution of T helper type 1 and type 2 cells to antibody production, the ratio of total IgG to the serum CypB and ART4 peptide in 6 healthy donors (HD3, 5, 15, 28, 32, and 37) and the ratio of IgG subclass to peptide-specific IgG were examined (Janeway, C.A. Jr., et al., 1999, Immunobiology, 4^{th} ed., New York/ London: Garland Publishing Inc.; 2Van der Zee at al., ibid). There was no significant difference between the rate of T helper type 2 cell-enhancing antibodies (IgG4 and IgE) to allergic peptide (CypB₈₄ and ART4₇₅) and the rate to non-allergic peptide (CypB₉₁ and ART4₇₅). In other words, the priority IgG subtype of all IgG was IgG1 (59-79% of all IgG; average: 69%), and the secondary subtype was IgG2 (17-29% of all IgG; average: 21%). The relative quantities of IgG3 and IgG4 were 8-3% and 6-2%, respectively. The distributions of peptide-specific IgG subtypes to 4 peptides (CypB₈₄, CypB₉₁, ART4₁₃ and ART4₇₅) were similar to that of total IgG, or 86-60%, 32-7%, 7-4%, and 5-2% respectively.

### (Substitution of Amino Acid)

To determine the amino acid residue important for the elucidation of skin reaction in 7 healthy donors, CypB₈₄ and ART4₁₃, and homolog peptides in which the individual amino acid residue was substituted with glycine, were prepared (Table 22).

### <Table 22>

CypB₈₄ with glycine substituted at position 1, 2, 3, 4, 5, 6, 7, 8, or 9 significantly decreased the skin reaction in 1, 1, 2, 1, 0, 3, 1, 0, or 2 donors respectively (<=0.25 of the rate in redness range). These findings suggest that the crucial amino acid residue varies significantly depending on the individual. In contrast, glycine substitution at positions 2 and 4 of ART4₁₃ did not induce skin reaction in any of 4 donors, but glycine substitutions at position 3, 5, 6, and 8 each induced a significant decrease in skin reaction in one donor.

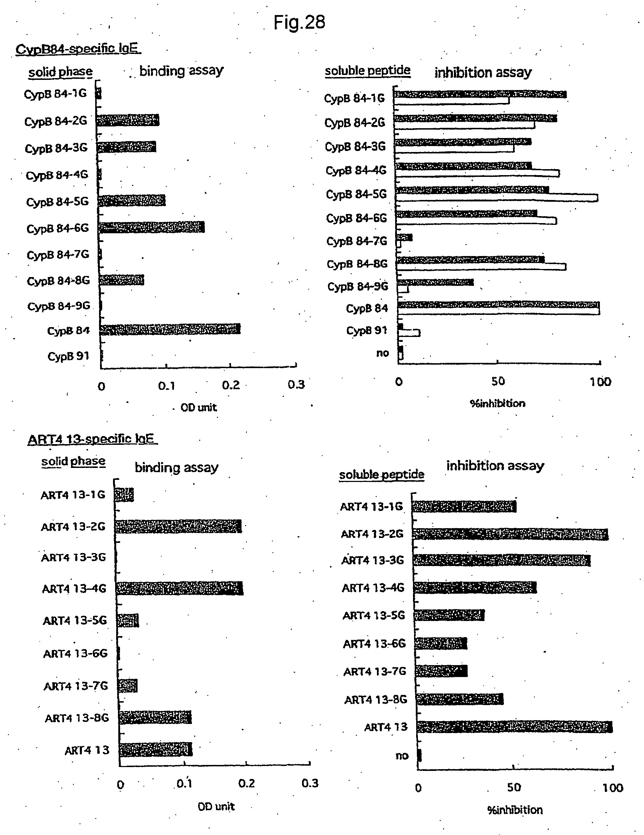

Amino acid residues important in the recognition of antibody were determined using these peptides. Figure 28 shows typical results.

As shown in Figure 28, the substitution of amino acids at positions 1, 4, 7, and 9 of CypB₈₄ was important for CypB₈₄-specific IgE binding in one case (HD6 in Table 22) (Figure 28, left of upper column).

The importance of amino acids at position 7 and 9 was further confirmed by inhibition assay (Figure 28, right of upper column). In three cases (HD10, HD15, and HD40), the amino acid residues at positions 1, 7, and 9 of CypB₈₄ peptide were important for the recognition of antibody in the binding assay, and the amino acid residues at positions 1, 6, 7, and 9 were important for the recognition of antibody in inhibition assay. In one case (HD11 of Table 22), the amino acid residues at positions 1, 3, 5, 6, and 7 or the amino acid residues at position 5, 6, 7, and 8 of ART4₁₃ were important for the recognition of antibody by anti-ART4₁₃-specific IgE in binding and inhibition assays (Figure 28, lower column). In two cases (HD32 and HD40), the amino acid residues at position 1, 3, 6, and 7 in ART4₁₃ were important for the recognition of antibody in binding assay, and the amino acid residues at position 1, 6, 7, and 8 were important for the recognition of antibody in inhibition assay. The absence of correlation between binding and inhibition assays appears to reflect the difference in antibody affinity detected in these two assays. Overall, the amino acid residues at position 7 and 9 of CypB₈₄ peptide and the amino acid residues at position 1, 6, and 7 of ART4₁₃ were more important for the recognition of antibody compared to other amino acid residues. However, there was no common amino acid residue critical for the clarification of skin reaction and recognition of serum antibody in healthy donors. These results show considerable diversity in the epitope of a great many amino acid residues and heterogeneity of each individual, in addition to the fact that the results reflect the polyclonality of peptide-specific IgE antibody.

### (Skin Reaction to Various CypB₈₄ Homologs)

CypB is a molecule highly preserved, and found in various species including vertebrates, invertebrates, protozoans, plants, and fungi. The sequences of CypB₈₄ and CypB₉₁ homologs are also found in these species, and Table 23 shows the typical sequence of these CypB₈₄ homologs.

### <Table 23>

To better understand the involvement of extrinsic antigen-derived peptide in type I allergy induced by CypB₈₄, the skin reactions to CypB₈₄ homologs were examined in 5 healthy donors. The result was that all CypB₈₄ homologs exhibited positive skin reaction in all 5 donors, and the degree of skin reaction to these homologs was comparable to that to human CypB₈₄ (Table 3). The only exception was the Streptomyces chrysomallus derived CypB₈₄ homolog, which induces weaker skin reaction than the reaction to human CypB₈₄. In contrast to CypB, to the knowledge of the inventor, human ART4, SART2 and SART3 homologs have not been identified in any species other than mammals at the level of nucleotide and protein. In addition, unlike CypB, natural sensitization by microbes or foods barely influenced Ig4 response to ART4, SART2, and SART3 described in this embodiment.

### (Kinetics of Antibody Production and Allergic Reaction in Peptide Vaccine Recipients)

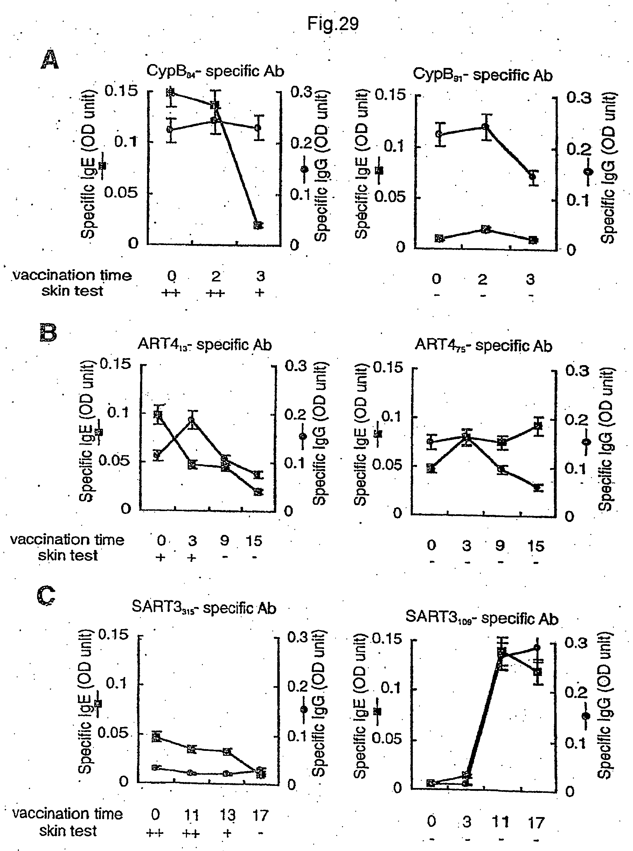

To better understand the influence of non-allergic peptide vaccine on allergic adjacent peptide, the correlation between the kinetics of peptide-specific serum IgE and IgG level and the type I allergy in non-allergic peptide vaccine recipients was examined. In this embodiment, three patients were vaccinated with CypB₉₁, ART4₇₅, and SART3₁₀₉. Figure 29A, 29B, and 29C show the results.

In these patients, CypB₈₄, ART4₁₃, and SART3₃₁₅ were not used in the pre-vaccination skin test because these peptides induced type I allergy. In pre-vaccination serum, significant levels of IgE to CypB₈₄, ART4₁₃, and SART3₃₁₅ were detected. These levels decreased after vaccination in the serum of patients who were vaccinated with non-allergic CypB₉₁, ART4₇₅, and SART3₁₀₉. Conversely, IgE to non-allergic CypB₉₁ and SART3₁₀₉ was not detected in pre-vaccination serum, but low levels of IgE to ART4₇₅ were detected in pre-vaccination serum. IgE to CypB₉₁ was not detected in the serum after vaccination with CypB₉₁ (two or three doses), but the IgE level to ART4₇₅ increased in the serum of patients after vaccination with ART4₇₅ (3, 9, and 15 doses), and IgE specific to SART3₁₀₉ was detected in the serum of patients after vaccination with SART3₁₀₉ (11 and 17 doses). It is noteworthy that ART4₁₃ and SART3₃₁₅ did not induce type I allergy in pre-vaccination skin tests that were performed before the 9^{th} and 17^{th} dose of vaccination with ART4₁₃ and SART3₃₁₅ peptides. Furthermore, ART4₁₃ and SART3₃₁₅ peptides can be safely administered at the 9^{th,} 17^{th} and later doses of vaccination. Even when these ART4 and SART3 peptides were administered, the peptides did not induce any adverse reactions in cancer patients, including immediate local or generalized hypersensitivity reaction. CypB₉₁ induced type I allergy in the 3^{rd} pre-vaccination skin test of CypB₉₁ peptide, but the degree of skin reaction decreased significantly. The distribution of all subclasses of IgG and peptide-specific IgG to these peptides showed no significant change over the course of vaccination.

### (Discussion)

It is expected that the results for healthy donors and cancer patients indicated in this embodiment may lead to a better immunological and pathological understanding of autoantibodies in host immunity studies of cancer and hypersensitivity. However, at this point, the biological implications of IgE response specific to non-mutating auto-peptide recognized by healthy donors and type I allergy is not clear.

This embodiment brings out two interesting points about the serum of patients with atopic dermatitis: (1) serum IgE and IgG specific to allergic autopeptide in patients with atopic dermatitis are significantly higher than those of healthy donors and cancer patients; (2) the pattern of serum IgE to non-mutating autopeptide in atopic dermatitis patients is markedly different from the pattern in healthy donors and cancer patients. Therefore, this point needs to be studied further.

There is a report stating that the IgE of patients who have been sensitized with environmental allergens, such as Aspergillus fumigatus, cross-reacted with mitochondrial superoxide dismutase (MnSOD), profilin, p2 ribosome protein, cyclophilin B and SART1 (Appenzeller, U., et al., Arch. Allergy Immunol, 118:193; 1999 and others). However, allergic skin reaction and autopeptide-specific IgE detected in healthy donors cannot be detected primarily by the environmental allergen. This is because (1) none of the healthy donors studied in this embodiment had atopic disease; (2) the levels of serum IgE which responded to Aspergillosis allergens or house dust did not correlate with the levels of autopeptide; and (3) ART4, SART2, and SART3 homologs cannot be detected in fungi and other environmental microbes.

The cross-binding and subsequent degranulation effect of IgE-binding high affinity FcεR-I to mast cells by an allergen is the main pathway of allergic skin reaction. In this embodiment, a peptide comprising 9 amino acids was used to examine such allergic reactions. The mechanism where such a small peptide induces allergic reaction might be similar to that of the reaction induced by antibiotics or other drugs with low molecular weight. More specifically, the peptide molecule may form a complex with serum protein or extracellular matrix protein, or bind to the surface of a cell after subcutaneous injection. The complex, rather than aggregate of peptides, cross-reacts with FcεR-I on mast cells. In relation to the ability to induce allergic skin reaction, the fact that centrifugation is not effective in eliminating the aggregate supports this hypothesis. Since there is no clear correlation between the allergic reaction to a peptide and HLA-A or HLA-DR type subjects, it appears that there is no relationship between the binding activity of peptide to HLA-A or HLA-DR molecule and the serum complex.

Inhibiting the allergic peptide specific IgE response and the immediate skin reaction caused by repeated vaccination with non-allergic adjacent peptide derived from the same protein could provide a new tool in the treatment of allergic diseases. The traditional desensitization using whole allergen extract always has a risk inducing systemic anaphylaxis. As another method, allergen-specific desensitization by immunity using class II restricted peptide recognized by CD4⁺T cells has been attempted, but such class II restricted peptides have the possibility of augmenting not only Th1 response, but also Th2 response in a vaccinated host. In the process of developing specific immunotherapy using peptides in cancer patients, the inventors found that the immediate skin reaction which responds to specific IgE and is derived from the same protein molecule is inhibited by vaccinating repeatedly with a non-allergic autopeptide recognized by HLA class restricted CTL. As an explanation of these results, repeated vaccination with class I restricted and non-allergic peptides might induce clone expansion of non-allergic peptide-specific CD8⁺T cells, which may be caused by inhibition of preexisting specific IgE response to allergic adjacent peptide. Although the mechanisms are unknown at this point, these results provide new evidence to develop allergen specific desensitization for patients with allergic diseases by immunizing using class I restricted and non-allergic adjacent peptide.

### Industrial Applicability

Immunotherapy using the antigens and peptides of the present invention is necessary and useful, both from medical and financial viewpoints, for the prevention and treatment of cancers, virus infections, autoimmune diseases, and the like. According to the present invention, the use of vaccines for the treatment of such diseases is clearly superior to approaches using traditional pharmaceutical compositions, especially when population is expanding worldwide. Furthermore, the culture protocol related to this invention may be advantageously applied to the detection and monitoring of pre- and post-vaccination CTL response.

The present invention is not "quantitative" like traditional methods, but "semiquantitative" as examined in Example 5. However, since the present invention uses only small amounts of blood (lymphocytes) from patients and may evaluate many antigen peptides in a short period of time, it is effective in determining the peptide to be used in treatment before the treatment of the patient starts (before vaccination).

As a result of comparative study of the FLISA and ELISA methods, the FMAT scanner was demonstrated to detect IFN-γ accurately using beads and fluorescence. The FLISA method was found to be comparable to the traditional ELISA method in linear dynamic range and sensitivity. Furthermore, measurements of IFN-γ production are widely employed in many clinical studies which use cancer vaccines, and the ELISA method is a well-established method for the measurement of IFN-γ. However, the method requires multiple processes of culture and washing, as well as an excessive amount of antibodies. On the other hand, limiting dilution often requires a very large number of plates.

In contrast, the FLISA method shortens the measurement time of IFN-γ and is cheaper. Since it is a homogeneous assay, it has the major advantages that it does not require a washing process to eliminate non-binding antibodies, ligands, and fluorescent groups, and further, the standing time is shorter, and the quantity of antibodies required is less.

Furthermore, FLISA has the major advantage that, using 384-well plates, it is possible to further shorten the standing time and reduce overall measurement time of IFN-γ significantly.

Of particular importance in this invention's method of detecting antigens which react with antigen-specific T cells, not all cancer cells necessarily express common tumor antigens; given that treatment using multiple tumor antigen peptides is effective because two or more different tumor antigen peptides are presented on one cancer cell, this method is very useful.

Furthermore, the method is useful when monitoring the fluctuation of antigen-specific T cells before and after vaccination, or examining if the treated antigen peptide is effective.

The method according to the invention enables micro-method to detect antigen-specific T cells systematically.

In this invention, if the frequency of CTL precursor to various antigen peptides is measured in peripheral blood T cells of cancer patients by the new cellular immune assay, and highly reactive antigen peptides are selected, treatment of cancer patients using cancer vaccines will bring improved results.

Furthermore, repeated vaccination with non-allergic peptide derived from an allergen containing allergen peptides may inhibit both type 2 allergic peptide-specific IgE responses and type I allergic responses, and provides a new approach for desensitization using peptides.

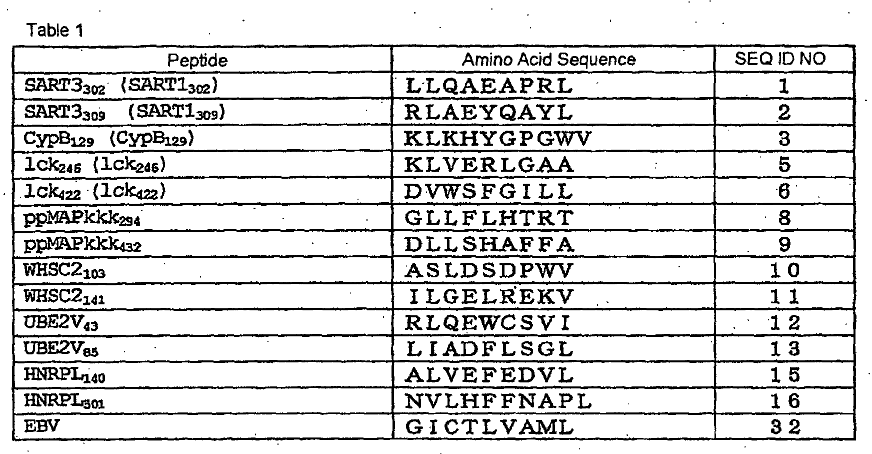

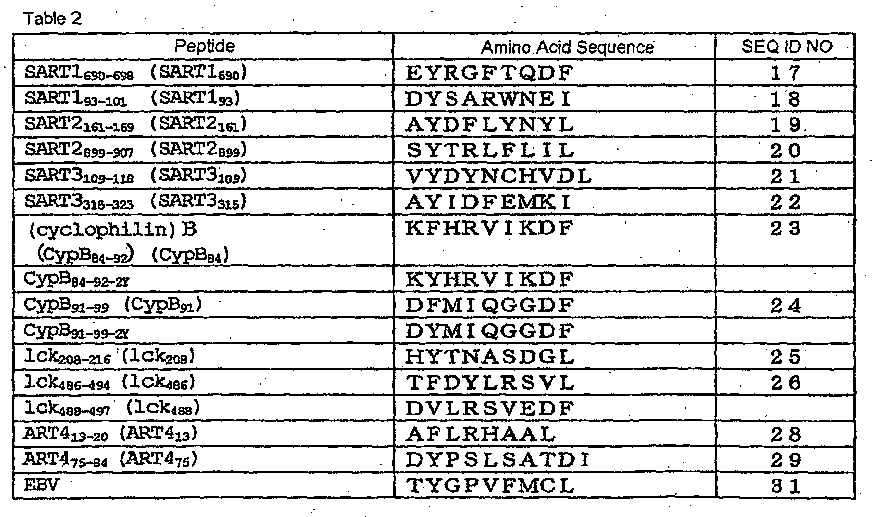

**Table 3:**

| Culture Protocol for the Induction of Peptide-Specific T Cells | | | | | | | | |
|---|---|---|---|---|---|---|---|---|
| | Culture | | Total incubation (days) | Added cells/well | Yield cells/well | IFN-γ production (pg/ml) | | |
| Protocol | Interval (days) | Stimulation (times) | | (× 10⁴) | (× 10⁴) | EBV peptide | Flu peptide | SART3 109-118 |
| A | 1 | 10 | 11 | 10 | 5 | 0 | 0 | 0 |
| B | 2 6 | | 11 | 10 | 45 | 59 | 227 | 12 |
| C | 3 | 5 | 13 | 10 | 45 | 400 | 242 | 0 |
| D | 4 | 4 | 13 | 10 | 55 | 319 | 29 | 0 |
| E | 5 | 3 | 11 | 10 | 60 | 161 | 16 | 0 |
| F | 6 | 3 | 13 | 10 | 70 | 0 | 0 | 0 |
| G | 7 | 3 | 21 | 200 | 400 | 100 | 150 | 0 |

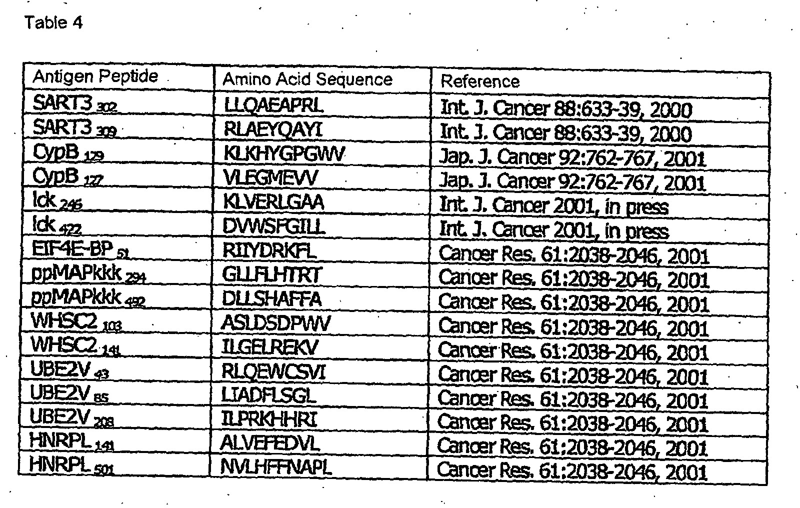

**Table 5**

| Antigen Peptide | **IFN**-γ production **(pg/well)** | | | |
|---|---|---|---|---|
| | 1st well | 2nd well | 3rd well | 4th well |
| **SART3** _{**302**} | **<0** | **<0** | **8** | **<0** |
| | ***p=0.612*** | ***p=0.265*** | ***p=0.887*** | ***p=0.612*** |
| **SART3** _{**309**} | **<0** | **<0** | **9** | **28** |
| | ***p=0.008*** | ***p=0.618*** | ***p=0.255*** | ***p=0.414*** |
| **CypB** _{**172**} | **22** | **<0** | **<0** | **<0** |
| | ***p=0.220*** | ***p=0.557*** | ***p=0.054*** | ***p=0.285*** |
| **lck** _{**246**} | **19** | **<0** | **<0** | **<0** |
| | ***p=0*.*724*** | ***p=0.271*** | ***p=0.141*** | ***p=0.286*** |
| **lck** _{**422**} | **<0** | **<0** | **<0** | **8** |
| | ***p=0.916*** | ***p=0.400*** | ***p=0.279*** | ***p=0.885*** |
| **ppMAPkkk** _{**294**} | **133** | **<0** | **<0** | **<0** |
| | ***p=0.062*** | ***p=0.373*** | ***p=0.361*** | ***p=0.325*** |
| **ppMAPkkk** _{**432**} | **137** | **12** | **<0** | **<0** |
| | ***p=0.022*** | ***p=0.429*** | ***p=0.164*** | ***p=0*.*861*** |
| **WHSC2** _{**103**} | **74** | **<0** | **<0** | **<0** |
| | ***p=0.007*** | ***p=0.950*** | ***p=0.300*** | ***p=0.922*** |
| **WHSC2** _{**141**} | **10** | **<0** | **<0** | **<0** |
| | ***p=0.134*** | ***p=0.830*** | ***p=0.858*** | ***p=0.748*** |
| **UBE2V** _{**43**} | **13** | **11** | **<0** | **<0** |
| | ***p=0.617*** | ***p=0.728*** | ***p=0.988*** | ***p=0.074*** |
| **UBE2V** _{**85**} | **7** | **34** | **<0** | **<0** |
| | ***p=0.735*** | ***p=0.331*** | ***p=0.512*** | ***p=0.393*** |
| **HNRPL** _{**140**} | **24** | **<0** | **13** | **40** |
| | ***p=0.452*** | ***p=0.423*** | ***p=0.670*** | ***p=0.372*** |
| **HNRPL** _{**501**} | **24** | **<0** | **<0** | **18** |
| | ***p=0.214*** | ***p=0.079*** | ***p=0.093*** | ***p=0.035*** |

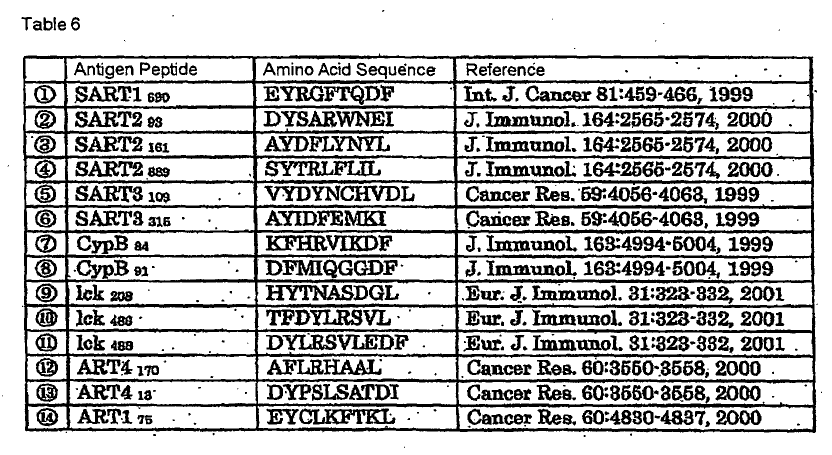

**Table 7**

| Antigen Peptide | **IFN**-γ production¹⁾ **(pg/well)** | | |
|---|---|---|---|
| | 1st well | 2nd well | 3rd well |
| **SART1**_{**690**} | **<0** | **N.D*** | **133** |
| | ***p=0.159*** | | ***p=0.284*** |
| **SART2** _{**93**} | **<0** | **33** | **99** |
| | ***p=0.074*** | ***p=0.500*** | ***p=0.416*** |
| **SART2** _{**161**} | **<0** | **<0** | **84** |
| | ***p=0.002*** | ***p=0.795*** | ***p=0.040*** |
| **SART2** _{**889**} | **<0** | **<0** | **49** |
| | ***p=0.167*** | ***p=0.261*** | ***p=0.009*** |
| **SART3** _{**109**} | **275** | **<0** | **N.D** |
| | ***p=0.251*** | ***p=0.585*** | |
| **SART3** _{**315**} | **38** | **7** | **424** |
| | ***p=0.163*** | ***p=0.830*** | ***p=0.092*** |
| **CypB** _{**84**} | **<0** | **<0** | **20** |
| | ***p=0.140*** | ***p=0.896*** | ***p=0.314*** |
| **CypB** _{**91**} | **N.D** | **2** | **<0** |
| | | ***p=0.929*** | ***p=0.776*** |
| **lck** _{**208**} | **<0** | **<0** | **76** |
| | ***p=0.067*** | ***p=0.244*** | ***p=0.014*** |
| **lck** _{**486**} | **<0** | **<0** | **31** |
| | ***p=0.001*** | ***p=0.979*** | ***p=0.111*** |
| **lck** _{**488**} | **<0** | **<0** | **25** |
| | ***p=0.150*** | ***p=0.657*** | ***p=0.153*** |
| **ART4** _{**170**} | **<0** | **22** | **<0** |
| | ***p=0.754*** | ***p=0.678*** | ***p=0.166*** |
| **ART4** _{**13**} | **30** | **<0** | **35** |
| | ***p=0.028*** | ***p=0.106*** | ***p=0.144*** |

| | | | |
|---|---|---|---|
| *) N.D. means "not detectable" | | | |

**Table 8.**

| Antigen Peptide | **IFN-γ** production¹⁾ **(pg/well)** | | |
|---|---|---|---|
| | 1st well | 2nd well | 3rd well |
| **SART1** _{**690**} | **34** | **148** | **329** |
| | ***p=0.486*** | ***p=0.092*** | ***p=0.225*** |
| **SART2** _{**93**} | **12** | **58** | **21** |
| | ***p=0.714*** | ***p=0.355*** | ***p=0.122*** |
| **SART2** _{**161**} | **12** | **<0** | **76** |
| | ***p=0.551*** | ***p=0.291*** | ***p=0.471*** |
| **SART2** _{**889**} | **75** | **N.D.*** | **<0** |
| | ***p=0.231*** | | ***p=0.787*** |
| **SART3** _{**109**} | **325** | **N.D.** | **N.D.** |
| | ***p=0.334*** | | |
| **SART3** _{**315**} | **84** | **<0** | **446** |
| | ***p=0.078*** | ***p=0.112*** | ***p=0.121*** |
| **CypB** _{**84**} | **58** | **<0** | **60** |
| | ***p=0.58*** | ***p=0.878*** | ***p=0.155*** |
| **CypB** _{**91**} | **24 168** | | **N.D.** |
| | ***p=0.419*** | ***p=0.474*** | |
| **lck** _{**208**} | **41** | **1473** | **N.D.** |
| | ***p=0.209*** | ***p=0.275*** | |
| **lck** _{**486**} | **2** | **24** | **<0** |
| | ***p=0.972*** | ***p=0.568*** | ***p=0.739*** |
| **lck** _{**488**} | **25** | **99** | **N.D.** |
| | ***p=0.098*** | ***p=0.038*** | |
| **ART4** _{**13**} | **187** | **<0** | **30** |
| | ***p=0.088*** | ***p=0.361*** | ***p=0.131*** |
| **ART4** _{**75**} | **9** | **2** | **60** |
| | ***p=0.768*** | ***p=0.834*** | ***p=0.078*** |
| **ART1**_{**170**} | **3** | **48** | **53** |
| | ***p=0.877*** | ***p=0.052*** | ***p=0.250*** |

| | | | |
|---|---|---|---|
| *) N.D. means "not detectable" | | | |

**Table 9**

| Antigen Peptide | **IFN**-γ production **(pg/ml)** | | |
|---|---|---|---|
| | Pre-Vaccination | After 3rd dose of vaccine | After 6th dose of vaccine |
| **SART2** _{**161**} | **364** | **423** | **495** |
| **SART2** _{**889**} | **702** | **689** | **697** |
| **lck** _{**208**} | **602** | **676** | **745** |
| **ART4** _{**13**} | **262** | **580** | **565** |

**Table 10**

| Antigen Peptide | **IFN**-γ production **(pg/well)** | | | |
|---|---|---|---|---|
| | 1st well | 2nd well | 3rd well | 4th well |
| **SART3** _{**302**} | **<0** | **<0** | **<0** | **<0** |
| | ***p=0.126*** | | ***p=0.246*** | ***p=0.070*** |
| **SART3** _{**303**} | **<0** | **<0** | **<0** | **<0** |
| | ***p=0.065*** | ***p=0.350*** | ***p=0.755*** | ***p=0.206*** |
| **CypB** _{**172**} | **<0** | **0** | **<0** | **<0** |
| | ***p=0.342*** | ***p=0.989*** | ***p=0.154*** | ***p=0.286*** |
| **lck** _{**246**} | **<0** | **<0** | **<0** | **<0** |
| | ***p=0.084*** | ***p=0.543*** | ***p=0.141*** | ***p=0.055*** |
| **lck** _{**422**} | **1** | **<0** | **<0** | **<0** |
| | ***p=0.929*** | ***p=0.676*** | ***p=0.196*** | ***p=0.040*** |
| **ppMAPkkk** _{**294**} | **<0** | **34** | **<0** | **<0** |
| | ***p=0.562*** | ***p=0.462*** | ***p=0.174*** | ***p=0.365*** |
| **ppMAPkkk** _{**482**} | **43** | **64** | **<0** | **5** |
| | ***p=0.056*** | ***p=0.008*** | ***p=0.140*** | ***p=0.509*** |
| **WHSC2** _{**103**} | **3** | **0** | **<0** | **<0** |
| | ***p=0.629*** | ***p=0.990*** | ***p=0.250*** | ***p=0.077*** |
| **WHSC2** _{**141**} | **6** | **14** | **<0** | **9** |
| | ***p=0.678*** | ***p=0.518*** | ***p=0.495*** | ***p=0.438*** |
| **UBE2V** _{**43**} | **<0** | **<0** | **<0** | **<0** |
| | ***p=0.572*** | ***p=0.396*** | ***p=0.633*** | ***p=0.338*** |
| **UBE2V** _{**85**} | **0** | **<0** | **7** | **<0** |
| | ***p=0.999*** | ***p=0.475*** | ***p=0.540*** | ***p=0.158*** |
| **HNRPL** _{**140**} | **16** | **<0** | **<0** | **<0** |
| | ***p=0.582*** | ***p=0.745*** | ***p=0.528*** | ***p=0.032*** |
| **HNRPL** _{**501**} | **<0** | **7** | **<0** | **<0** |
| | ***p=0.965*** | ***p=0.756*** | ***p=0.093*** | ***p=0.035*** |

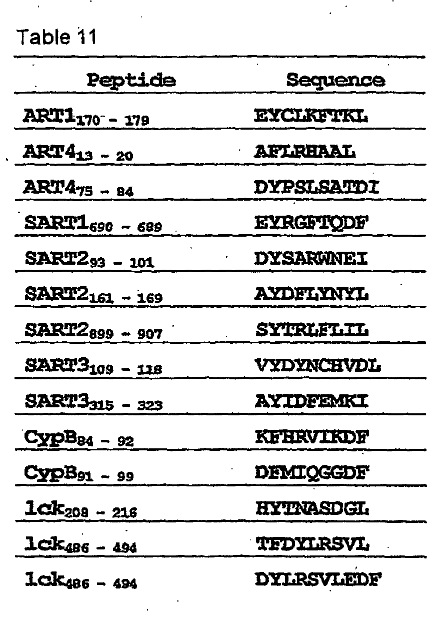

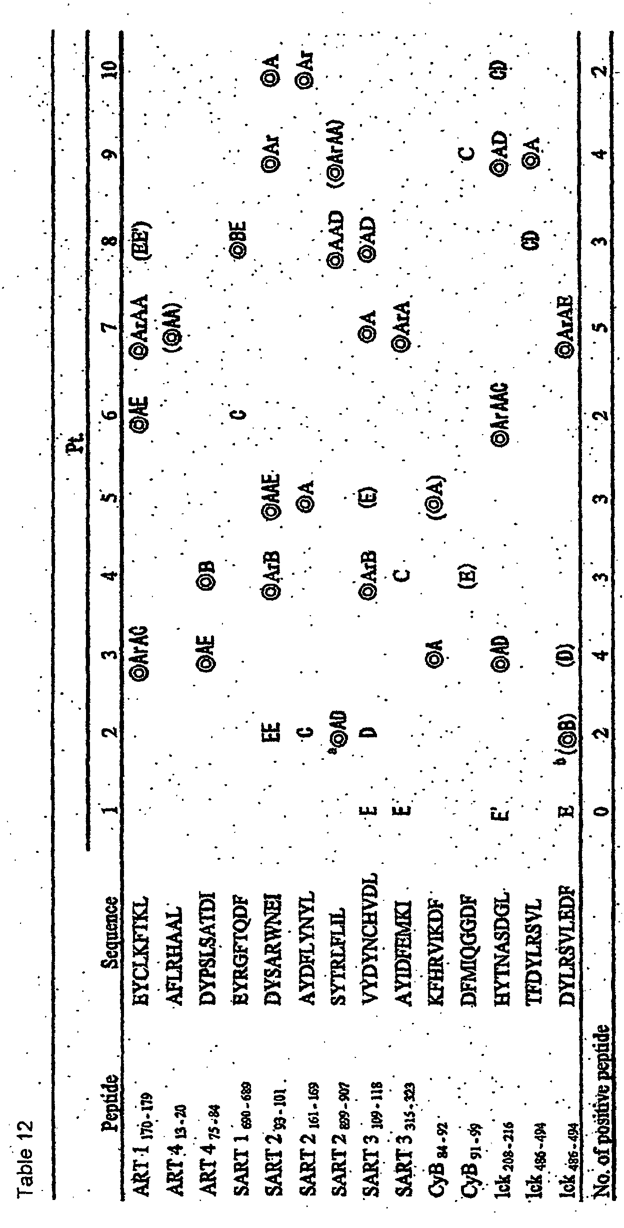

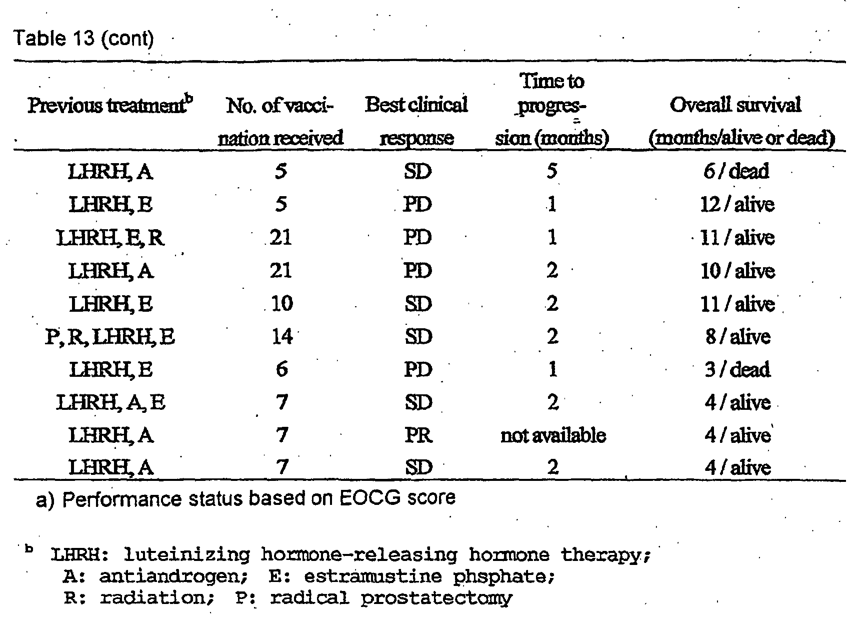

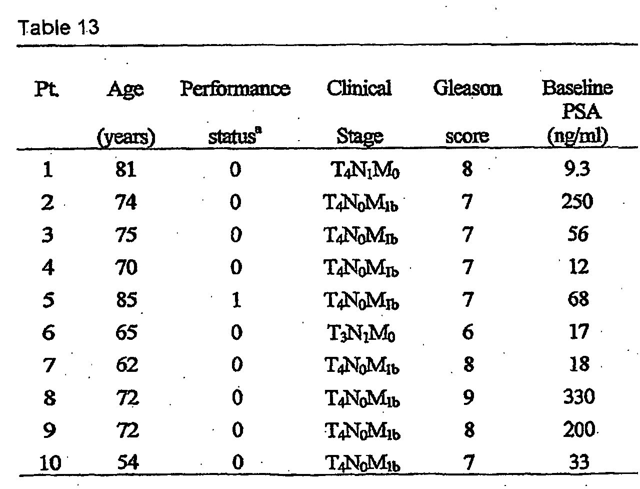

**Table 14**

| Peptide | Pancreatic cancer cell line | | Pancreatic cancer tissue | | Non-cancerous pancreatic tissue | |
|---|---|---|---|---|---|---|
| | Cytoplasmic fraction | Cell nucleus | Cytoplasmic fraction | Cell nucleus | Cytoplasmic fraction | Cell nucleus |
| SAPZ1₂₅₉ | 3/6 | 0/6 | 4/7 | 0/7 | 0/5 | 0/5 |
| SART2 | 7/9 | 7/9 | 7/7 | 5/7 | 0/5 | 0/5 |
| SART3 | 8/9 | 8/9 | 5/7 | 5/7 | 0/5 | 0/5 |
| ART4 | 5/6 | 6/6 | 5/7 | 6/7 | 0/5 | 0/5 |

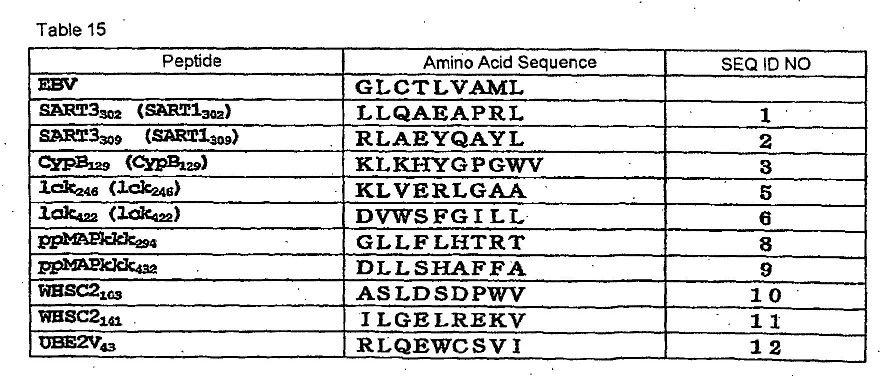

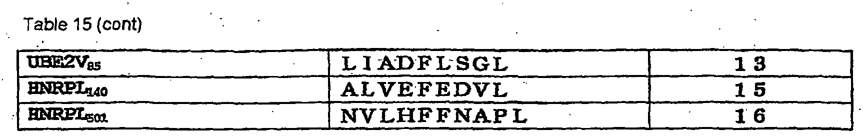

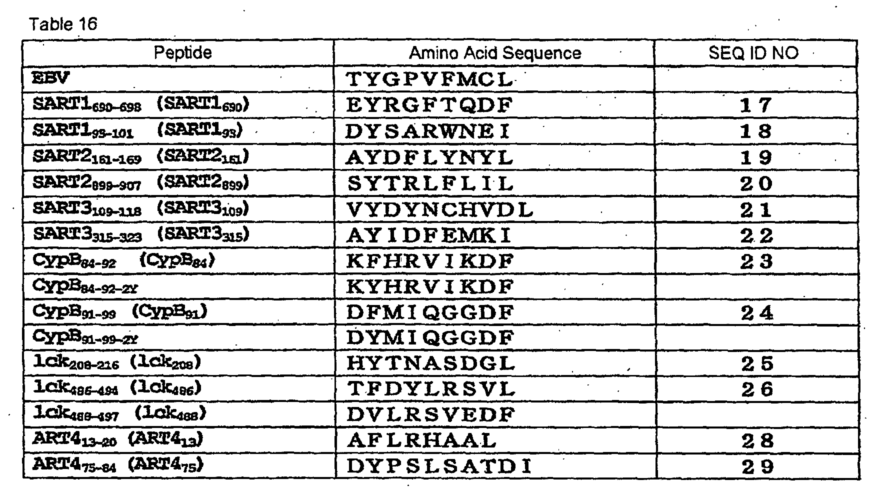

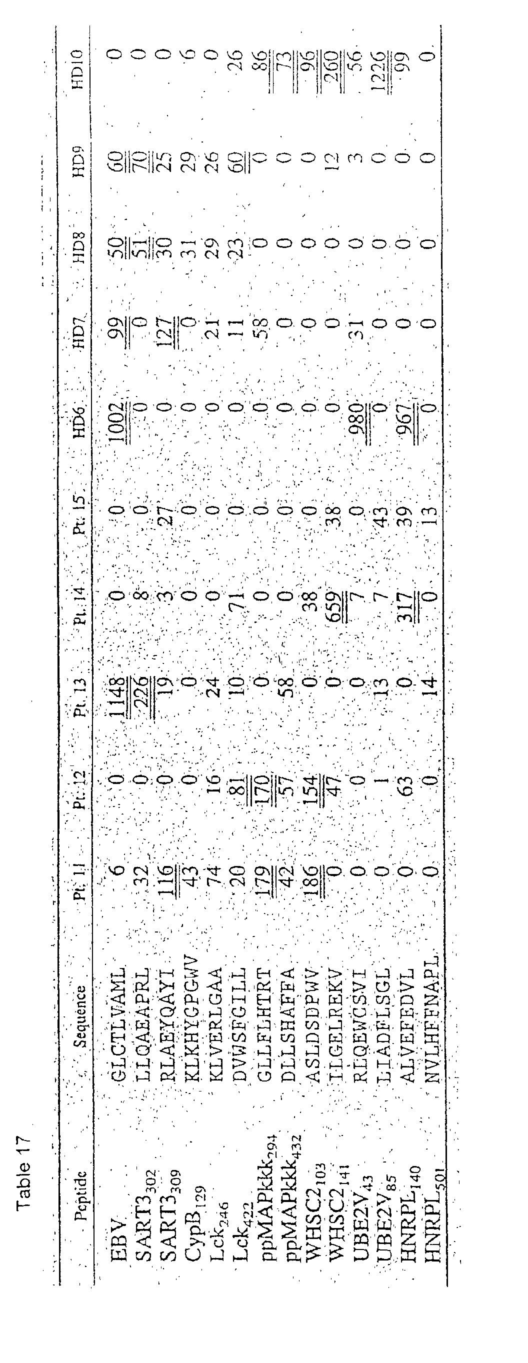

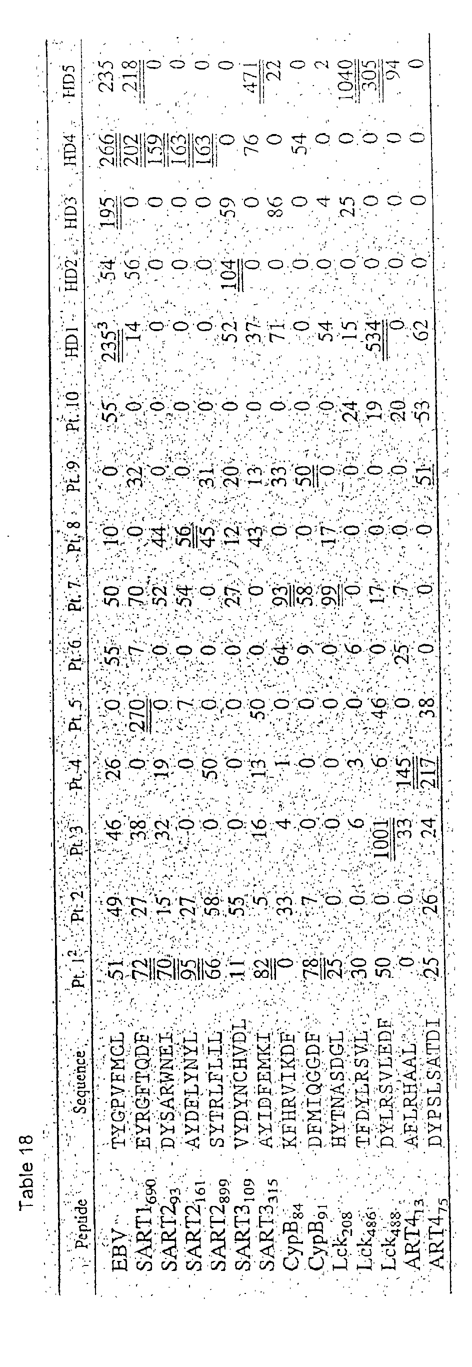

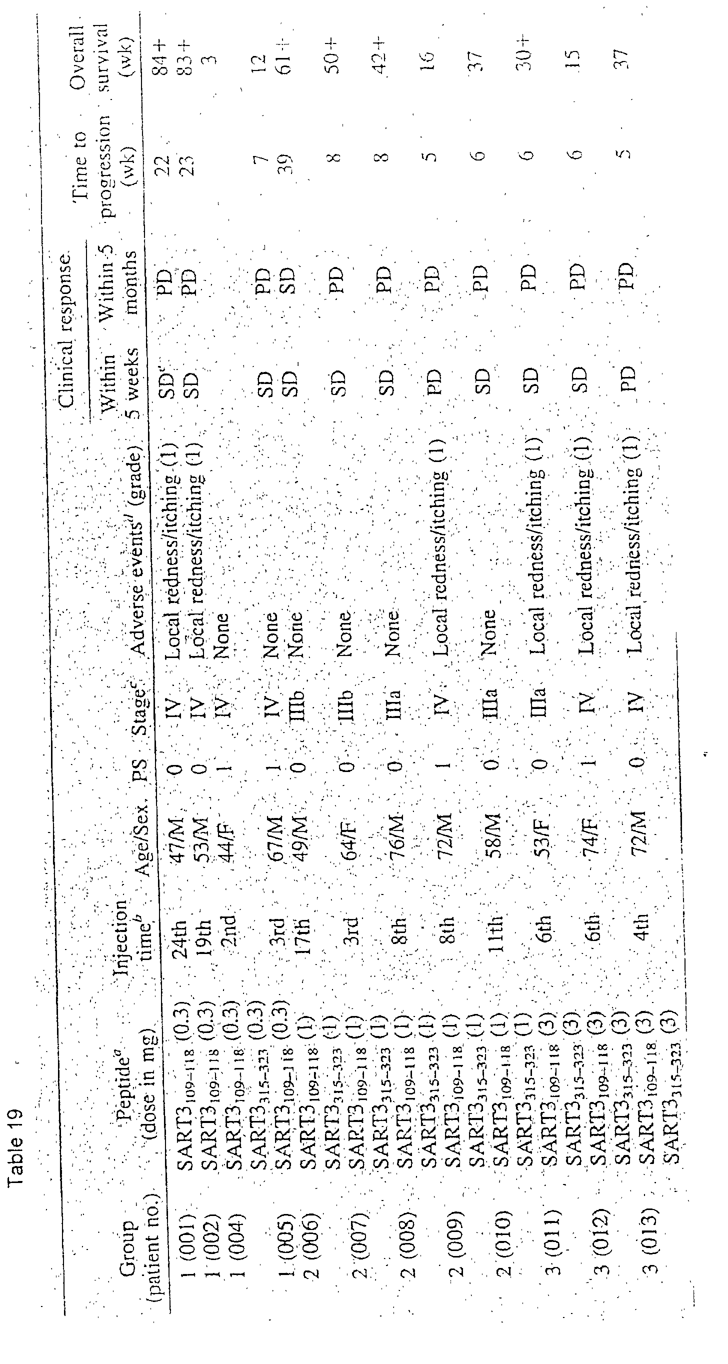

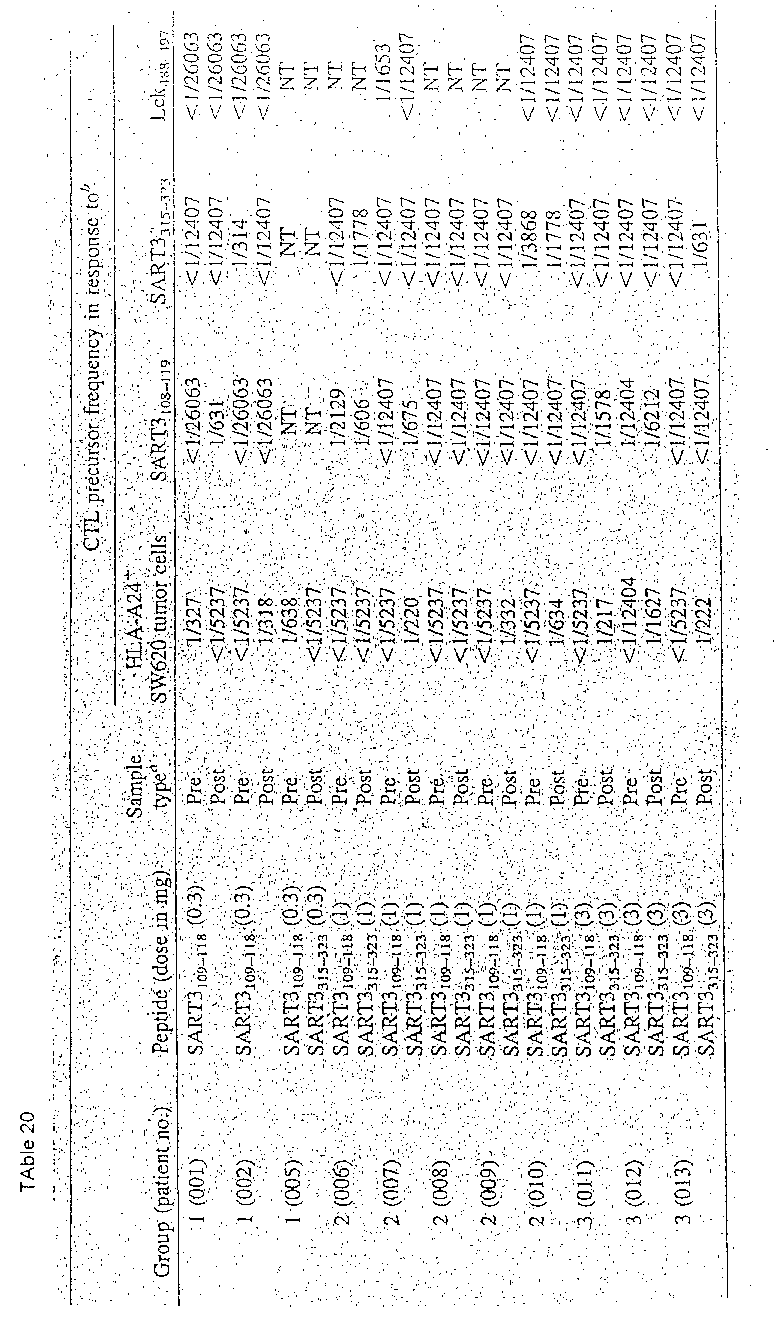

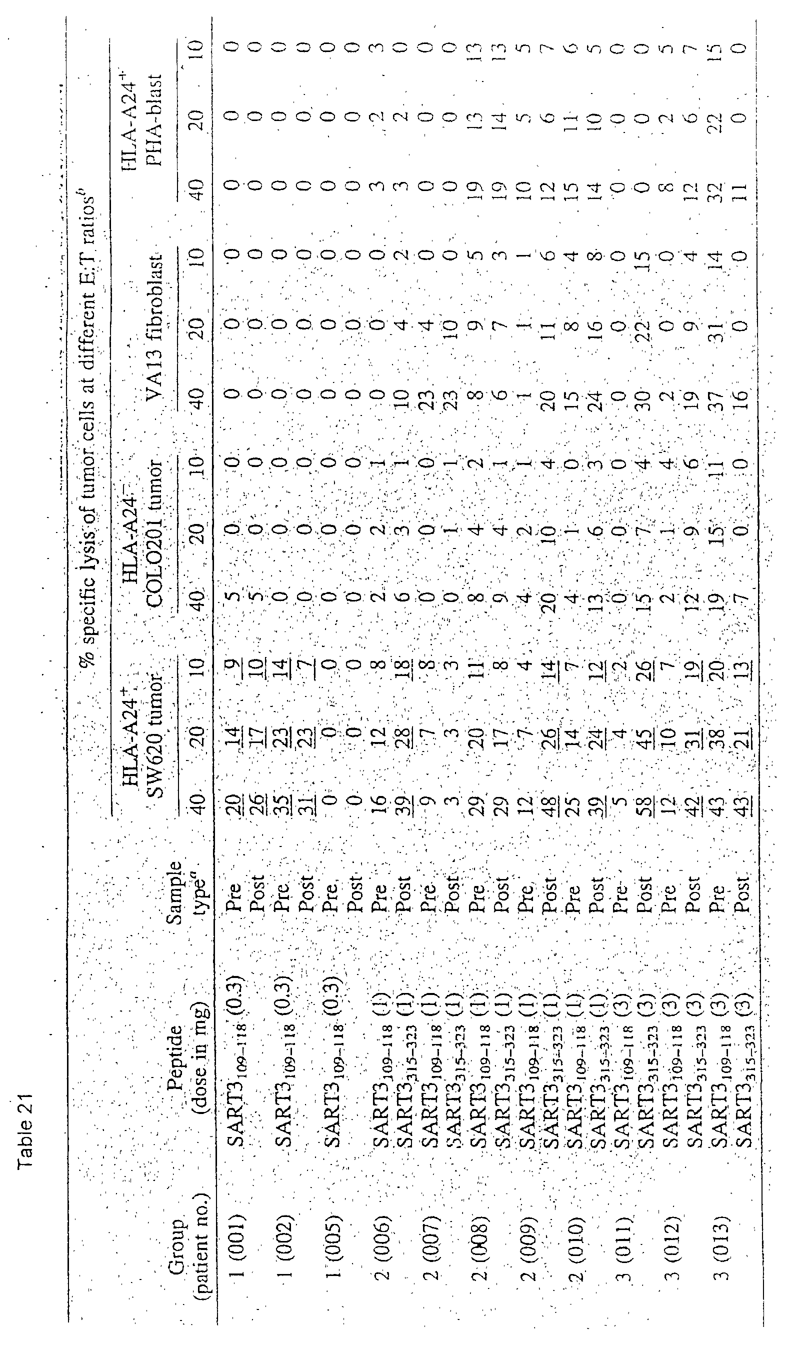

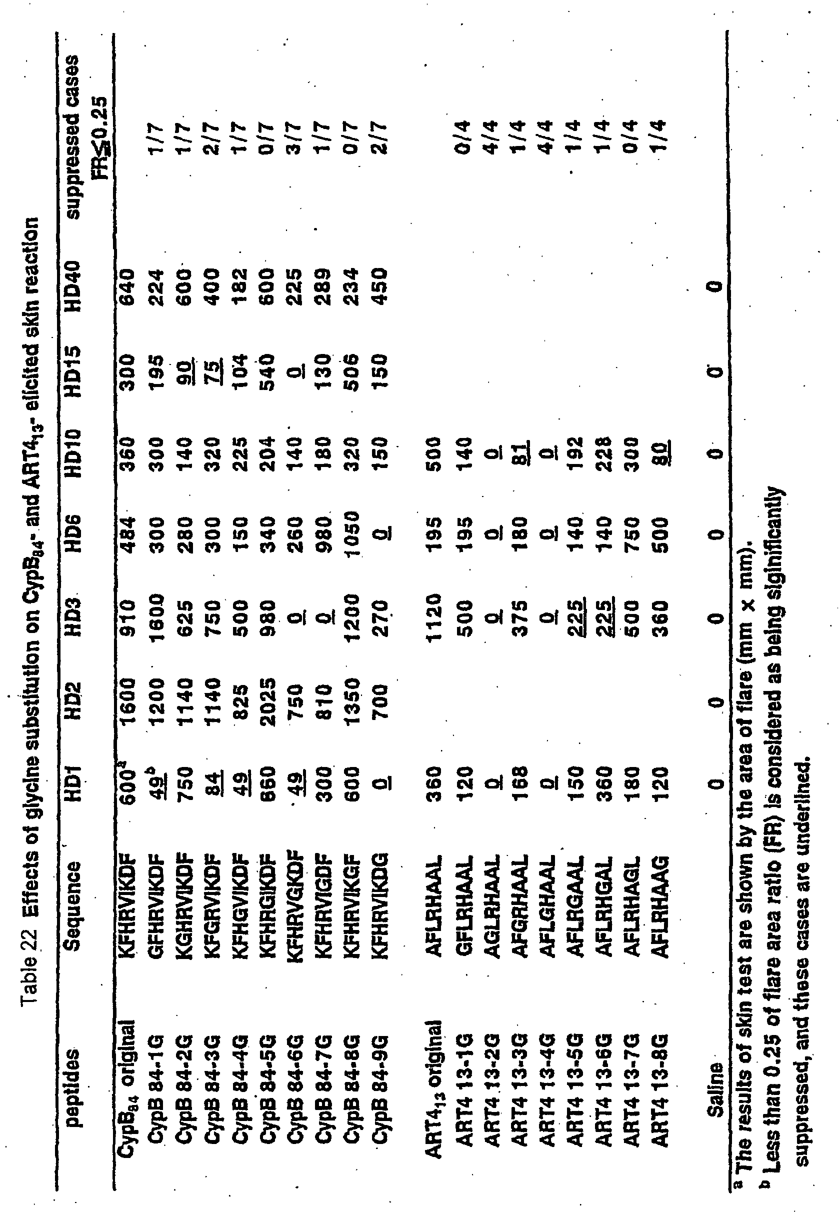

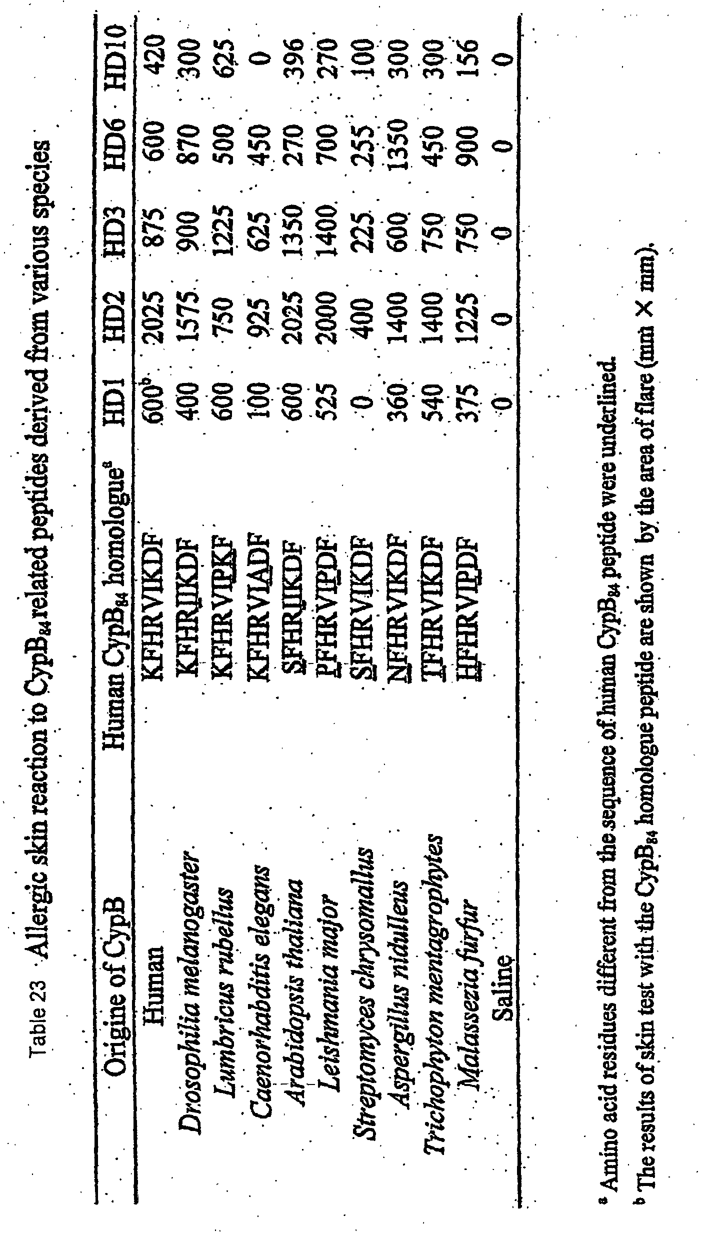

## Claims

1. A method for detecting antigen-specific T cells, comprising the steps of:
(1) collecting peripheral blood mononuclear cells;
(2) stimulating the collected peripheral blood mononuclear cells with an antigen frequently; and
(3) detecting the antigen-specific T cells in the stimulated peripheral blood mononuclear cells,
wherein the step of stimulating peripheral blood mononuclear cells in process (2) is performed without directly using antigen presenting cells.

2. A method for detecting antigen-specific T cells, comprising frequent stimulation with an antigen without newly adding antigen-presenting cells to the collected peripheral blood mononuclear cells, and detection of the antigen-specific T cells in the peripheral blood mononuclear cells.

3. The method of claim 1 or 2, wherein the peripheral blood mononuclear cells are collected from a patient with a disease.

4. The method of claim 1, 2, or 3, wherein the patient with the disease is a patient with cancer, a virus infection or autoimmune disease.

5. The method of any one of claims 1 to 4, wherein the patient is HLA-A2 positive or HLA-A24 positive.

6. The method of any one of claims 1 to 5, wherein the antigen is an antigen protein or an antigen peptide derived from the antigen protein.

7. The method of any one of claims 1 to 6, wherein the protein or the antigen peptide is a tumor rejection antigen.

8. The method of any one of claims 1 to 7, wherein the protein or the antigen peptide is represented by any one of SEQ ID NOs: 1 to 30.

9. The method of any one of claims 1 to 8, wherein frequent stimulation comprises multiple stimulations at a frequency of more than twice a week.

10. The method of any one of claims 1 to 9, wherein frequent stimulation comprises multiple stimulations at an interval of 2 to 5 days.

11. The method of any one of claims 1 to 10, wherein the detection of the antigen-specific T cells is performed using the amount of cytokine produced by the T cells as an index.

12. The method of any one of claims 1 to 11, wherein the cytokine is IFN-γ.

13. The method of any one of claims 1 to 12, wherein the detection of the antigen-specific T cells is performed by ELISA or FLISA.

14. The method of any one of claims 1 to 13, wherein frequent stimulation is performed in the wells of a microplate.

15. The method of any one of claims 1 to 14, wherein the microplate is a microplate having U-shaped wells.

16. The method of any one of claims 1 to 15, wherein the cell count of peripheral blood mononuclear cells is 2.5x10⁴ cells/well - 2x10⁵ cells/well using a 96-well microplate as the microplate.

17. The method of any one of claims 1 to 16, wherein the cell count of peripheral blood mononuclear cells is 2.5x10⁴ cells/well - 1x10⁵ cells/well using a 384-well microplate as the microplate.

18. A method for detecting an antigen that reacts with antigen-specific T cells comprising the steps of:
(1) collecting peripheral blood mononuclear cells;
(2) stimulating the collected peripheral blood mononuclear cells with multiple antigen candidates frequently; and
(3) determining which antigen candidates induced antigen-specific T cells,
wherein the step of stimulating the peripheral blood mononuclear cells in process (2) is performed without directly using antigen presenting cells.

19. A method for detecting antigen, **characterized by** frequent stimulation of the collected peripheral blood mononuclear cells with an antigen without adding new antigen presenting cells, and determination of whether T cells specific to the antigen are induced in the peripheral blood mononuclear cells.

20. The method of claim 18 or 19, wherein the peripheral blood mononuclear cells are collected from a patient with a disease.

21. The method of claim 18,19, or 20, wherein the patient with the disease is a patient with cancer, a virus infection, or an autoimmune disease.

22. The method of any one of claims 18 to 21, wherein the patient is HLA-A2 positive or HLA-A24 positive.

23. The method of any one of claims 18 to 22, wherein the antigen is an antigen protein or an antigen peptide derived from the antigen protein.

24. The method of any one of claims 17 to 23, wherein the protein or the antigen peptide is a tumor rejection antigen.

25. The method of any one of claims 18 to 24, wherein the protein or the antigen peptide is represented by any one of SEQ ID NOs: 1 to 30.

26. The method of any one of claims 18 to 25, wherein frequent stimulation comprises multiple stimulations at a frequency of more than twice a week.

27. The method of any one of claims 18 to 26, wherein frequent stimulation comprises multiple stimulations at an interval of 2 to 5 days.

28. The method of any one of claims 18 to 27, wherein the determination of the antigen-specific T cells is performed by quantifying the amount of cytokine produced by the T cells.

29. The method of any one of claims 18 to 28, wherein the cytokine is IFN-γ.

30. The method of any one of claims 18 to 29, wherein the detection of the antigen-specific T cells is performed by ELISA or FLISA.

31. The method of any one of claims 18 to 30, wherein frequent stimulation is performed in the wells of a microplate.

32. The method of any one of claims 18 to 31, wherein the microplate is a microplate having U-shaped wells.

33. The method of any one of claims 18 to 32, wherein the cell count of peripheral blood mononuclear cells is 2.5x10⁴ cells/well - 2x10⁵ cells/well using a 96-well microplate as the microplate.

34. The method of any one of claims 18 to 33, wherein the cell count of peripheral blood mononuclear cells is 2.5x10⁴ cells/well - 1x10⁵ cells/well using a 384-well microplate as the microplate.

35. A tailored pharmaceutical composition for treating a target disease in a patient, which is dedicated to the target disease in the patient, comprising an antigen related to the target disease in the patient.

36. A tailored pharmaceutical composition for treating a target disease in a patient, which is dedicated to the target disease in the patient, comprising one or more antigens related to the target disease in the patient, wherein the antigen is selected by a method comprising the steps of:
(1) collecting peripheral blood mononuclear cells;
(2) stimulating the collected peripheral blood mononuclear cells with multiple antigen candidates frequently, said candidates being thought to be related to the target disease; and
(3) determining which antigen candidates induced antigen-specific T cells,
wherein the step of stimulating the peripheral blood mononuclear cells in process (2) is performed without directly using antigen presenting cells.

37. A tailored pharmaceutical composition for treating a target disease in a patient, which is dedicated to the target disease in the patient, comprising an antigen selected by a method comprising stimulating collected peripheral blood mononuclear cells with an antigen frequently without adding new antigen presenting cells, and determining whether T cells specific to the antigen are induced in the peripheral blood mononuclear cells.

39. The tailored pharmaceutical composition of any one of claims 35 to 38, wherein the peripheral blood mononuclear cells are collected from a patient with the disease.

40. The tailored pharmaceutical composition of any one of claims 35 to 39, wherein the patient with the disease is a patient with cancer, a virus infection or an autoimmune disease.

41. The tailored pharmaceutical composition of any one of claims 35 to 40, wherein the patient is HLA-A2 positive or HLA-A24 positive.

42. The tailored pharmaceutical composition of any one of claims 35 to 41, wherein the antigen is an antigen protein or an antigen peptide derived from the antigen protein.

43. The tailored pharmaceutical composition of any one of claims 35 to 42, wherein the antigen peptide is represented by any one of SEQ ID NOs: 1 to 30.

44. The tailored pharmaceutical composition of any one of claims 35 to 43, wherein one or more of the antigen peptides are combined.

45. The tailored pharmaceutical composition of any one of claims 35 to 44, wherein the disease is cancer, a virus infection, or an autoimmune disease.

46. A method for treating a disease by tailored medical therapy dedicated to a target disease in a patient, comprising administering to the patient a therapeutically effective amount of an antigen related to the target disease in the patient.

47. A method for treating a disease by tailored medical therapy dedicated to a target disease in a patient, comprising administering to the patient one or more antigens related to the target disease in the patient, wherein the antigens are selected by a method comprising the steps of:
(1) collecting peripheral blood mononuclear cells;
(2) stimulating the collected peripheral blood mononuclear cells with multiple antigen candidates frequently, said candidates being thought to be related to the target disease; and
(3) determining which antigen candidates induced antigen-specific T cells,
wherein the step of stimulating the peripheral blood mononuclear cells in process (2) is performed without directly using antigen presenting cells.

48. A method for treating a disease by tailored medical therapy dedicated to a target disease in a patient, comprising administering to the patient one or more antigens selected by a method comprising stimulating collected peripheral blood mononuclear cells with an antigen frequently without adding new antigen presenting cells, and determination of whether T cells specific to the antigen are induced in the peripheral blood mononuclear cells.

49. The method of any one of claims 46 to 48, wherein the peripheral blood mononuclear cells are collected from a patient with the disease.

50. The method of any one of claims 46 to 49, wherein the patient with the disease is a patient with cancer, a virus infection, or an autoimmune disease.

51. The method of any one of claims 46 to 50, wherein the patient is HLA-A2 positive or HLA-A24 positive.

52. The method of any one of claims 46 to 51, wherein the antigen is an antigen protein or an antigen peptide derived from the antigen protein.

53. The method of any one of claims 46 to 52, wherein the disease is cancer, a virus infection, or an autoimmune disease.

54. A method for detecting allergy comprising detecting type I allergy using a non-mutating autoantigen peptide that is a tumor rejection antigen peptide recognized by HLA-A class I restricted cancer-specific cytotoxic T cells.

55. A method for inhibiting allergy comprising vaccinating with a non-allergic peptide derived from an allergic peptide-retaining antigen to inhibit allergic peptide-specific IgE response and type I allergy.
